# EUROPEAN PATENT APPLICATION

(11) **EP 4 309 734 A1**
(43) Date of publication of application: **24.01.2024**
(21) Application number: 23196321.6
(22) Date of filing: 09.07.2021
(51) Int. Cl.: A61P 9/00, A61P 9/10, C07K 16/24, A61K 39/00

(54) **METHODS FOR TREATING CARDIOVASCULAR DISEASE**

(30) Priority: 10.07.2020 US 202063050441 P
(62) Divisional of application: 21748526.7
(71) Applicant: Novo Nordisk A/S, 2880 Bagsværd (DK)
(72) Inventor: DEVALARAJA, Madhav N, Waltham, Massachusetts, 02451 (US); DAVIDSON, Michael H, Waltham, Massachusetts, 02451 (US); KAKKAR, Rahul, Waltham, Massachusetts, 02451 (US)

(57) **Abstract**

The disclosure provides methods of treating cardivascular disease, including pateints with atherosclerotic cardiovascular disease and/or chronic kidney disease.. The method comprises administering a therapeutically effective amount of ziltivekimab .

## Description

### CROSS REFERENCE TO RELATED APPLICATIONS

This application claims priority to U.S. Provisional Application No. 63/050,441 filed July 10, 2020, which is hereby incorporated herein by reference in its entirety.

### BACKGROUND

Chronic inflammation is a characteristic of many diseases, including both the classical rheumatic disorders such as rheumatoid arthritis, juvenile idiopathic arthritis, psoriatic arthritis, and inflammatory bowel disease, as well as other systemic diseases that are increasingly understood to be associated with chronic inflammation, such as cardiovascular disease, renal disease, neuroinflammatory diseases, anemias, cancer and aging.

The pro-inflammatory cytokine, IL-6, often plays a critical role in chronic inflammation through activation of the JAK-STAT signaling pathway, and IL-6 inhibitors have been developed to treat certain inflammatory disorders in which IL-6 has been shown to contribute significantly to disease etiology. The anti-IL-6 receptor antibody, tocilizumab (ACTEMRA), has been approved for treatment of rheumatoid arthritis, giant cell arteritis, polyarticular juvenile idiopathic arthritis, systemic juvenile idiopathic arthritis, and iatrogenic cytokine release syndrome. The anti-IL-6 receptor antibody, sarilumab (KEVZARA), has been approved to treat adult patients with moderately to severely active rheumatoid arthritis.

Although inhibition of IL-6 can be effective, treatment of chronic inflammation with IL-6 inhibitors using current dose regimens often leads to immune suppression. Immunosuppression can result in increased susceptibility to pathogens such as bacteria, fungi, and viruses. The FDA-approved product label for ACTEMRA warns of the risk of serious infections leading to hospitalization or death, including tuberculosis, bacterial, invasive fungal, viral, and other opportunistic infection; the KEVZARA label warns of serious infections leading to hospitalization or death including bacterial, viral, invasive fungal, and other opportunistic infections.

There is, therefore, a need for new methods for treating IL-6 mediated inflammation that do not lead to immune suppression.

### SUMMARY

We have demonstrated that IL-6 antagonists can be administered at a dose, on a schedule, and for a period sufficient to reduce cardiovascular morbidity, mortality and inflammation without causing immune suppression.

Accordingly, in one aspect, methods for treating cardiovascular morbidity, mortality of a patient are provided. The methods comprise: administering an IL-6 antagonist to a patient with chronic kidney disease at a dose that is sufficient to treat cardiovascular morbidity and mortality. In some embodiments, the patient has inflammation, e.g. IL-6 mediated inflammation. In some embodiments, the treatment is sufficient to reduce the inflammation without causing immune suppression. In some embodiments, the cardiovascular morbidity and mortality is selected from a group consisting of (i) nonfatal myocardial infarction, (ii) nonfatal stroke, and (iii) cardiovascular death. In some embodiments, cardiovascular morbidity and mortality is heart failure.

In another aspect, methods for treating IL-6-mediated inflammation in a patient are provided. The methods comprise: administering an IL-6 antagonist to a patient with IL-6-mediated inflammation at a dose that is sufficient to reduce inflammation without causing immune suppression.

In some embodiments, inflammation is measured by C-reactive protein (CRP) levels. In some embodiments, the patient has an elevated pre-treatment C-reactive protein (CRP) level. In some embodiments, the pre-treatment CRP level of the patient is at least 2 mg/L. In some embodiments, the pre-treatment CRP level of the patient is at least 4 mg/L. In some embodiments, the pre-treatment CRP level of the patient is at least 6 mg/L. In some embodiments, the pre-treatment CRP level of the patient is at least 10 mg/L.

In some embodiments, the patient has an elevated pre-treatment serum IL-6 level. In some embodiments, the pre-treatment serum IL-6 level of the patient is at least 4 pg/mL. In some embodiments, the pre-treatment serum IL-6 level of the patient is at least 4 pg/mL. In some embodiments, the pre-treatment serum IL-6 level of the patient is at least 5 pg/mL. In some embodiments, the pre-treatment serum IL-6 level of the patient is at least 10 pg/mL.

In some embodiments, the inflammation is measured by the level of C-reactive protein (CRP). In some embodiments, the post-treatment CRP level is no more than 2 mg/L. In some embodiments, the post-treatment CRP level is no more than 1 mg/L. In some embodiments, the CRP level is decreased by at least 50% as compared to pre-treatment levels. In some embodiments, the CRP level is decreased by at least 70% as compared to pre-treatment levels. In some embodiments, the CRP level is decreased by at least 80% as compared to pre-treatment levels. In some embodiments, the CRP level is decreased by at least 90% as compared to pre-treatment levels.

In some embodiments, the immune suppression is measured by absolute neutrophil count (ANC). In some embodiments, the post-treatment ANC is at least 500 cells/µL. In some embodiments, the post-treatment ANC is at least 1000 cells/µL. In some embodiments, the post-treatment ANC is at least 1500 cells/µL. In some embodiments, the post-treatment ANC is at least 2000 cells/µL. In some embodiments, the ANC is decreased by no more than 2000 cells/µL as compared to pre-treatment levels. In some embodiments, the ANC is decreased by no more than 1500 cells/µL as compared to pre-treatment levels. In some embodiments, the ANC is decreased by no more than 1000 cells/µL as compared to pre-treatment levels. In some embodiments, the ANC is decreased by no more than 500 cells/µL as compared to pre-treatment levels. In some embodiments, the ANC is decreased by no more than 50% as compared to pre-treatment levels. In some embodiments, the ANC is decreased by no more than 40% as compared to pre-treatment levels. In some embodiments, the ANC is decreased by no more than 30% as compared to pre-treatment levels. In some embodiments, the ANC is decreased by no more than 20% as compared to pre-treatment levels. In some embodiments, the ANC is decreased by no more than 10% as compared to pre-treatment levels. In some embodiments, the ANC is not decreased as compared to pre-treatment levels.

In some embodiments, the IL-6 antagonist is administered at a monthly equivalent dose that is no more than 30% of the monthly equivalent dose for treating rheumatoid arthritis with the same IL-6 antagonist. In some embodiments, the IL-6 antagonist is administered at a monthly equivalent dose that is no more than 20% of the monthly equivalent dose for treating rheumatoid arthritis with the same IL-6 antagonist. In some embodiments, the IL-6 antagonist is administered at a monthly equivalent dose that is no more than 10% of the monthly equivalent dose for treating rheumatoid arthritis with the same IL-6 antagonist. In some embodiments, the IL-6 antagonist is administered at a monthly equivalent dose that is about 25% of a monthly equivalent dose for treating rheumatoid arthritis with the same IL-6 antagonist. In some embodiments, the IL-6 antagonist is administered at a monthly equivalent dose that is about 20% of a monthly equivalent dose for treating rheumatoid arthritis with the same IL-6 antagonist. In some embodiments, the IL-6 antagonist is administered at a monthly equivalent dose that is about 15% of a monthly equivalent dose for treating rheumatoid arthritis with the same IL-6 antagonist. In some embodiments, the IL-6 antagonist is administered at a monthly equivalent dose that is about 10% of a monthly equivalent dose for treating rheumatoid arthritis with the same IL-6 antagonist. In some embodiments, the IL-6 antagonist is administered at a monthly equivalent dose that is about 5% of a monthly equivalent dose for treating rheumatoid arthritis with the same IL-6 antagonist.

In some embodiments, the IL-6 antagonist is an anti-IL-6 antibody.

In some embodiments, the anti-IL-6 antibody is ziltivekimab. In some embodiments, ziltivekimab is administered intravenously at a monthly equivalent dose of 2-40 mg. In some embodiments, ziltivekimab is administered intravenously at a monthly equivalent dose of 7.5-30 mg. In some embodiments, ziltivekimab is administered intravenously at a monthly equivalent dose of 7.5-15 mg. In some embodiments, ziltivekimab is administered intravenously at a monthly equivalent dose of 15-30 mg. In some embodiments, ziltivekimab is administered intravenously at a monthly equivalent dose of about 2 mg. In some embodiments, ziltivekimab is administered intravenously at a monthly equivalent dose of about 4 mg. In some embodiments, ziltivekimab is administered intravenously at a monthly equivalent dose of about 6 mg. In some embodiments, ziltivekimab is administered intravenously at a monthly equivalent dose of about 7.5 mg. In some embodiments, ziltivekimab is administered intravenously at a monthly equivalent dose of about 10 mg. In some embodiments, ziltivekimab is administered intravenously at a monthly equivalent dose of about 15 mg. In some embodiments, ziltivekimab is administered intravenously at a monthly equivalent dose of about 20 mg. In some embodiments, ziltivekimab is administered intravenously at a monthly equivalent dose of about 30 mg. In some embodiments, ziltivekimab is administered intravenously at a monthly equivalent dose of about 40 mg.

In some embodiments, ziltivekimab is administered subcutaneously at a monthly equivalent dose of 3-70 mg. In some embodiments, ziltivekimab is administered subcutaneously at a monthly equivalent dose of 7.5-30 mg. In some embodiments, ziltivekimab is administered subcutaneously at a monthly equivalent dose of 7.5-15 mg. In some embodiments, ziltivekimab is administered subcutaneously at a monthly equivalent dose of 15-30 mg. In some embodiments, ziltivekimab is administered subcutaneously at a monthly equivalent dose of about 3 mg. In some embodiments, ziltivekimab is administered subcutaneously at a monthly equivalent dose of about 7 mg. In some embodiments, ziltivekimab is administered subcutaneously at a monthly equivalent dose of about 7.5 mg. In some embodiments, ziltivekimab is administered subcutaneously at a monthly equivalent dose of about 10 mg. In some embodiments, ziltivekimab is administered subcutaneously at a monthly equivalent dose of about 15 mg. In some embodiments, ziltivekimab is administered subcutaneously at a monthly equivalent dose of about 17 mg. In some embodiments, ziltivekimab is administered subcutaneously at a monthly equivalent dose of about 30 mg. In some embodiments, ziltivekimab is administered subcutaneously at a monthly equivalent dose of about 35 mg. In some embodiments, ziltivekimab is administered subcutaneously at a monthly equivalent dose of about 70 mg.

In some embodiments, the patient has a hepcidin-mediated disorder.

In some embodiments, the patient has kidney disease. In some embodiments, the patient has chronic kidney disease. In some embodiments, the patient has KDOQI stage 1-5 chronic kidney disease. In some embodiments, the patient has KDOQI stage 3-5 chronic kidney disease. In some embodiments, the patient is not on dialysis. In some embodiments, the patient has KDOQI stage 5 chronic kidney disease. In some embodiments, the patient is on dialysis. In some embodiments, the patient has cardiorenal syndrome (CRS). In some embodiments, the patient has CRS Type 4.

In some embodiments, the patient has atherosclerotic cardiovascular disease (ASCVD).

In some embodiments, the patient has cardiovascular disease. In some embodiments, the patient has diuretic resistant heart failure. In some embodiments, the patient has congestive heart failure (CHF). In some embodiments, the patient has congestive heart failure (CHF) with reduced ejection fraction. In some embodiments, the patient has congestive heart failure (CHF) with mid-range ejection fraction. In some embodiments, the patient has congestive heart failure (CHF) with preserved ejection fraction. In some embodiments, the patient has acute coronary syndrome. In some embodiments, the patient has atherosclerosis. In some embodiments, the patient has atherosclerotic cardiovascular disease (ASCVD).

In some embodiments, the patient has anemia. In some embodiments, the patient has anemia of chronic disease. In some embodiments, the patient has iron-refractory iron-deficiency anemia (IRIDA).

In some embodiments, the patient has diabetes. In some embodiments, the patient has type II diabetes. In some embodiments, the patient has insulin-resistant diabetes.

In some embodiments, the patient has liver disease. In some embodiments, the patient has non-alcoholic steatohepatitis (NASH).

In some embodiments, the patient has osteoporosis.

In some embodiments, the patient has depression.

In some embodiments, the patient has asthma.

In some embodiments, the patient has neuroinflammatory disorder. In some embodiments, the patient has Alzheimer's disease. In some embodiments, the patient has Parkinson's disease. In some embodiments, the patient has multiple sclerosis. In some embodiments, the patient has amyotrophic lateral sclerosis (ALS).

In some embodiments, the patient has age-related macular degeneration (AMD).

In some embodiments, the patient has cancer. In some embodiments, the cancer is selected from the group consisting of: solid tumors, small cell lung cancer, non-small cell lung cancer, hematological cancer, multiple myeloma, leukemia, chronic lymphocytic leukemia (CLL), chronic myeloid leukemia (CML), lymphomas, Hodgkin's lymphoma and hepatic adenoma.

In some embodiments, patient has skin disease.

In some embodiments, the method prevents aging in the patient.

In another aspect, methods for treating inflammation in a patient with cardiovascular disease are provided herein. The methods comprise: administering an IL-6 antagonist to a patient with cardiovascular disease and CRP level greater than 2 mg/L at a dose that is sufficient to reduce CRP levels to 2 mg/L or less without causing neutropenia.

In some embodiments, the IL-6 antagonist is administered at a monthly equivalent dose that is no more than 30% of the monthly equivalent dose for treating rheumatoid arthritis with the same IL-6 antagonist. In some embodiments, the IL-6 antagonist is administered at a monthly equivalent dose that is no more than 20% of the monthly equivalent dose for treating rheumatoid arthritis with the same IL-6 antagonist. In some embodiments, the IL-6 antagonist is administered at a monthly equivalent dose that is no more than 10% of the monthly equivalent dose for treating rheumatoid arthritis with the same IL-6 antagonist.

In another aspect, methods for treating inflammation in a patient with chronic kidney disease (CKD) are provided herein. The methods comprise: administering an IL-6 antagonist to a patient with CKD and a CRP level greater than 2 mg/L at a dose that is sufficient to reduce CRP levels to 2 mg/L or less without causing neutropenia.

In some embodiments, the IL-6 antagonist is administered at a monthly equivalent dose that is no more than 30% of the monthly equivalent dose for treating rheumatoid arthritis with the same IL-6 antagonist. In some embodiments, the IL-6 antagonist is administered at a monthly equivalent dose that is no more than 20% of the monthly equivalent dose for treating rheumatoid arthritis with the same IL-6 antagonist. In some embodiments, the IL-6 antagonist is administered at a monthly equivalent dose that is no more than 10% of the monthly equivalent dose for treating rheumatoid arthritis with the same IL-6 antagonist.

### BRIEF DESCRIPTION OF THE DRAWINGS

These and other features, aspects, and advantages of the present invention will become better understood with regard to the following description, and accompanying drawings, where:
**FIG. 1** presents the dose escalation schematic for the phase 1/phase 2 randomized, double-blind, placebo-controlled trial of COR-001 in hemodialysis patients described in Example 1.
**FIG. 2** shows the timeline and the efficacy analysis of the treatment phase and the safety follow-up phase.

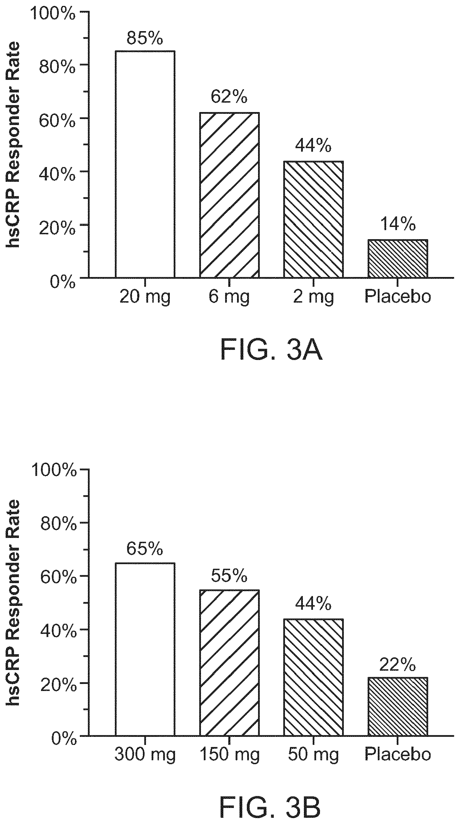

**FIGs. 3A** and **3B** show the results of C-reactive protein (CRP) responder analysis after treatment with COR-001 (anti-IL-6) or canakinumab (anti-IL1β). **FIG. 3A** shows the C-reactive protein responder rate after intravenous treatment with COR-001 in patients with stage 5 chronic kidney disease who were on dialysis in the clinical trial described in Example 1. The baseline hsCRP was 12.4 mg/L. Responder was defined as Week 12 average hsCRP < 2 mg/L. **FIG. 3B** shows the C-reactive protein responder rate after treatment with canakinumab in the CANTOS trial, as described in the research literature. The baseline hsCRP was 5.5 mg/L. Responder was defined as 3-month hsCRP < 2 mg/L.
**FIG. 4** shows the results of hemoglobin responder analysis after treatment with COR-001 at doses of 2 mg, 6 mg, and 20 mg. Hemoglobin responder was defined as increase by 1g/dL or more after Day 29. Investigators were not permitted to change ESA dosing until after Day 29.
**FIG. 5** shows the effect of COR-001 on the diastolic cardiac parameter, NTproBNP.
**FIGs. 6A** and **6B** show the adverse responder rate for neutrophils and platelets. **FIG. 6A** shows the neutrophils adverse responder rate. An Adverse Responder was defined as Week 12 average neutrophils < 2 × 10⁶/mL. **FIG 6B** shows the platelets adverse responder rate. Adverse responder was defined as Week 12 average platelets < 100 × 10⁶/mL.
**FIG. 7** presents the schematic of study design of the phase 2 RESCUE clinical trial described in Example 2.
**FIG. 8** presents the schematic of patient disposition of the phase 2 RESCUE clinical trial described in Example 2.
**FIGs. 9A and 9B** show the reduction of hsCRP in patients treated with placebo or Ziltivekimab at 7.5 mg, 15 mg, or 30 mg, with **FIG. 9A** showing the median percent reduction in hsCRP and **FIG. 9B** showing the hsCRP levels at baseline, week 13, and end of treatment.
**FIGs. 10A, 10B, 10C, and 10D** show the hsCRP responder rate with increasing doses of Ziltivekimab in comparison to canakinumab in CANTOS trial, with **FIG. 10A** showing the hsCRP responder rate defined by CRP reduction greater than or equal to 50%, **FIG. 10B** showing the hsCRP responder rate defined by hsCRP level less than 2 mg/L, **FIG. 10C** showing the hsCRP responder rate defined by CRP reduction greater than or equal to 50% and hsCRP level less than 2 mg/L, and **FIG. 10D** showing the hsCRP responder rate with canakinumab in CANTOS trial.
**FIG. 11** shows the effect of different doses of Ziltivekimab on hsCRP suppression at end of treatment.
**FIGs. 12A and 12B** show the suppression of hsCRP over treatment period, with **FIG. 12A** showing the change of hsCRP level and **FIG. 12B** showing the percent change of hsCRP.
**FIGs. 13A, 13B, 13C, and 13D** show the change of hsCRP level through week 23/24, with **FIG. 13A** showing the change of hsCRP level in patients treated with placebo (n=31), **FIG. 13B** showing the change of hsCRP level in patients treated with 7.5 mg Ziltivekimab (n=32), **FIG. 13C** showing the change of hsCRP level in patients treated with 15 mg Ziltivekimab (n=34), and **FIG. 13D** showing the change of hsCRP level in patients treated with 30 mg Ziltivekimab (n=32).
**FIG. 14** shows the absolute neutrophil counts (ANC) in patients treated with placebo or 7.5 mg, 15 mg, or 30 mg of Ziltivekimab over 32 weeks.
**FIGs. 15A and 15B** show the number and percentage of Grade 1-4 neutropenia in patients treated with placebo or Ziltivekimab, with **FIG. 15A** showing the number and percentage of patients with post-baseline worst value of ANC below 2000 and **FIG. 15B** showing the number and percentage of patients with sustained neutropenia (at least 2 visits in a row).
**FIGs. 16A and 16B** show the number and percentage of Grade 1-4 neutropenia in patients treated with placebo or sirukumab (anti-IL-6 antibody) or sarilumab (anti-IL-6R antibody), with **FIG. 16A** showing the number and percentage of patients with post-baseline values by maximum toxicity grade for neutrophils through 18 weeks of exposure to placebo or sirukumab and **FIG. 16B** showing the number and percentage of patients with decreased ANC by maximum grade with or without the treatment of sarilumab.
**FIG. 17** shows the platelet counts in patients treated with placebo or 7.5 mg, 15 mg, or 30 mg of Ziltivekimab over 32 weeks.
**FIGs. 18A and 18B** show the number and percentage of Grade 1-4 thrombocytopenia in patients treated with placebo or Ziltivekimab, with **FIG. 18A** showing the number and percentage of patients with post-baseline worst value of platelet count below 100,000 and **FIG. 18B** showing the number and percentage of patients with sustained thrombocytopenia (at least 2 visits in a row).
**FIG. 19** shows the number and percentage of patients with post-baseline values by maximum toxicity grade for platelets through 18 weeks of exposure to placebo or sirukumab.
**FIGs. 20A and 20B** show levels of alanine transaminase (ALT) and aspartate transaminase (AST) in patients treated with placebo or 7.5 mg, 15 mg, or 30 mg of Ziltivekimab over 32 weeks, with **FIG. 20A** showing the level of ALT and **FIG. 20B** showing the level of AST.
**FIG. 21** shows the number and percentage of ALT and AST abnormalities in patients treated with placebo or Ziltivekimab.
**FIG. 22** shows the number and percentage of ALT and AST abnormalities in patients treated with placebo or sirukumab.
**FIGs. 23A, 23B, 23C, and 23D** show the changes of LDL, triglyceride (TG), HDL, and TC/HDL at week 13 (W13) and end of treatment (ET) from baseline, with **FIG 23A** showing the change of LDL, **FIG 23B** showing the change of TG, **FIG 23C** showing the change of HDL, and **FIG 23D** showing the change of TC/HDL.
**FIGs. 24A, 24B, and 24C** show the changes of ApoB, ApoA1, and ApoB/ApoA1 at week 13 (W13) and end of treatment (ET) from baseline, with **FIG 24A** showing the change of ApoB, **FIG 24B** showing the change of ApoA1, and **FIG 24C** showing the change of ApoB/ApoA1.
**FIGs. 25A and 25B** show the reduction of fibrinogen in patients treated with Ziltivekimab at 7.5 mg, 15 mg, or 30 mg compared to placebo, with **FIG. 25A** showing the median percent reduction of fibrinogen and **FIG. 25B** showing the fibrinogen levels at baseline, week 13, and end of treatment.
**FIGs. 26A and 26B** show the reduction of serum amyloid A (SAA) in patients treated with Ziltivekimab at 7.5 mg, 15 mg, or 30 mg compared to placebo, with **FIG. 26A** showing the median percent reduction of SAA and **FIG. 26B** showing the SAA levels at baseline, week 13, and end of treatment.
**FIGs. 27A and 27B** show the reduction of haptoglobin in patients treated with Ziltivekimab at 7.5 mg, 15 mg, or 30 mg compared to placebo, with **FIG. 27A** showing the median percent reduction of haptoglobin and **FIG. 27B** showing the haptoglobin levels at baseline, week 13, and end of treatment.
**FIGs. 28A and 28B** show the reduction of secretory phospholipase A2 (sPLA2) in patients treated with Ziltivekimab at 7.5 mg, 15 mg, or 30 mg compared to placebo, with **FIG. 28A** showing the median percent reduction of sPLA2 and **FIG. 28B** showing the sPLA2 levels at baseline, week 13, and end of treatment.
**FIGs. 29A and 29B** show the reduction of lipoprotein(a) (LP(a)) in patients treated with Ziltivekimab at 7.5 mg, 15 mg, or 30 mg compared to placebo, with **FIG. 29A** showing the median percent reduction of LP(a) and **FIG. 29B** showing the LP(a) levels at baseline, week 13, and end of treatment.
**FIGs. 30A and 30B** show the change of hemoglobin in patients treated with placebo or 7.5 mg, 15 mg, or 30 mg of Ziltivekimab over 32 weeks, with **FIG. 30A** showing the change of hemoglobin level in all patients and **FIG. 30B** showing the change of hemoglobin level in the anemia subgroup (hemoglobin baseline <11g/dL).
**FIGs. 31A, 31B, 31C, and 31D** show the changes of hepcidin, iron, ferritin, and transferrin saturation (TSAT) at week 13 (W13) and end of treatment (ET) from baseline, with **FIG 31A** showing the change of hepcidin, **FIG 31B** showing the change of iron, **FIG 31C** showing the change of ferritin, and **FIG 31D** showing the change of TSAT.
**FIGs. 32A and 32B** show the change of NTproBNP at end of treatment (ET) from baseline, with **FIG 32A** showing the change of NTproBNP level in all patients and **FIG 32B** showing the change of NTproBNP level in patients with a >250 pg/mL baseline.
**FIGs. 33A and 33B** show the change of albumin in patients treated with placebo or Ziltivekimab at 7.5 mg, 15 mg, or 30 mg, with **FIG. 33A** showing the median percent change of albumin and **FIG. 33B** showing the albumin levels at baseline, week 13, and end of treatment.
**FIGs. 34A and 34B** show the change of prealbumin in patients treated with placebo or Ziltivekimab at 7.5 mg, 15 mg, or 30 mg, with **FIG. 34A** showing the median percent change of prealbumin and **FIG. 34B** showing the prealbumin levels at baseline, week 13, and end of treatment.
**FIGs. 35A, 35B, and 35C** show the changes of estimated glomerular filtration rate (eGFR) and urine albumin-to-creatinine ratio (UACR) at week 13 (W13) and end of treatment (ET) from baseline, with **FIG 35A** showing the change of eGFR estimated by creatinine, **FIG 35B** showing the change of eGFR estimated by cystatin C, and **FIG 35C** showing the change of UACR.

### DETAILED DESCRIPTION

### 1. Definitions

Unless defined otherwise, all technical and scientific terms used herein have the meaning commonly understood by one of ordinary skill in the art to which the invention pertains.

As used herein, "**interleukin 6 (IL-6)**" or "**IL-6 polypeptide**" refers to a polypeptide or fragment thereof having at least about 85% or greater amino acid identity to the amino acid sequence provided at NCBI Accession No. NP_000591 and having IL-6 biological activity. IL-6 is a pleotropic cytokine with multiple biologic functions. Exemplary IL-6 biological activities include immunostimulatory and pro-inflammatory activities. An exemplary IL-6 amino acid sequence is provided below:

1 MCVGARRLGR GPCAALLLLG LGLSTVTGLH CVGDTYPSND RCCHECRPGN GMVSRCSRSQ

61 NTVCRPCGPG FYNDVVSSKP CKPCTWCNLR SGSERKQLCT ATQDTVCRCR AGTQPLDSYK

121 PGVDCAPCPP GHFSPGDNQA CKPWTNCTLA GKHTLQPASN SSDAICEDRD PPATQPQETQ

181 GPPARPITVQ PTEAWPRTSQ GPSTRPVEVP GGRAVAAILG LGLVLGLLGP LAILLALYLL

241 RRDQRLPPDA HKPPGGGSFR TPIQEEQADA HSTLAKI (SEQ ID NO:1)

As used herein, "**interleukin 6 (IL-6) nucleic acid**" refers to a polynucleotide encoding an interleukin 6 (IL-6) polypeptide. An exemplary interleukin 6 (IL-6) nucleic acid sequence is provided at NCBI Accession No. NM_000600. The exemplary sequence at NCBI Accession No. NM_000600 is provided below:

1 AATATTAGAG TCTCAACCCC CAATAAATAT AGGACTGGAG ATGTCTGAGG CTCATTCTGC

61 CCTCGAGCCC ACCGGGAACG AAAGAGAAGC TCTATCTCCC CTCCAGGAGC CCAGCTATGA
121 ACTCCTTCTC CACAAGCGCC TTCGGTCCAG TTGCCTTCTC CCTGGGGCTG CTCCTGGTGT
181 TGCCTGCTGC CTTCCCTGCC CCAGTACCCC CAGGAGAAGA TTCCAAAGAT GTAGCCGCCC
241 CACACAGACA GCCACTCACC TCTTCAGAAC GAATTGACAA ACAAATTCGG TACATCCTCG
301 ACGGCATCTC AGCCCTGAGA AAGGAGACAT GTAACAAGAG TAACATGTGT GAAAGCAGCA
361 AAGAGGCACT GGCAGAAAAC AACCTGAACC TTCCAAAGAT GGCTGAAAAA GATGGATGCT
421 TCCAATCTGG ATTCAATGAG GAGACTTGCC TGGTGAAAAT CATCACTGGT CTTTTGGAGT
481 TTGAGGTATA CCTAGAGTAC CTCCAGAACA GATTTGAGAG TAGTGAGGAA CAAGCCAGAG
541 CTGTGCAGAT GAGTACAAAA GTCCTGATCC AGTTCCTGCA GAAAAAGGCA AAGAATCTAG
601 ATGCAATAAC CACCCCTGAC CCAACCACAA ATGCCAGCCT GCTGACGAAG CTGCAGGCAC
661 AGAACCAGTG GCTGCAGGAC ATGACAACTC ATCTCATTCT GCGCAGCTTT AAGGAGTTCC
721 TGCAGTCCAG CCTGAGGGCT CTTCGGCAAA TGTAGCATGG GCACCTCAGA TTGTTGTTGT
781 TAATGGGCAT TCCTTCTTCT GGTCAGAAAC CTGTCCACTG GGCACAGAAC TTATGTTGTT
841 CTCTATGGAG AACTAAAAGT ATGAGCGTTA GGACACTATT TTAATTATTT TTAATTTATT
901 AATATTTAAA TATGTGAAGC TGAGTTAATT TATGTAAGTC ATATTTATAT TTTTAAGAAG
961 TACCACTTGA AACATTTTAT GTATTAGTTT TGAAATAATA ATGGAAAGTG GCTATGCAGT
1021 TTGAATATCC TTTGTTTCAG AGCCAGATCA TTTCTTGGAA AGTGTAGGCT TACCTCAAAT
1081 AAATGGCTAA CTTATACATA TTTTTAAAGA AATATTTATA TTGTATTTAT ATAATGTATA
1141 AATGGTTTTT ATACCAATAA ATGGCATTTT AAAAAATTCA GCAAAAAAAA AAAAAAAAAA
1201 A (SEQ ID NO:2)

As used herein, "**interleukin 6 receptor (IL-6R) complex**" refers to a protein complex comprising an IL-6 receptor subunit alpha (IL-6Rα) and interleukin 6 signal transducer glycoprotein 130 (gp130), also termed interleukin 6 receptor subunit β (IL-6Rβ).

As used herein, "**interleukin 6 receptor subunit α (IL-6Rα) polypeptide**" refers to a polypeptide or fragment thereof having at least about 85% or greater amino acid identity to the amino acid sequence provided at NCBI Accession No. NP_000556 or NP_852004 and having IL-6 receptor biological activity. Exemplary IL-6Rα biological activities include binding to IL-6, binding to glycoprotein 130 (gp130), and regulation of cell growth and differentiation. An exemplary IL-6R sequence is provided below:

1 MLAVGCALLA ALLAAPGAAL APRRCPAQEV ARGVLTSLPG DSVTLTCPGV EPEDNATVHW

61 VLRKPAAGSH PSRWAGMGRR LLLRSVQLHD SGNYSCYRAG RPAGTVHLLV DVPPEEPQLS

121 CFRKSPLSNV VCEWGPRSTP SLTTKAVLLV RKFQNSPAED FQEPCQYSQE SQKFSCQLAV

181 PEGDSSFYIV SMCVASSVGS KFSKTQTFQG CGILQPDPPA NITVTAVARN PRWLSVTWQD

241 PHSWNSSFYR LRFELRYRAE RSKTFTTWMV KDLQHHCVIH DAWSGLRHVV QLRAQEEFGQ

301 GEWSEWSPEA MGTPWTESRS PPAENEVSTP MQALTTNKDD DNILFRDSAN ATSLPVQDSS

361 SVPLPTFLVA GGSLAFGTLL CIAIVLRFKK TWKLRALKEG KTSMHPPYSL GQLVPERPRP

421 TPVLVPLISP PVSPSSLGSD NTSSHNRPDA RDPRSPYDIS NTDYFFPR (SEQ ID NO:3)

As used herein, "**glycoprotein 130 (gp130)**" or "**interleukin 6 receptor subunit β (IL-6Rβ) polypeptide"** refers to a polypeptide or fragment thereof having at least about 85% or greater amino acid identity to the amino acid sequence provided at NCBI Accession No. NP_002175, NP_786943, or NP_001177910 and having IL-6 receptor biological activity. Exemplary IL-6Rβ biological activities include binding to IL-6Rα, IL-6 receptor signaling activity, and regulation of cell growth, differentiation, hepcidin expression etc. An exemplary IL-6Rβ sequence is provided below:

1 MLTLQTWLVQ ALFIFLTTES TGELLDPCGY ISPESPVVQL HSNFTAVCVL KEKCMDYFHV
61 NANYIVWKTN HFTIPKEQYT IINRTASSVT FTDIASLNIQ LTCNILTFGQ LEQNVYGITI
121 ISGLPPEKPK NLSCIVNEGK KMRCEWDGGR ETHLETNFTL KSEWATHKFA DCKAKRDTPT
181 SCTVDYSTVY FVNIEVWVEA ENALGKVTSD HINFDPVYKV KPNPPHNLSV INSEELSSIL
241 KLTWTNPSIK SVIILKYNIQ YRTKDASTWS QIPPEDTAST RSSFTVQDLK PFTEYVFRIR
301 CMKEDGKGYW SDWSEEASGI TYEDRPSKAP SFWYKIDPSH TQGYRTVQLV WKTLPPFEAN
361 GKILDYEVTL TRWKSHLQNY TVNATKLTVN LTNDRYLATL TVRNLVGKSD AAVLTIPACD
421 FQATHPVMDL KAFPKDNMLW VEWTTPRESV KKYILEWCVL SDKAPCITDW QQEDGTVHRT
481 YLRGNLAESK CYLITVTPVY ADGPGSPESI KAYLKQAPPS KGPTVRTKKV GKNEAVLEWD
541 QLPVDVQNGF IRNYTIFYRT IIGNETAVNV DSSHTEYTLS SLTSDTLYMV RMAAYTDEGG
601 KDGPEFTFTT PKFAQGEIEA IVVPVCLAFL LTTLLGVLFC FNKRDLIKKH IWPNVPDPSK
661 SHIAQWSPHT PPRHNFNSKD QMYSDGNFTD VSVVEIEAND KKPFPEDLKS LDLFKKEKIN
721 TEGHSSGIGG SSCMSSSRPS ISSSDENESS QNTSSTVQYS TVVHSGYRHQ VPSVQVFSRS
781 ESTQPLLDSE ERPEDLQLVD HVDGGDGILP RQQYFKQNCS QHESSPDISH FERSKQVSSV
841 NEEDFVRLKQ QISDHISQSC GSGQMKMFQE VSAADAFGPG TEGQVERFET VGMEAATDEG
901 MPKSYLPQTV RQGGYMPQ (SEQ ID NO:4)

Unless otherwise specified, "**IL-6 antagonist**" refers an agent that is capable of decreasing the biological activity of IL-6. IL-6 antagonists include agents that decrease the level of IL-6 polypeptide in serum, including agents that decrease the expression of an IL-6 polypeptide or nucleic acid; agents that decrease the ability of IL-6 to bind to the IL-6R; agents that decrease the expression of the IL-6R; and agents that decrease signal transduction by the IL-6R receptor when bound by IL-6. In preferred embodiments, the IL-6 antagonist decreases IL-6 biological activity by at least about 10%, 20%, 30%, 50%, 70%, 80%, 90%, 95%, or even 100%. As further described below, IL-6 antagonists include IL-6 binding polypeptides, such as anti-IL-6 antibodies and antigen binding fragments or derivatives thereof; IL-6R binding polypeptides, such as anti-IL-6R antibodies and antigen binding fragments or derivatives thereof; and synthetic chemical molecules, such as JAK1 and JAK3 inhibitors.

The term "**IL-6 antibody**" or "**anti-IL-6 antibody**" refers to an antibody that specifically binds IL-6. Anti-IL-6 antibodies include monoclonal and polyclonal antibodies that are specific for IL-6, and antigen-binding fragments or derivatives thereof. IL-6 antibodies are described in greater detail below.

As used herein, the term "**IL-6 mediated inflammation**" or "**IL-6 mediated inflammatory disorder**" refers to inflammation or inflammation related disorder in which IL-6 is known or suspected to contribute to the etiology or symptoms of the inflammation.

The term "**C-reactive protein**" or "**CRP**" refers to a polypeptide or fragment thereof having at least about 85% or greater amino acid identity to the amino acid sequence provided at NCBI Accession No. NP_000558 and having complement activating activity. CRP levels increase in response to inflammation, and can be measured with an hsCRP (highsensitivity C-reactive protein) test. An exemplary CRP sequence is provided below:

1 MEKLLCFLVL TSLSHAFGQT DMSRKAFVFP KESDTSYVSL KAPLTKPLKA FTVCLHFYTE

61 LSSTRGYSIF SYATKRQDNE ILIFWSKDIG YSFTVGGSEI LFEVPEVTVA PVHICTSWES

121 ASGIVEFWVD GKPRVRKSLK KGYTVGAEAS IILGQEQDSF GGNFEGSQSL VGDIGNVNMW

181 DFVLSPDEIN TIYLGGPFSP NVLNWRALKY EVQGEVFTKP QLWP (SEQ ID NO:5)

As used herein, "**hepcidin**" refers to a polypeptide having at least about 85% or greater amino acid identity to the amino acid sequence provided at NCBI Accession No. NP_066998 ("hepcidin preprotein"), or biologically active fragment thereof. Exemplary hepcidin biological activities include binding and reducing the levels of the iron export channel ferroportin, inhibiting iron transport, inhibiting intestinal iron absorption, and inhibiting iron release from macrophages and the liver. An exemplary hepcidin preprotein amino acid sequence is provided below: 
1   MALSSQIWAA CLLLLLLLAS LTSGSVFPQQ TGQLAELQPQ DRAGARASWM PMFQRRRRRD

61  THFPICIFCC GCCHRSKCGM CCKT (SEQ ID NO:6)
 With reference to the sequence above, hepcidin exists in various forms, including as a preprohormone (amino acids 25-84), prohormone (amino acids 25-84), and mature forms termed hepcidin-25 (amino acids 60-84), hepcidin-22 (amino acids 63-84), and hepcidin-20 (amino acids 65-84).

A "**hepcidin-mediated disorder**" is any disorder in which hepcidin expression contributes to the etiology of the disorder or any of its symptoms.

The term "**immune suppression**" or "**immunosuppression**" refers to a reduction of the activation or efficacy of the immune system. Immune suppression can be measured by the number of white blood cells, such as neutrophils.

As used herein, "**neutrophil**" of "**neutrocyte**" refers to a type of white blood cell that is an essential part of the innate immune system. The absolute neutrophil count (ANC) can be used in diagnosis and prognosis. Low neutrophil counts are termed neutropenia.

The term "**agent**" refers to any compound or composition suitable to be administered in therapy, and explicitly includes chemical compounds; proteins, including antibodies or antigen-binding fragments thereof; peptides; and nucleic acid molecules.

The term "**subject**" refers to a human or non-human mammal, including, but not limited to, bovine, equine, canine, ovine, feline, and rodent, including murine and rattus, subjects. A "**patient**" is a human subject in need of treatment.

As used herein, the terms "**treat**," "**treating**," "**treatment**," and the like refer to reducing or ameliorating a disorder, and/or signs or symptoms associated therewith, or slowing or halting the progression thereof. It will be appreciated that, although not precluded, treating a disorder or condition does not require that the disorder, condition or symptoms associated therewith be completely eliminated.

As used herein, "**pre-treatment**" means prior to the first administration of an IL-6 antagonist according the methods described herein. Pre-treatment does not exclude, and often includes, the prior administration of treatments other than an IL-6 antagonist.

As used herein, "**post-treatment**" means after the administration of an IL-6 antagonist according the methods described herein. Post-treatment includes after any administration of an IL-6 antagonist at any dosage described herein. Post-treatment also includes after the treatment phase of an IL-6 antagonist.

In this disclosure, "**comprises**," "**comprising**," "**containing**," "**having**," "**includes**," "**including**," and linguistic variants thereof have the meaning ascribed to them in U.S. Patent law, permitting the presence of additional components beyond those explicitly recited.

The term "**biological sample**" refers to any tissue, cell, fluid, or other material derived from an organism (e.g., human subject). In certain embodiments, the biological sample is serum or blood.

Unless otherwise specified, antibody constant region residue numbering is according to the EU index as in Kabat.

### 2. Methods of Treating IL-6 Mediated Inflammation

In a first aspect, methods of treating IL-6-mediated inflammation in a patient are presented. The methods comprise administering an IL-6 antagonist to a patient with IL-6-mediated inflammation at a dose that is sufficient to reduce inflammation without causing immune suppression.

### 2.1. Pre-Treatment Serum CRP and IL-6 Levels

In the methods described herein, the patient has an IL-6-mediated inflammation.

In typical embodiments, the patient has elevated pre-treatment levels of C-reactive protein (CRP).

In some embodiments, the patient has a pre-treatment CRP level at least 2 mg/L. In some embodiments, the patient has a pre-treatment CRP level at least 2 mg/L, 2.5 mg/L, 3 mg/L, 3.5 mg/L, 4 mg/L, 4.5 mg/L, or 5 mg/L. In some embodiments, the patient has pre-treatment CRP levels at least 7.5 mg/L, 10 mg/L, 12.5 mg/L, or 15 mg/L. In various embodiments, the patient has a pre-treatment CRP level at least 2 mg/L. In various embodiments, the patient has a pre-treatment CRP level at least 2.5 mg/L. In various embodiments, the patient has a pre-treatment CRP level at least 5 mg/L. In various embodiments, the patient has a pre-treatment CRP level at least 7.5 mg/L. In various embodiments, the patient has a pre-treatment CRP level at least 10 mg/L. In various embodiments, the patient has a pre-treatment CRP level at least 12.5 mg/L. In various embodiments, the patient has a pre-treatment CRP level at least 15 mg/L.

In some embodiments of the methods described herein, the patient has elevated pre-treatment serum levels of IL-6.

In some embodiments, the patient has a pre-treatment serum IL-6 level of at least 2 pg/ml. In various embodiments, the patient has a pre-treatment serum IL-6 level of at least 2 pg/ml, at least 3 pg/ml, at least 4 pg/ml, at least 5 pg/ml, at least 6 pg/ml, at least 7 pg/ml, at least 8 pg/ml, at least 9 pg/ml, at least 10 pg/ml, at least 11 pg/ml, at least 12 pg/ml, at least 13 pg/ml, at least 14 pg/ml, or at least 15 pg/ml. In certain embodiments, the patient has a pre-treatment serum IL-6 level of at least 2 pg/ml. In certain embodiments, the patient has a pre-treatment serum IL-6 level of at least 2.5 pg/ml. In certain embodiments, the patient has a pre-treatment serum IL-6 level of at least 4 pg/ml. In certain embodiments, the patient has a pre-treatment serum IL-6 level of at least 5 pg/ml. In certain embodiments, the patient has a pre-treatment serum IL-6 level of at least 7.5 pg/ml. In certain embodiments, the patient has a pre-treatment serum IL-6 level of at least 10 pg/ml. In certain embodiments, the patient has a pre-treatment serum IL-6 level of at least 12.5 pg/ml. In certain embodiments, the patient has a pre-treatment serum IL-6 level of at least 15 pg/ml.

In some embodiments, the patient has elevated pre-treatment serum levels of CRP and elevated pre-treatment IL-6 levels. In certain embodiments, the patient has a pre-treatment serum IL-6 level of at least 2 pg/ml and a pre-treatment CRP level at least 2 mg/L. In certain embodiments, the patient has a pre-treatment serum IL-6 level of at least 2 pg/ml and a pre-treatment CRP level at least 2.5 mg/L. In certain embodiments, the patient has a pre-treatment serum IL-6 level of at least 2 pg/ml and a pre-treatment CRP level at least 5 mg/L. In certain embodiments, the patient has a pre-treatment serum IL-6 level of at least 2 pg/ml and a pre-treatment CRP level at least 10 mg/L. In certain embodiments, the patient has a pre-treatment serum IL-6 level of at least 4 pg/ml and a pre-treatment CRP level at least 2 mg/L. In certain embodiments, the patient has a pre-treatment serum IL-6 level of at least 4 pg/ml and a pre-treatment CRP level at least 2.5 mg/L. In certain embodiments, the patient has a pre-treatment serum IL-6 level of at least 4 pg/ml and a pre-treatment CRP level at least 5 mg/L. In certain embodiments, the patient has a pre-treatment serum IL-6 level of at least 4 pg/ml and a pre-treatment CRP level at least 10 mg/L. In certain embodiments, the patient has a pre-treatment serum IL-6 level of at least 5 pg/ml and a pre-treatment CRP level at least 2 mg/L. In certain embodiments, the patient has a pre-treatment serum IL-6 level of at least 5 pg/ml and a pre-treatment CRP level at least 2.5 mg/L. In certain embodiments, the patient has a pre-treatment serum IL-6 level of at least 5 pg/ml and a pre-treatment CRP level at least 5 mg/L. In certain embodiments, the patient has a pre-treatment serum IL-6 level of at least 5 pg/ml and a pre-treatment CRP level at least 10 mg/L. In certain embodiments, the patient has a pre-treatment serum IL-6 level of at least 10 pg/ml and a pre-treatment CRP level at least 2 mg/L. In certain embodiments, the patient has a pre-treatment serum IL-6 level of at least 10 pg/ml and a pre-treatment CRP level at least 2.5 mg/L. In certain embodiments, the patient has a pre-treatment serum IL-6 level of at least 10 pg/ml and a pre-treatment CRP level at least 5 mg/L. In certain embodiments, the patient has a pre-treatment serum IL-6 level of at least 10 pg/ml and a pre-treatment CRP level at least 10 mg/L.

### 2.2. Reduction of IL-6 and C-Reactive Protein (CRP)

In typical embodiments, the IL-6 antagonist is administered at a dose sufficient to reduce the patient's free serum IL-6 levels below pre-treatment levels.

In some embodiments, the free serum IL-6 level is decreased by at least 10% as compared to pre-treatment levels. In various embodiments, the free serum IL-6 level is decreased by at least 20%, 30%, 40%, 50%, 60%, 70%, 80%, or 90% as compared to pre-treatment levels. In certain embodiments, the free serum IL-6 level is decreased by at least 20% as compared to pre-treatment levels. In certain embodiments, the free serum IL-6 level is decreased by at least 30% as compared to pre-treatment levels. In certain embodiments, the free serum IL-6 level is decreased by at least 40% as compared to pre-treatment levels. In certain embodiments, the free serum IL-6 level is decreased by at least 50% as compared to pre-treatment levels. In certain embodiments, the free serum IL-6 level is decreased by at least 60% as compared to pre-treatment levels. In certain embodiments, the free serum IL-6 level is decreased by at least 70% as compared to pre-treatment levels. In certain embodiments, the free serum IL-6 level is decreased by at least 80% as compared to pre-treatment levels. In certain embodiments, the free serum IL-6 level is decreased by at least 90% as compared to pre-treatment levels.

In some embodiments, the IL-6 antagonist is administered at a dose sufficient to reduce the patient's CRP levels below pre-treatment levels. In some embodiments, the IL-6 mediated inflammation is measured by the CRP levels.

In certain embodiments, the post-treatment CRP level is no more than 5 mg/L. In certain embodiments, the post-treatment CRP level is no more than 2.5 mg/L. In certain embodiments, the post-treatment CRP level is no more than 2 mg/L. In certain embodiments, the post-treatment CRP level is no more than 1 mg/L.

In some embodiments, the CRP level is decreased by at least 10% as compared to pre-treatment levels. In various embodiments, the CRP level is decreased by at least 20%, 30%, 40%, 50%, 60%, 70%, 80%, or 90% as compared to pre-treatment levels. In certain embodiments, the CRP level is decreased by at least 20% as compared to pre-treatment levels. In certain embodiments, the CRP level is decreased by at least 30% as compared to pre-treatment levels. In certain embodiments, the CRP level is decreased by at least 40% as compared to pre-treatment levels. In certain embodiments, the CRP level is decreased by at least 50% as compared to pre-treatment levels. In certain embodiments, the CRP level is decreased by at least 60% as compared to pre-treatment levels. In certain embodiments, the CRP level is decreased by at least 70% as compared to pre-treatment levels. In certain embodiments, the CRP level is decreased by at least 80% as compared to pre-treatment levels. In certain embodiments, the CRP level is decreased by at least 90% as compared to pre-treatment levels.

### 2.3. Neutrophil Level

### 2.3.1. Absolute Neutrophil Count (ANC)

In the methods described herein, the IL-6 antagonist is administered at a dose sufficient to reduce inflammation without causing immune suppression.

In some embodiments, the immune suppression of the patient is measured by Absolute Neutrophil Count (ANC).

In some embodiments, the post-treatment ANC is at least 300 cells/µL. In various embodiments, the post-treatment ANC is at least 500 cells/µL, 600 cells/µL, 700 cells/µL, 800 cells/µL, 900 cells/µL, 1000 cells/µL, 1100 cells/µL, 1200 cells/µL, 1300 cells/µL, 1400 cells/µL, 1500 cells/µL, 1600 cells/µL, 1700 cells/µL, 1800 cells/µL, 1900 cells/µL, or 2000 cells/µL. In certain embodiments, the post-treatment ANC is at least 500 cells/µL. In certain embodiments, the post-treatment ANC is at least 750 cells/µL. In certain embodiments, the post-treatment ANC is at least 1000 cells/µL. In certain embodiments, the post-treatment ANC is at least 1250 cells/µL. In certain embodiments, the post-treatment ANC is at least 1500 cells/µL. In certain embodiments, the post-treatment ANC is at least 1750 cells/µL. In certain embodiments, the post-treatment ANC is at least 2000 cells/µL.

In some embodiments, the ANC is decreased by no more than 2500 cells/µL as compared to pre-treatment levels. In various embodiments, the ANC is decreased by no more than 2000 cells/µL, 1900 cells/µL, 1800 cells/µL, 1700 cells/µL, 1600 cells/µL, 1500 cells/µL, 1400 cells/µL, 1300 cells/µL, 1200 cells/µL, 1100 cells/µL, 1000 cells/µL, 900 cells/µL, 800 cells/µL, 700 cells/µL, 600 cells/µL, or 500 cells/µL, as compared to pre-treatment levels. In certain embodiments, the ANC is decreased by no more than 2000 cells/µL as compared to pre-treatment levels. In certain embodiments, the ANC is decreased by no more than 1750 cells/µL as compared to pre-treatment levels. In certain embodiments, the ANC is decreased by no more than 1500 cells/µL as compared to pre-treatment levels. In certain embodiments, the ANC is decreased by no more than 1250 cells/µL as compared to pre-treatment levels. In certain embodiments, the ANC is decreased by no more than 1000 cells/µL as compared to pre-treatment levels. In certain embodiments, the ANC is decreased by no more than 750 cells/µL as compared to pre-treatment levels. In certain embodiments, the ANC is decreased by no more than 500 cells/µL as compared to pre-treatment levels.

In some embodiments, the ANC is decreased by no more than 70% as compared to pre-treatment levels. In various embodiments, the ANC is decreased by no more than 60%, 50%, 40%, 30%, 20%, 10%, or 5% as compared to pre-treatment levels. In certain embodiments, the ANC is decreased by no more than 60% as compared to pre-treatment levels. In certain embodiments, the ANC is decreased by no more than 50% as compared to pre-treatment levels. In certain embodiments, the ANC is decreased by no more than 40% as compared to pre-treatment levels. In certain embodiments, the ANC is decreased by no more than 30% as compared to pre-treatment levels. In certain embodiments, the ANC is decreased by no more than 20% as compared to pre-treatment levels. In certain embodiments, the ANC is decreased by no more than 10% as compared to pre-treatment levels. In certain embodiments, the ANC is decreased by no more than 5% as compared to pre-treatment levels.

In some embodiments, the ANC is not decreased as compared to pre-treatment levels.

### 2.4. Lipoprotein(a) Level

In some embodiments, the IL-6 antagonist is administered at a dose sufficient to reduce the patient's lipoprotein(a) levels below pre-treatment levels.

In some embodiments, the lipoprotein(a) level is decreased by at least 10% as compared to pre-treatment levels. In various embodiments, the lipoprotein(a) level is decreased by at least 20%, 30%, 40%, 50%, 60%, 70%, 80%, or 90% as compared to pre-treatment levels. In certain embodiments, the lipoprotein(a) level is decreased by at least 20% as compared to pre-treatment levels. In certain embodiments, the lipoprotein(a) level is decreased by at least 30% as compared to pre-treatment levels. In certain embodiments, the lipoprotein(a) level is decreased by at least 40% as compared to pre-treatment levels. In certain embodiments, the lipoprotein(a) level is decreased by at least 50% as compared to pre-treatment levels. In certain embodiments, the lipoprotein(a) level is decreased by at least 60% as compared to pre-treatment levels. In certain embodiments, the lipoprotein(a) level is decreased by at least 70% as compared to pre-treatment levels. In certain embodiments, the lipoprotein(a) level is decreased by at least 80% as compared to pre-treatment levels. In certain embodiments, the lipoprotein(a) level is decreased by at least 90% as compared to pre-treatment levels.

### 2.5. LDL Level

In some embodiments, the IL-6 antagonist is administered at a dose sufficient to reduce the patient's lipoprotein(a) levels without significantly increasing the patent's lowdensity lipoprotein (LDL) levels.

In some embodiments, the LDL level is increased by no more than 15% as compared to pre-treatment levels. In various embodiments, the LDL level is increased by no more than 12%, 10%, 8%, 6%, 5%, 4%, 3%, 2% or 1% as compared to pre-treatment levels. In certain embodiments, the LDL level is increased by no more than 12% as compared to pre-treatment levels. In certain embodiments, the LDL level is increased by no more than 10% as compared to pre-treatment levels. In certain embodiments, the LDL level is increased by no more than 8% as compared to pre-treatment levels. In certain embodiments, the LDL level is increased by no more than 6% as compared to pre-treatment levels. In certain embodiments, the LDL level is increased by no more than 5% as compared to pre-treatment levels. In certain embodiments, the LDL level is increased by no more than 4% as compared to pre-treatment levels. In certain embodiments, the LDL level is increased by no more than 3% as compared to pre-treatment levels. In certain embodiments, the LDL level is increased by no more than 2% as compared to pre-treatment levels. In certain embodiments, the LDL level is increased by no more than 1% as compared to pre-treatment levels.

In certain embodiments, the LDL level is not increased as compared to pre-treatment levels.

### 2.6. IL-6 Mediated Inflammatory Disorders

In the methods described herein, the patient has an IL-6 mediated inflammatory disorder.

### 2.6.1. Non-hepcidin-mediated inflammatory disorders

In various embodiments, the IL-6 mediated inflammatory disorder is not a hepcidin-mediated disorder. Hepcidin-mediated disorders are described in US 2017/0029499, the disclosure of which is incorporated herein by reference in its entirety.

### 2.6.2. Hepcidin-mediated inflammatory disorders

In various embodiments, the IL-6 mediated inflammatory disorder is a hepcidin-mediated disorder. Hepcidin-mediated disorders are described in US 2017/0029499, the disclosure of which is incorporated herein by reference in its entirety. In particular embodiments, the patient has a hepcidin-mediated disorder and at least one copy of the major allele at the TMPRSS6 rs855791 SNP (amino acid 736A). In other embodiments, the patient has a hepcidin-mediated disorder and is homozygous for the minor allele at the TMPRSS6 rs855791 SNP (amino acid 736V). In certain embodiments, the patient has a hepcidin-mediated disorder and unknown genotype at the TMPRSS6 rs855791 SNP.

### 2.6.3. Non-autoimmune inflammatory disorder

In various embodiments, the IL-6 mediated inflammatory disorder is a non-autoimmune IL-6 mediated inflammatory disorder. In particular embodiments, the patient has an IL-6 mediated disorder other than rheumatoid arthritis, giant cell arteritis, polyarticular juvenile idiopathic arthritis, or systemic juvenile idiopathic arthritis.

### 2.6.4. Kidney Disease

In various embodiments, the patient has kidney disease. In some embodiments, the kidney disease is chronic kidney disease (CKD).

In some embodiments, the patient has KDOQI stage 1-5 chronic kidney disease. In some embodiments, the patient has KDOQI stage 3-5 chronic kidney disease. In some embodiments, the patient has KDOQI stage 1 chronic kidney disease, KDOQI stage 2 chronic kidney disease, KDOQI stage 3 chronic kidney disease, KDOQI stage 4 chronic kidney disease, or KDOQI stage 5 chronic kidney disease. In certain embodiments, the patient has KDOQI stage 5 chronic kidney disease.

In some embodiments, the patient is on dialysis. In some embodiments, the patient is not on dialysis. In certain embodiment, the patient has KDOQI stage 3-5 chronic kidney disease, wherein the patient is not on dialysis. In certain embodiment, the patient has KDOQI stage 5 chronic kidney disease, wherein the patient is on dialysis.

In some embodiments, the patient has cardiorenal syndrome (CRS). In certain embodiments, the patient has CRS Type 4.

In some embodiments, the CKD patient has atherosclerotic cardiovascular disease (ASCVD). In some embodiments, the CKD patient is at risk of atherosclerotic cardiovascular disease (ASCVD). In some embodiments, the IL-6 antagonist is administered at a dose sufficient to decrease the risk of ASCVD in the CKD patient.

In some embodiments, the patient has been treated with dialysis.

### 2.6.5. Cardiovascular Disease

In various embodiments, the patient has cardiovascular disease.

In some embodiments, the patient has had a previous myocardial infarction. In particular embodiments, the patient has had a previous myocardial infarction and has a CRP level of 2 mg/L or more.

In certain embodiments, the patient has suffered a myocardial infarction within the 60 days prior to first administration of an IL-6 antagonist. In particular embodiments, the patient has suffered a myocardial infarction within the 30 days, 14 days, 7 days, 48 hours, or 24 hours prior to first administration of an IL-6 antagonist.

In some embodiments, the patient has atherosclerosis but has not had a myocardial infarction. In particular embodiments, the patient has atherosclerosis, has not had a myocardial infarction, and has a CRP level of 2 mg/L or more.

In some embodiments, the cardiovascular disease is congestive heart failure (CHF). In certain embodiments, the patient has congestive heart failure (CHF) with reduced ejection fraction. In certain embodiments, the patient has congestive heart failure (CHF) with mid-range ejection fraction. In certain embodiments, the patient has congestive heart failure (CHF) with preserved ejection fraction.

In various embodiments, the IL-6 mediated inflammatory disorder is heart failure that is not diuretic resistant. Diuretic resistant heart failure is described in WO 2018/144773, the disclosure of which is incorporated herein by reference in its entirety.

In some embodiments, the cardiovascular disease is diuretic resistant heart failure. Diuretic resistant heart failure is described in WO 2018/144773, the disclosure of which is incorporated herein by reference in its entirety.

In some embodiments, the cardiovascular disease is acute coronary syndrome.

In certain embodiments, the IL-6 antagonist is administered at a dose sufficient to reduce nonfatal myocardial infarction, nonfatal stroke, and/or cardiovascular death. In some embodiments, the IL-6 antagonist is administered at a dose sufficient to reduce the risk of heart failure. In some embodiments, the IL-6 antagonist is administered at a dose sufficient to increase cardiac function. In some embodiments, the IL-6 antagonist is administered at a dose sufficient to reduce fibrosis after acute myocardial infarction.

In certain embodiments, the patient has atherosclerotic cardiovascular disease (ASCVD). In particular embodiments, the patient has ASCVD and has a CRP level of 2 mg/L or more. In some embodiments, the IL-6 antagonist is administered at a dose sufficient to decrease the risk of ASCVD.

### 2.6.6. Anemia

In various embodiments, the patient has anemia.

In some embodiments, the patient has anemia of chronic disease. In some embodiments, the patient has iron-refractory iron-deficiency anemia (IRIDA).

In some of these embodiments, the patient has been treated with an erythropoiesisstimulating agent (ESA). In some embodiments, the patient has been treated with iron supplementation. In some embodiments, the patient has been treated with transfusion of blood or packed red blood cells.

In some embodiments, the IL-6 antagonist is administered at a dose sufficient to reverse functional iron deficiency.

### 2.6.7. Diabetes

In some embodiments, the patient has diabetes. In certain embodiments, the patient has type II diabetes. In certain embodiments, the patient has insulin-resistant diabetes.

### 2.6.8. Liver Disease

In some embodiments, the patient has liver disease. In certain embodiments, the patient has non-alcoholic steatohepatitis (NASH).

### 2.6.9. Osteoporosis

In some embodiments, the patient has osteoporosis.

### 2.6.10. Depression

In some embodiments, the patient has depression.

### 2.6.11. Asthma

In some embodiments, the patient has asthma.

### 2.6.12. Neuroinflammatory Disorder

In some embodiments, the patient has neuroinflammatory disorder. In certain embodiments, the patient has Alzheimer's disease. In certain embodiments, the patient has Parkinson's disease. In certain embodiments, the patient has multiple sclerosis. In certain embodiments, the patient has amyotrophic lateral sclerosis (ALS).

### 2.6.13. Age-Related Macular Degeneration

In some embodiments, the patient has age-related macular degeneration (AMD).

### 2.6.14. Cancer

In various embodiments, the patient has cancer.

In some embodiments, the cancer is selected from the group consisting of: solid tumors, small cell lung cancer, non-small cell lung cancer, hematological cancer, multiple myeloma, leukemia, chronic lymphocytic leukemia (CLL), chronic myeloid leukemia (CML), lymphomas, and Hodgkin's lymphoma.

### 2.6.15. Skin Disease

In various embodiments, the patient has skin disease, such as atopic dermatitis or psoriasis.

### 2.6.16. Aging

In some embodiments, the method prevents aging in the patient.

### 2.7. IL-6 Antagonists

The IL-6 antagonist used in the methods described herein is capable of decreasing the biological activity of IL-6.

### 2.7.1. Anti-IL-6 Antibodies

In various embodiments, the IL-6 antagonist is an anti-IL-6 antibody or antigen-binding fragment or derivative thereof.

In typical embodiments, the anti-IL-6 antibody neutralizes the biological activity of IL-6. In some embodiments, the neutralizing antibody prevents binding of IL-6 to the IL-6 receptor.

In some embodiments, the IL-6 antagonist is an anti-IL-6 monoclonal antibody. In some embodiments, the IL-6 antagonist is a polyclonal composition comprising a plurality of species of anti-IL-6 antibodies, each of the plurality having unique CDRs.

In some embodiments, the anti-IL-6 antibody is a Fab, Fab', F(ab')₂ , Fv, scFv, (scFv)₂, single chain antibody molecule, dual variable domain antibody, single variable domain antibody, linear antibody, or V domain antibody.

In some embodiments, the anti-IL-6 antibody comprises a scaffold. In certain embodiments, the scaffold is Fc, optionally human Fc. In some embodiments, the anti-IL-6 antibody comprises a heavy chain constant region of a class selected from IgG, IgA, IgD, IgE, and IgM. In certain embodiments, the anti-IL-6 antibody comprises a heavy chain constant region of the class IgG and a subclass selected from IgG1, IgG2, IgG3, and IgG4.

In some embodiments, the IL-6 antagonist is immunoconjugate or fusion protein comprising an IL-6 antigen-binding fragment.

In some embodiments, the antibody is bispecific or multispecific, with at least one of the antigen-binding portions having specificity for IL-6.

In some embodiments, the antibody is fully human. In some embodiments, the antibody is humanized. In some embodiments, the antibody is chimeric and has non-human V regions and human C region domains. In some embodiments, the antibody is murine.

In typical embodiments, the anti-IL-6 antibody has a K_{D} for binding human IL-6 of less than 100 nM. In some embodiments, the anti-IL-6 antibody has a K_{D} for binding human IL-6 of less than 75 nM, 50 nM, 25 nM, 20 nM, 15 nM, or 10 nM. In particular embodiments, the anti-IL-6 antibody has a K_{D} for binding human IL-6 of less than 5 nM, 4 nM, 3 nM, or 2 nM. In selected embodiments, the anti-IL-6 antibody has a K_{D} for binding human IL-6 of less than 1 nM, 750 pM, or 500 pM. In specific embodiments, the anti-IL-6 antibody has a K_{D} for binding human IL-6 of no more than 500 pM, 400 pM, 300 pM, 200 pM, or 100 pM.

In typical embodiments, the anti-IL-6 antibody has an elimination half-life following intravenous administration of at least 7 days. In certain embodiments, the anti-IL-6 antibody has an elimination half-life of at least 14 days, at least 21 days, or at least 30 days.

In some embodiments, the anti-IL-6 antibody has a human IgG constant region with at least one amino acid substitution that extends serum half-life as compared to the unsubstituted human IgG constant domain.

In certain embodiments, the IgG constant domain comprises substitutions at residues 252, 254, and 256, wherein the amino acid substitution at amino acid residue 252 is a substitution with tyrosine, the amino acid substitution at amino acid residue 254 is a substitution with threonine, and the amino acid substitution at amino acid residue 256 is a substitution with glutamic acid ("YTE"). *See* U.S. Pat. No. 7,083,784, incorporated herein by reference in its entirety. In certain extended half-life embodiments, the IgG constant domain comprises substitutions selected from T250Q/M428L (Hinton et al., J. Immunology 176:346-356 (2006)); N434A (Yeung et al., J. Immunology 182:7663-7671 (2009)); or T307A/E380A/N434A (Petkova et al., International Immunology, 18: 1759-1769 (2006)).

In some embodiments, the elimination half-life of the anti-IL-6 antibody is increased by utilizing the FcRN-binding properties of human serum albumin. In certain embodiments, the antibody is conjugated to albumin (Smith et al., Bioconjug. Chem., 12: 750-756 (2001)). In some embodiments, the anti-IL-6 antibody is fused to bacterial albumin-binding domains (Stork et al., Prot. Eng. Design Science 20: 569-76 (2007)). In some embodiments, the anti-IL-6 antibody is fused to an albumin-binding peptide (Nguygen et al., Prot Eng Design Sel 19: 291-297 (2006)). In some embodiments, the anti-IL-6 antibody is bispecific, with one specificity being to IL-6, and one specificity being to human serum albumin (Ablynx, WO 2006/122825 (bispecific Nanobody)).

In some embodiments, the elimination half-life of the anti-IL-6 antibody is increased by PEGylation (Melmed et al., Nature Reviews Drug Discovery 7: 641-642 (2008)); by HPMA copolymer conjugation (Lu et al., Nature Biotechnology 17: 1101-1104 (1999)); by dextran conjugation (Nuclear Medicine Communications, 16: 362-369 (1995)); by conjugation with homo-amino-acid polymers (HAPs; HAPylation) (Schlapschy et al., Prot Eng Design Sel 20: 273-284 (2007)); or by polysialylation (Constantinou et al., Bioconjug. Chem. 20: 924-931 (2009)).

### 2.7.1.1. COR-001 and Derivatives

In certain preferred embodiments, the anti-IL-6 antibody or antigen-binding portion thereof comprises all six CDRs of COR-001. The COR-001 antibody (also known as "ziltivekimab" and "MEDI5117") is described in WO 2010/088444 and US 2012/0034212, the disclosures of which are incorporated herein by reference in their entireties. In particular embodiments, the antibody or antigen-binding portion thereof comprises the COR-001 heavy chain V region and light chain V region. In specific embodiments, the antibody is the full-length COR-001 antibody. The COR-001 antibody has the following CDR and heavy and light chain sequences:
COR-001 VH CDR1
   SNYMI (SEQ ID NO:7)
COR-001 VH CDR2
   DLYYYAGDTYYADSVKG (SEQ ID NO:8)
COR-001 VH CDR3
   WADDHPPWIDL (SEQ ID NO:9)
COR-001 VL CDR1
   RASQGISSWLA (SEQ ID NO:10)
COR-001 VL CDR2
   KASTLES (SEQ ID NO:11)
COR-001 VL CDR3
   QQSWLGGS (SEQ ID NO:12)
COR-001 Heavy chain 
EVQLVESGGGLVQPGGSLRLSCAASGFTISSNYMIWVRQAPGKGLEWVSDLYYYAGDTYY
ADSVKGRFTMSRDISKNTVYLQMNSLRAEDTAVYYCARWADDHPPWIDLWGRGTLVTVSS
ASTKGPSVFPLAPSSKSTSGGTAALGCLVKDYFPEPVTVSWNSGALTSGVHTFPAVLQSS
GLYSLSSVVTVPSSSLGTQTYICNVNHKPSNTKVDKRVEPKSCDKTHTCPPCPAPELLGG
PSVFLFPPKPKDTLYITREPEVTCVVVDVSHEDPEVKFNWYVDGVEVHNAKTKPREEQYN
STYRVVSVLTVLHQDWLNGKEYKCKVSNKALPAPIEKTISKAKGQPREPQVYTLPPSREE
MTKNQVSLTCLVKGFYPSDIAVEWESNGQPENNYKTTPPVLDSDGSFFLYSKLTVDKSRW
QQGNVFSCSVMHEALHNHYTQKSLSLSPGK (SEQ ID NO:13)
COR-001 Light chain

DIQMTQSPSTLSASVGDRVTITCRASQGISSWLAWYQQKPGKAPKVLIYKASTLESGVPS
RFSGSGSGTEFTLTISSLQPDDFATYYCQQSWLGGSFGQGTKLEIKRTVAAPSVFIFPPS
DEQLKSGTASVVCLLNNFYPREAKVQWKVDNALQSGNSQESVTEQDSKDSTYSLSSTLTL
SKADYEKHKVYACEVTHQGLSSPVTKSFNRGEC (SEQ ID NO:14)

In various embodiments, the anti-IL-6 antibody is a derivative of COR-001.

In some embodiments, the COR-001 derivative includes one or more amino acid substitutions in the COR-001 heavy and/or light chain V regions.

In certain embodiments, the COR-001 derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the COR-001 anti-IL-6 antibody, while retaining specificity for human IL-6.

In certain embodiments, the COR-001 derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of COR-001. The percent sequence identity is determined using BLAST algorithms using default parameters.

In certain embodiments, the COR-001 derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of COR-001. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6).

### 2.7.1.2. Siltuximab and Derivatives

In certain embodiments, the anti-IL-6 antibody or antigen-binding portion thereof comprises all six CDRs of siltuximab. In particular embodiments, the antibody or antigen-binding portion thereof comprises the siltuximab heavy chain V region and light chain V region. In specific embodiments, the antibody is the full-length siltuximab antibody.

In various embodiments, the anti-IL-6 antibody is a derivative of siltuximab.

In some embodiments, the siltuximab derivative includes one or more amino acid substitutions in the siltuximab heavy and/or light chain V regions.

In certain embodiments, the siltuximab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the siltuximab anti-IL-6 antibody, while retaining specificity for human IL-6.

In certain embodiments, the siltuximab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of siltuximab. The percent sequence identity is determined using BLAST algorithms using default parameters.

In certain embodiments, the siltuximab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of siltuximab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6).

### 2.7.1.3. Gerilizumab and Derivatives

In certain embodiments, the anti-IL-6 antibody or antigen-binding portion thereof comprises all six CDRs of gerilizumab. In particular embodiments, the antibody or antigen-binding portion thereof comprises the gerilizumab heavy chain V region and light chain V region. In specific embodiments, the antibody is the full-length gerilizumab antibody.

In various embodiments, the anti-IL-6 antibody is a derivative of gerilizumab.

In some embodiments, the gerilizumab derivative includes one or more amino acid substitutions in the gerilizumab heavy and/or light chain V regions.

In certain embodiments, the gerilizumab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the gerilizumab anti-IL-6 antibody, while retaining specificity for human IL-6.

In certain embodiments, the gerilizumab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of gerilizumab. The percent sequence identity is determined using BLAST algorithms using default parameters.

In certain embodiments, the gerilizumab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of gerilizumab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6).

### 2.7.1.4. Sirukumab and Derivatives

In certain embodiments, the anti-IL-6 antibody or antigen-binding portion thereof comprises all six CDRs of sirukumab. In particular embodiments, the antibody or antigen-binding portion thereof comprises the sirukumab heavy chain V region and light chain V region. In specific embodiments, the antibody is the full-length sirukumab antibody.

In various embodiments, the anti-IL-6 antibody is a derivative of sirukumab.

In some embodiments, the sirukumab derivative includes one or more amino acid substitutions in the sirukumab heavy and/or light chain V regions.

In certain embodiments, the sirukumab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the sirukumab anti-IL-6 antibody, while retaining specificity for human IL-6.

In certain embodiments, the sirukumab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of sirukumab. The percent sequence identity is determined using BLAST algorithms using default parameters.

In certain embodiments, the sirukumab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of sirukumab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6).

### 2.7.1.5. Clazakizumab and Derivatives

In certain embodiments, the anti-IL-6 antibody or antigen-binding portion thereof comprises all six CDRs of clazakizumab. In particular embodiments, the antibody or antigen-binding portion thereof comprises the clazakizumab heavy chain V region and light chain V region. In specific embodiments, the antibody is the full-length clazakizumab antibody.

In various embodiments, the anti-IL-6 antibody is a derivative of clazakizumab.

In some embodiments, the clazakizumab derivative includes one or more amino acid substitutions in the clazakizumab heavy and/or light chain V regions.

In certain embodiments, the clazakizumab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the clazakizumab anti-IL-6 antibody, while retaining specificity for human IL-6.

In certain embodiments, the clazakizumab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of clazakizumab. The percent sequence identity is determined using BLAST algorithms using default parameters.

In certain embodiments, the clazakizumab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of clazakizumab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6).

### 2.7.1.6. Olokizumab and Derivatives

In certain embodiments, the anti-IL-6 antibody or antigen-binding portion thereof comprises all six CDRs of olokizumab. In particular embodiments, the antibody or antigen-binding portion thereof comprises the olokizumab heavy chain V region and light chain V region. In specific embodiments, the antibody is the full-length olokizumab antibody.

In various embodiments, the anti-IL-6 antibody is a derivative of olokizumab.

In some embodiments, the olokizumab derivative includes one or more amino acid substitutions in the olokizumab heavy and/or light chain V regions.

In certain embodiments, the olokizumab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the olokizumab anti-IL-6 antibody, while retaining specificity for human IL-6.

In certain embodiments, the olokizumab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of olokizumab. The percent sequence identity is determined using BLAST algorithms using default parameters.

In certain embodiments, the olokizumab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of olokizumab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6).

### 2.7.1.7. Other Anti-IL-6 Antibodies and Derivatives

In certain embodiments, the anti-IL-6 antibody or antigen-binding portion thereof comprises all six CDRs of an antibody selected from the group consisting of: VX30 (VOP-R003; Vaccinex), EB-007 (EBI-029; Eleven Bio), and FM101. In particular embodiments, the antibody or antigen-binding portion thereof comprises the heavy chain V region and light chain V region of an antibody selected from the group consisting of: VX30 (VOP-R003; Vaccinex), EB-007 (EBI-029; Eleven Bio), and FM101. In specific embodiments, the antibody is a full-length antibody selected from the group consisting of: VX30 (VOP-R003; Vaccinex), EB-007 (EBI-029; Eleven Bio), and FM101.

In various embodiments, the anti-IL-6 antibody is a derivative of an antibody selected from the group consisting of: VX30 (VOP-R003; Vaccinex), EB-007 (EBI-029; Eleven Bio), and FM101.

### 2.7.2. Anti-IL-6 Receptor Antibodies

In various embodiments, the IL-6 antagonist is an anti-IL-6 receptor (anti-IL-6R) antibody or antigen-binding fragment or derivative thereof.

In typical embodiments, the anti-IL-6R reduces the biological activity of IL-6 receptor.

In some embodiments, the IL-6 antagonist is an anti-IL-6R monoclonal antibody. In some embodiments, the IL-6 antagonist is a polyclonal composition comprising a plurality of species of anti-IL-6R antibodies, each of the plurality having unique CDRs.

In some embodiments, the anti-IL-6R antibody is a Fab, Fab', F(ab')₂ , Fv, scFv, (scFv)₂, single chain antibody molecule, dual variable domain antibody, single variable domain antibody, linear antibody, or V domain antibody.

In some embodiments, the anti-IL-6R antibody comprises a scaffold. In certain embodiments, the scaffold is Fc, optionally human Fc. In some embodiments, the anti-IL-6R antibody comprises a heavy chain constant region of a class selected from IgG, IgA, IgD, IgE, and IgM. In certain embodiments, the anti-IL-6R antibody comprises a heavy chain constant region of the class IgG and a subclass selected from IgG1, IgG2, IgG3, and IgG4.

In some embodiments, the IL-6 antagonist is immunoconjugate or fusion protein comprising an IL-6R antigen-binding fragment.

In some embodiments, the antibody is bispecific or multispecific, with at least one of the antigen-binding portions having specificity for IL-6 receptor.

In some embodiments, the antibody is fully human. In some embodiments, the antibody is humanized. In some embodiments, the antibody is chimeric and has non-human V regions and human C region domains. In some embodiments, the antibody is murine.

In typical embodiments, the anti-IL-6R antibody has a K_{D} for binding human IL-6 receptor of less than 100 nM. In some embodiments, the anti-IL-6R antibody has a K_{D} for binding human IL-6 receptor of less than 75 nM, 50 nM, 25 nM, 20 nM, 15 nM, or 10 nM. In particular embodiments, the anti-IL-6R antibody has a K_{D} for binding human IL-6 receptor of less than 5 nM, 4 nM, 3 nM, or 2 nM. In selected embodiments, the anti-IL-6R antibody has a K_{D} for binding human IL-6 receptor of less than 1 nM, 750 pM, or 500 pM. In specific embodiments, the anti-II,-6R antibody has a K_{D} for binding human IL-6 receptor of no more than 500 pM, 400 pM, 300 pM, 200 pM, or 100 pM.

In typical embodiments, the anti-IL-6R antibody has an elimination half-life following intravenous administration of at least 7 days. In certain embodiments, the anti-IL-6R antibody has an elimination half-life of at least 14 days, at least 21 days, or at least 30 days.

In some embodiments, the anti-IL-6R antibody has a human IgG constant region with at least one amino acid substitution that extends serum half-life as compared to the unsubstituted human IgG constant domain.

In certain embodiments, the IgG constant domain comprises substitutions at residues 252, 254, and 256, wherein the amino acid substitution at amino acid residue 252 is a substitution with tyrosine, the amino acid substitution at amino acid residue 254 is a substitution with threonine, and the amino acid substitution at amino acid residue 256 is a substitution with glutamic acid ("YTE"). *See* U.S. Pat. No. 7,083,784, incorporated herein by reference in its entirety. In certain extended half-life embodiments, the IgG constant domain comprises substitutions selected from T250Q/M428L (Hinton et al., J. Immunology 176:346-356 (2006)); N434A (Yeung et al., J. Immunology 182:7663-7671 (2009)); or T307A/E380A/N434A (Petkova et al., International Immunology, 18: 1759-1769 (2006)).

In some embodiments, the elimination half-life of the anti-IL-6R antibody is increased by utilizing the FcRN-binding properties of human serum albumin. In certain embodiments, the antibody is conjugated to albumin (Smith et al., Bioconjug. Chem., 12: 750-756 (2001)). In some embodiments, the anti-IL-6R antibody is fused to bacterial albumin-binding domains (Stork et al., Prot. Eng. Design Science 20: 569-76 (2007)). In some embodiments, the anti-IL-6R antibody is fused to an albumin-binding peptide (Nguygen et al., Prot Eng Design Sel 19: 291-297 (2006)). In some embodiments, the anti-IL-6R antibody is bispecific, with one specificity being to IL-6 receptor, and one specificity being to human serum albumin (Ablynx, WO 2006/122825 (bispecific Nanobody)).

In some embodiments, the elimination half-life of the anti-IL-6R antibody is increased by PEGylation (Melmed et al., Nature Reviews Drug Discovery 7: 641-642 (2008)); by HPMA copolymer conjugation (Lu et al., Nature Biotechnology 17: 1101-1104 (1999)); by dextran conjugation (Nuclear Medicine Communications, 16: 362-369 (1995)); by conjugation with homo-amino-acid polymers (HAPs; HAPylation) (Schlapschy et al., Prot Eng Design Sel 20: 273-284 (2007)); or by polysialylation (Constantinou et al., Bioconjug. Chem. 20: 924-931 (2009)).

### 2.7.2.1. Tocilizumab and Derivatives

In certain embodiments, the anti-IL-6R antibody or antigen-binding portion thereof comprises all six CDRs of tocilizumab. In particular embodiments, the antibody or antigen-binding portion thereof comprises the tocilizumab heavy chain V region and light chain V region. In specific embodiments, the antibody is the full-length tocilizumab antibody.

In various embodiments, the anti-IL-6R antibody is a derivative of tocilizumab.

In some embodiments, the tocilizumab derivative includes one or more amino acid substitutions in the tocilizumab heavy and/or light chain V regions.

In certain embodiments, the tocilizumab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the tocilizumab anti-IL-6R antibody, while retaining specificity for human IL-6 receptor.

In certain embodiments, the tocilizumab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of tocilizumab. The percent sequence identity is determined using BLAST algorithms using default parameters.

In certain embodiments, the tocilizumab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of tocilizumab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6 receptor).

### 2.7.2.2. Sarilumab and Derivatives

In certain embodiments, the anti-IL-6R antibody or antigen-binding portion thereof comprises all six CDRs of sarilumab. In particular embodiments, the antibody or antigen-binding portion thereof comprises the sarilumab heavy chain V region and light chain V region. In specific embodiments, the antibody is the full-length sarilumab antibody.

In various embodiments, the anti-IL-6R antibody is a derivative of sarilumab.

In some embodiments, the sarilumab derivative includes one or more amino acid substitutions in the sarilumab heavy and/or light chain V regions.

In certain embodiments, the sarilumab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the sarilumab anti-IL-6R antibody, while retaining specificity for human IL-6 receptor.

In certain embodiments, the sarilumab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of sarilumab. The percent sequence identity is determined using BLAST algorithms using default parameters.

In certain embodiments, the sarilumab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of sarilumab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6 receptor).

### 2.7.2.3. Vobarilizumab and Derivatives

In certain embodiments, the anti-IL-6R antibody or antigen-binding portion thereof comprises all six CDRs of vobarilizumab. In particular embodiments, the antibody or antigen-binding portion thereof comprises the vobarilizumab heavy chain V region and light chain V region. In specific embodiments, the antibody is the full-length vobarilizumab antibody.

In various embodiments, the anti-IL-6R antibody is a derivative of vobarilizumab.

In some embodiments, the vobarilizumab derivative includes one or more amino acid substitutions in the vobarilizumab heavy and/or light chain V regions.

In certain embodiments, the vobarilizumab derivative comprises fewer than 25 amino acid substitutions, fewer than 20 amino acid substitutions, fewer than 15 amino acid substitutions, fewer than 10 amino acid substitutions, fewer than 5 amino acid substitutions, fewer than 4 amino acid substitutions, fewer than 3 amino acid substitutions, fewer than 2 amino acid substitutions, or 1 amino acid substitution relative to the original V_{H} and/or V_{L} of the vobarilizumab anti-IL-6R antibody, while retaining specificity for human IL-6 receptor.

In certain embodiments, the vobarilizumab derivative comprises an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the V_{H} and V_{L} domain of vobarilizumab. The percent sequence identity is determined using BLAST algorithms using default parameters.

In certain embodiments, the vobarilizumab derivative comprises an amino acid sequence in which the CDRs comprise an amino acid sequence that is at least 45%, at least 50%, at least 55%, at least 60%, at least 65%, at least 70%, at least 75%, at least 80%, at least 85%, at least 90%, at least 95%, or at least 99% identical to the amino acid sequence of the respective CDRs of vobarilizumab. The percent sequence identity is determined using BLAST algorithms using default parameters.

In certain embodiments, the V_{H} and/or V_{L} CDR derivatives comprise conservative amino acid substitutions at one or more predicted nonessential amino acid residues (i.e., amino acid residues which are not critical for the antibody to specifically bind to human IL-6 receptor).

### 2.7.2.4. Other Anti-IL-6R Antibodies and Derivatives

In certain embodiments, the anti-IL-6R antibody or antigen-binding portion thereof comprises all six CDRs of an antibody selected from the group consisting of: SA237 (Roche), NI-1201 (NovImmune), and an antibody described in US 2012/0225060. In particular embodiments, the antibody or antigen-binding portion thereof comprises the heavy chain V region and light chain V region of an antibody selected from the group consisting of: SA237 (Roche), NI-1201 (NovImmune), and an antibody described in US 2012/0225060. In specific embodiments, the antibody is a full-length selected from the group consisting of: SA237 (Roche), NI-1201 (NovImmune), and an antibody described in US 2012/0225060.

In various embodiments, the anti-IL-6R antibody is a derivative of an antibody selected from the group consisting of: SA237 (Roche), NI-1201 (NovImmune), or an antibody described in US 2012/0225060.

### 2.7.3. Anti-IL-6:IL-6R Complex Antibodies

In various embodiments, the IL-6 antagonist is an antibody specific for the complex of IL-6 and IL-6R. In certain embodiments, the antibody has the six CDRs of an antibody selected from those described in US 2011/0002936, which is incorporated herein by reference in its entirety.

### 2.7.4. JAK and STAT Inhibitors

In various embodiments, the IL-6 antagonist is an inhibitor of the JAK signaling pathway. In some embodiments, the JAK inhibitor is a JAK1-specific inhibitor. In some embodiments, the JAK inhibitor is a JAK3-specific inhibitor. In some embodiments, the JAK inhibitor is a pan-JAK inhibitor.

In certain embodiments, the JAK inhibitor is selected from the group consisting of tofacitinib (Xeljanz), decernotinib, ruxolitinib, upadacitinib, baricitinib, filgotinib, lestaurtinib, pacritinib, peficitinib, INCB-039110, ABT-494, INCB-047986 and AC-410.

In various embodiments, the IL-6 antagonist is a STAT3 inhibitor. In a specific embodiment, the inhibitor is AZD9150 (AstraZeneca, Isis Pharmaceuticals), a STAT3 antisense molecule.

### 2.7.5. Additional IL-6 Antagonists

In various embodiments, the IL-6 antagonist is an antagonist peptide.

In certain embodiments, the IL-6 antagonist is C326 (an IL-6 inhibitor by Avidia, also known as AMG220), or FE301, a recombinant protein inhibitor of IL-6 (Ferring International Center S.A., Conaris Research Institute AG). In some embodiments, the anti-IL-6 antagonist comprises soluble gp130, FE301 (Conaris/Ferring).

### 2.8. Pharmaceutical Composition

The IL-6 antagonists used in the methods described herein can be formulated in any appropriate pharmaceutical composition for administration by any suitable route of administration. Suitable routes of administration include, but are not limited to, the intravitreal, intraarterial, intradermal, intramuscular, intraperitoneal, intravenous, nasal, parenteral, pulmonary, and subcutaneous routes.

The pharmaceutical composition may comprise one or more pharmaceutical excipients. Any suitable pharmaceutical excipient may be used, and one of ordinary skill in the art is capable of selecting suitable pharmaceutical excipients. Accordingly, the pharmaceutical excipients provided below are intended to be illustrative, and not limiting. Additional pharmaceutical excipients include, for example, those described in the Handbook of Pharmaceutical Excipients, Rowe et al. (Eds.) 6th Ed. (2009), incorporated by reference in its entirety.

### 2.9. Dosage Regimens

The IL-6 antagonist is administered at a dose sufficient to reduce inflammation without causing immune suppression.

### 2.9.1. Antibodies, Antigen-Binding Fragments, Peptides

In typical embodiments, antibody, antigen-binding fragments, and peptide IL-6 antagonists are administered parenterally.

In some parenteral embodiments, the IL-6 antagonist is administered intravenously. In certain intravenous embodiments, the IL-6 antagonist is administered as a bolus. In certain intravenous embodiments, the IL-6 antagonist is administered as an infusion. In certain intravenous embodiments, the IL-6 antagonist is administered as a bolus followed by infusion.

In some parenteral embodiments, the IL-6 antagonist is administered subcutaneously.

In various embodiments, the antibody, antigen-binding fragment, or peptide IL-6 antagonist is administered in a dose that is independent of patient weight or surface area (flat dose).

In some embodiments, the intravenous flat dose is 0.1 mg, 0.2 mg, 0.3 mg, 0.4 mg, 0.5 mg, 0.6 mg, 0.7 mg, 0.8 mg, 0.9 mg, or 1 mg. In some embodiments, the intravenous flat dose is 1 mg, 2 mg, 3 mg, 4 mg, 5 mg, 6 mg, 7 mg, 8 mg, 9 mg, or 10 mg. In some embodiments, the intravenous flat dose is 11 mg, 12 mg, 13 mg, 14 mg, 15 mg, 16 mg, 17 mg, 18 mg, 19 mg, or 20 mg. In some embodiments, the intravenous flat dose is 25 mg, 30 mg, 40 mg, or 50 mg. In some embodiments, the intravenous flat dose is 60 mg, 70 mg, 80 mg, 90 mg, or 100 mg. In some embodiments, the intravenous flat dose is 200 mg, 300 mg, 400 mg, or 500 mg. In some embodiments, the intravenous flat dose is 0.1 - 1 mg, 1 - 10 mg, 10 - 15 mg, 15 - 20 mg, 20 - 30 mg, 30 - 40 mg, or 40 - 50 mg. In some embodiments, the intravenous flat dose is 1 - 50 mg, 50 - 100 mg, or 100 mg - 500 mg.

In some embodiments, the subcutaneous flat dose is 0.1 mg, 0.2 mg, 0.3 mg, 0.4 mg, 0.5 mg, 0.6 mg, 0.7 mg, 0.8 mg, 0.9 mg, or 1 mg. In some embodiments, the subcutaneous flat dose is 1 mg, 2 mg, 3 mg, 4 mg, 5 mg, 6 mg, 7 mg, 8 mg, 9 mg, or 10 mg. In some embodiments, the subcutaneous flat dose is 11 mg, 12 mg, 13 mg, 14 mg, 15 mg, 16 mg, 17 mg, 18 mg, 19 mg, or 20 mg. In some embodiments, the subcutaneous flat dose is 25 mg, 30 mg, 40 mg, or 50 mg. In some embodiments, the subcutaneous flat dose is 60 mg, 70 mg, 80 mg, 90 mg, or 100 mg. In some embodiments, the subcutaneous flat dose is 200 mg, 300 mg, 400 mg, or 500 mg. In some embodiments, the subcutaneous flat dose is 0.1 - 1 mg, 1 - 10 mg, 10- 15 mg, 15 - 20 mg, 20 - 30 mg, 30 - 40 mg, or 40 - 50 mg. In some embodiments, the subcutaneous flat dose is 1 - 50 mg, 50- 100 mg, or 100 mg - 500 mg.

In various embodiments, the antibody, antigen-binding fragment, or peptide IL-6 antagonist is administered as a patient weight-based dose.

In some embodiments, the antagonist is administered at an intravenous dose of 0.01 mg/kg, 0.02 mg/kg, 0.03 mg/kg, 0.04 mg/kg, 0.05 mg/kg, 0.06 mg/kg, 0.07 mg/kg, 0.08 mg/kg, 0.09 mg/kg or 0.1 mg/kg. In some embodiments, the antagonist is administered at an intravenous dose of 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg or 1.0 mg/kg. In some embodiments, the antagonist is administered at an intravenous dose of 1.5 mg/kg, 2 mg/kg, 2.5 mg/kg, 3 mg/kg, 3.5 mg/kg, 4 mg/kg, 4.5 mg/kg, or 5 mg/kg.

In some embodiments, the antagonist is administered at a subcutaneous dose of 0.01 mg/kg, 0.02 mg/kg, 0.03 mg/kg, 0.04 mg/kg, 0.05 mg/kg, 0.06 mg/kg, 0.07 mg/kg, 0.08 mg/kg, 0.09 mg/kg or 0.1 mg/kg. In some embodiments, the antagonist is administered at a subcutaneous dose of 0.1 mg/kg, 0.2 mg/kg, 0.3 mg/kg, 0.4 mg/kg, 0.5 mg/kg, 0.6 mg/kg, 0.7 mg/kg, 0.8 mg/kg, 0.9 mg/kg or 1.0 mg/kg. In some embodiments, the antagonist is administered at a subcutaneous dose of 1.5 mg/kg, 2 mg/kg, 2.5 mg/kg, 3 mg/kg, 3.5 mg/kg, 4 mg/kg, 4.5 mg/kg, or 5 mg/kg.

In various intravenous embodiments, the IL-6 antagonist is administered once every 7 days, once every 14 days, once every 21 days, once every 28 days, or once a month. In various subcutaneous embodiments, the IL-6 antagonist is administered once every 14 days, once every 28 days, once a month, once every two months (every other month), or once every three months.

### 2.9.2. Small Molecule Inhibitors

In typical embodiments, small molecule JAK inhibitors and STAT inhibitors are administered orally.

In various embodiments, the inhibitor is administered once or twice a day at an oral dose of 0.1 - 1 mg, 1 - 10 mg, 10 - 20 mg, 20 - 30 mg, 30 - 40 mg, or 40 - 50 mg. In some embodiments, the inhibitor is administered once or twice a day at a dose of 50 - 60 mg, 60 - 70 mg, 70 - 80 mg, 80 - 90 mg, or 90 - 100 mg. In some embodiments, the inhibitor is administered at a dose of 0.1, 0.5, 1, 2, 5, 10, 15, 20, 25, 30, 35, 40, 45, or 50 mg PO once or twice a day. In some embodiments, the inhibitor is administered at a dose of 75 mg or 100 mg PO once or twice a day.

### 2.9.3. Monthly Equivalent Dose

In typical embodiments, the IL-6 antagonist is administered at a monthly equivalent dose that is less than the monthly equivalent dose for treating rheumatoid arthritis with the same IL-6 antagonist. "Monthly equivalent dose" is the calculated total dose administered per month, regardless of dose amount and dosage schedule.

In some embodiments, the IL-6 antagonist is administered at a monthly equivalent dose no more than 50% of a monthly equivalent dose for treating rheumatoid arthritis with the same IL-6 antagonist. In various embodiments, the IL-6 antagonist is administered at a monthly equivalent dose no more than 45%, 40%, 35%, 30%, 25%, 20%, 15%, 10%, or 5% of a monthly equivalent dose for treating rheumatoid arthritis with the same IL-6 antagonist. In certain embodiments, the IL-6 antagonist is administered at a monthly equivalent dose no more than 45% of a monthly equivalent dose for treating rheumatoid arthritis. In certain embodiments, the IL-6 antagonist is administered at a monthly equivalent dose no more than 40% of a monthly equivalent dose for treating rheumatoid arthritis. In certain embodiments, the IL-6 antagonist is administered at a monthly equivalent dose no more than 30% of a monthly equivalent dose for treating rheumatoid arthritis. In certain embodiments, the IL-6 antagonist is administered at a monthly equivalent dose no more than 25% of a monthly equivalent dose for treating rheumatoid arthritis. In certain embodiments, the IL-6 antagonist is administered at a monthly equivalent dose no more than 20% of a monthly equivalent dose for treating rheumatoid arthritis. In certain embodiments, the IL-6 antagonist is administered at a monthly equivalent dose no more than 15% of a monthly equivalent dose for treating rheumatoid arthritis. In certain embodiments, the IL-6 antagonist is administered at a monthly equivalent dose no more than 10% of a monthly equivalent dose for treating rheumatoid arthritis. In certain embodiments, the IL-6 antagonist is administered at a monthly equivalent dose no more than 5% of a monthly equivalent dose for treating rheumatoid arthritis.

In various embodiments, the IL-6 antagonist is administered at a monthly equivalent dose about 50%, 45%, 40%, 35%, 30%, 25%, 20%, 15%, 10%, or 5% of a monthly equivalent dose for treating rheumatoid arthritis with the same IL-6 antagonist. In certain embodiments, the IL-6 antagonist is administered at a monthly equivalent dose about 50% of a monthly equivalent dose for treating rheumatoid arthritis. In certain embodiments, the IL-6 antagonist is administered at a monthly equivalent dose about 40% of a monthly equivalent dose for treating rheumatoid arthritis. In certain embodiments, the IL-6 antagonist is administered at a monthly equivalent dose about 30% of a monthly equivalent dose for treating rheumatoid arthritis. In certain embodiments, the IL-6 antagonist is administered at a monthly equivalent dose about 25% of a monthly equivalent dose for treating rheumatoid arthritis. In certain embodiments, the IL-6 antagonist is administered at a monthly equivalent dose about 20% of a monthly equivalent dose for treating rheumatoid arthritis. In certain embodiments, the IL-6 antagonist is administered at a monthly equivalent dose about 15% of a monthly equivalent dose for treating rheumatoid arthritis. In certain embodiments, the IL-6 antagonist is administered at a monthly equivalent dose about 10% of a monthly equivalent dose for treating rheumatoid arthritis. In certain embodiments, the IL-6 antagonist is administered at a monthly equivalent dose about 5% of a monthly equivalent dose for treating rheumatoid arthritis.

In some embodiments, the IL-6 antagonist is the COR-001 antibody. In various embodiments, COR-001 is administered intravenously at a monthly equivalent dose of 0.5 - 50 mg, such as 0.5 - 1 mg, 0.5 - 2 mg, 0.5 - 5 mg, 0.5 - 7.5 mg, 0.5 - 10 mg, 0.5-15 mg, 0.5 - 20 mg, 0.5 - 25 mg, 0.5 - 30 mg, 0.5 - 40 mg, 1 - 2 mg, 1 - 5 mg, 1 - 7.5 mg, 1 - 10 mg, 1 - 15 mg, 1-20 mg, 1 - 25 mg, 1-30 mg, 1-40 mg, 1 - 50 mg, 2 - 5 mg, 2 - 7.5 mg, 2 - 10 mg, 2 - 15 mg, 2-20 mg, 2-25 mg, 2-30 mg, 2-40 mg, 2 - 50 mg, 5 - 7.5 mg, 5 - 10 mg, 5 - 15 mg, 5-20 mg, 5-25 mg, 5-30 mg, 5-40 mg, 5 - 50 mg, 7.5 - 10 mg, 7.5-15 mg, 7.5 - 20 mg, 7.5 - 25 mg, 7.5 - 30 mg, 7.5 - 40 mg, 7.5 - 50 mg, 10-15 mg, 10 - 20 mg, 10 - 25 mg, 10 - 30 mg, 10 - 40 mg, 10 - 50 mg, 15 - 20 mg, 15 - 25 mg, 15 - 30 mg, 15 - 40 mg, 15 - 50 mg, 20 - 25 mg, 20 - 30 mg, 20 - 40 mg, 20 - 50 mg, 25 - 30 mg, 25 - 40 mg, 25 - 50 mg, 30 - 40 mg, 30 - 50 mg, or 40 - 50 mg. In certain embodiments, COR-001 is administered intravenously at a monthly equivalent dose of 2 - 40 mg. In certain embodiments, COR-001 is administered intravenously at a monthly equivalent dose of 7.5 - 30 mg. In certain embodiments, COR-001 is administered intravenously at a monthly equivalent dose of 7.5 - 15 mg. In certain embodiments, COR-001 is administered intravenously at a monthly equivalent dose of 15 - 30 mg.

In various embodiments, COR-001 is administered intravenously at a monthly equivalent dose of about 0.5 mg, 1 mg, 2 mg, 3 mg, 4 mg, 5 mg, 6 mg, 7 mg, 7.5 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 35 mg, 40 mg, 45 mg, or 50 mg. In certain embodiments, COR-001 is administered intravenously at a monthly equivalent dose of about 1 mg. In certain embodiments, COR-001 is administered intravenously at a monthly equivalent dose of about 2 mg. In certain embodiments, COR-001 is administered intravenously at a monthly equivalent dose of about 3 mg. In certain embodiments, COR-001 is administered intravenously at a monthly equivalent dose of about 4 mg. In certain embodiments, COR-001 is administered intravenously at a monthly equivalent dose of about 5 mg. In certain embodiments, COR-001 is administered intravenously at a monthly equivalent dose of about 6 mg. In certain embodiments, COR-001 is administered intravenously at a monthly equivalent dose of about 7.5 mg. In certain embodiments, COR-001 is administered intravenously at a monthly equivalent dose of about 10 mg. In certain embodiments, COR-001 is administered intravenously at a monthly equivalent dose of about 12 mg. In certain embodiments, COR-001 is administered intravenously at a monthly equivalent dose of about 15 mg. In certain embodiments, COR-001 is administered intravenously at a monthly equivalent dose of about 20 mg. In certain embodiments, COR-001 is administered intravenously at a monthly equivalent dose of about 25 mg. In certain embodiments, COR-001 is administered intravenously at a monthly equivalent dose of about 30 mg. In certain embodiments, COR-001 is administered intravenously at a monthly equivalent dose of about 40 mg.

In various embodiments, COR-001 is administered subcutaneously at a monthly equivalent dose of 1 - 100 mg, such as 1 - 2 mg, 1 - 5 mg, 1 - 7.5 mg, 1 - 10 mg, 1 - 15 mg, 1-20 mg, 1 - 25 mg, 1-30 mg, 1-40 mg, 1 - 50 mg, 1-70 mg, 1 - 100 mg, 2 - 5 mg, 2-7.5 mg, 2 - 10 mg, 2 - 15 mg, 2-20 mg, 2-25 mg, 2-30 mg, 2-40 mg, 2 - 50 mg, 2-70 mg, 2 - 100 mg, 3 - 5 mg, 3 - 7.5 mg, 3 - 10 mg, 3 - 15 mg, 3-20 mg, 3-25 mg, 3-30 mg, 3-40 mg, 3 - 50 mg, 3-70 mg, 3 - 100 mg, 5 - 7.5 mg, 5 - 10 mg, 5 - 15 mg, 5-20 mg, 5-25 mg, 5-30 mg, 5-40 mg, 5 - 50 mg, 5 - 70 mg, 5 - 100 mg, 7.5 - 10 mg, 7.5 - 15 mg, 7.5 - 20 mg, 7.5 - 25 mg, 7.5 - 30 mg, 7.5 - 40 mg, 7.5 - 50 mg, 7.5 - 70 mg, 7.5 - 100 mg, 10 - 15 mg, 10 - 20 mg, 10 - 25 mg, 10 - 30 mg, 10 - 40 mg, 10 - 50 mg, 10 - 70 mg, 10 - 100 mg, 15 - 20 mg, 15 - 25 mg, 15 - 30 mg, 15 - 40 mg, 15 - 50 mg, 15 - 70 mg, 15 - 100 mg, 20 - 25 mg, 20 - 30 mg, 20 - 40 mg, 20 - 50 mg, 20 - 70 mg, 20 - 100 mg, 25 - 30 mg, 25 - 40 mg, 25 - 50 mg, 25 - 70 mg, 25 - 100 mg, 30 - 40 mg, 30 - 50 mg, 30 - 70 mg, 30 - 100 mg, or 40 - 100 mg. In certain embodiments, COR-001 is administered subcutaneously at a monthly equivalent dose of 3 - 70 mg. In certain embodiments, COR-001 is administered subcutaneously at a monthly equivalent dose of 7.5 - 30 mg. In certain embodiments, COR-001 is administered subcutaneously at a monthly equivalent dose of 7.5 - 15 mg. In certain embodiments, COR-001 is administered subcutaneously at a monthly equivalent dose of 15 - 30 mg.

In various embodiments, COR-001 is administered subcutaneously at a monthly equivalent dose of about 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 35 mg, 40 mg, 45 mg, 50 mg, 70 mg, or 100 mg. In certain embodiments, COR-001 is administered subcutaneously at a monthly equivalent dose of about 1 mg. In certain embodiments, COR-001 is administered subcutaneously at a monthly equivalent dose of about 2 mg. In certain embodiments, COR-001 is administered subcutaneously at a monthly equivalent dose of about 3 mg. In certain embodiments, COR-001 is administered subcutaneously at a monthly equivalent dose of about 4 mg. In certain embodiments, COR-001 is administered subcutaneously at a monthly equivalent dose of about 5 mg. In certain embodiments, COR-001 is administered subcutaneously at a monthly equivalent dose of about 6 mg. In certain embodiments, COR-001 is administered subcutaneously at a monthly equivalent dose of about 7.5 mg. In certain embodiments, COR-001 is administered subcutaneously at a monthly equivalent dose of about 10 mg. In certain embodiments, COR-001 is administered subcutaneously at a monthly equivalent dose of about 12 mg. In certain embodiments, COR-001 is administered subcutaneously at a monthly equivalent dose of about 15 mg. In certain embodiments, COR-001 is administered subcutaneously at a monthly equivalent dose of about 17 mg. In certain embodiments, COR-001 is administered subcutaneously at a monthly equivalent dose of about 20 mg. In certain embodiments, COR-001 is administered subcutaneously at a monthly equivalent dose of about 25 mg. In certain embodiments, COR-001 is administered subcutaneously at a monthly equivalent dose of about 30 mg. In certain embodiments, COR-001 is administered subcutaneously at a monthly equivalent dose of about 35 mg. In certain embodiments, COR-001 is administered subcutaneously at a monthly equivalent dose of about 40 mg. In certain embodiments, COR-001 is administered subcutaneously at a monthly equivalent dose of about 70 mg. In certain embodiments, COR-001 is administered subcutaneously at a monthly equivalent dose of about 100 mg.

In some embodiments, the IL-6 antagonist is siltuximab. In various embodiments, siltuximab is administered intravenously at a monthly equivalent dose of 10 - 500 mg, such as 10 - 20 mg, 10 - 30 mg, 10 - 40 mg, 10 - 50 mg, 10 - 100 mg, 10 - 150 mg, 10 - 200 mg, 10 - 300 mg, 10 - 400 mg, 20 - 30 mg, 20 - 40 mg, 20 - 50 mg, 20 - 100 mg, 20- 150 mg, 20 - 200 mg, 20 - 300 mg, 20 - 400 mg, 20 - 500 mg, 30 - 40 mg, 30 - 50 mg, 30 - 100 mg, 30 - 150 mg, 30 - 200 mg, 30 - 300 mg, 30 - 400 mg, 30 - 500 mg, 40 - 50 mg, 40 - 100 mg, 40 - 150 mg, 40 - 200 mg, 40 - 250 mg, 40 - 300 mg, 40 - 400 mg, 40 - 500 mg, 50 - 100 mg, 50 - 150 mg, 50 - 200 mg, 50 - 300 mg, 50 - 400 mg, 50 - 500 mg, 100 - 150 mg, 100 - 200 mg, 100 - 300 mg, 100 - 400 mg, 100 - 500 mg, 150 - 200 mg, 150 - 300 mg, 150 - 400 mg, 150 - 500 mg, 200 - 300 mg, 200 - 400 mg, 200 - 500 mg, 300 - 400 mg, 300 - 500 mg, or 400 - 500 mg. In certain preferred embodiments, siltuximab is administered intravenously at a monthly equivalent dose of 50 - 500 mg. In various embodiments, siltuximab is administered intravenously at a monthly equivalent dose of about 10 mg, 20 mg, 30 mg, 40 mg, 50 mg, 100 mg, 150 mg, 200 mg, 300 mg, 400 mg, or 500 mg. In certain embodiments, siltuximab is administered intravenously at a monthly equivalent dose of about 50 mg. In certain embodiments, siltuximab is administered intravenously at a monthly equivalent dose of about 100 mg. In certain embodiments, siltuximab is administered intravenously at a monthly equivalent dose of about 150 mg. In certain embodiments, siltuximab is administered intravenously at a monthly equivalent dose of about 200 mg. In certain embodiments, siltuximab is administered intravenously at a monthly equivalent dose of about 300 mg. In certain embodiments, siltuximab is administered intravenously at a monthly equivalent dose of about 500 mg.

In various embodiments, siltuximab is administered subcutaneously at a monthly equivalent dose of 50 - 1000 mg, such as 50 - 80 mg, 50 - 100 mg, 50- 160 mg, 50 - 200 mg, 50 - 240 mg, 50 - 320 mg, 50 - 480 mg, 50 - 800 mg, 80 - 100 mg, 80 - 160 mg, 80 - 200 mg, 80 - 240 mg, 80 - 320 mg, 80 - 480 mg, 80 - 800 mg, 80 - 1000 mg, 100 - 160 mg, 100 - 200 mg, 100 - 240 mg, 100 - 320 mg, 100 - 480 mg, 100 - 800 mg, 100 - 1000 mg, 160 - 200 mg, 160 - 240 mg, 160 - 320 mg, 160 - 480 mg, 160 - 800 mg, 160 - 1000 mg, 200 - 240 mg, 200 - 320 mg, 200 - 480 mg, 200 - 800 mg, 200 - 1000 mg, 240 - 320 mg, 240 - 480 mg, 240 - 800 mg, 240 - 1000 mg, 320 - 480 mg, 320 - 800 mg, 320 - 1000 mg, 480 - 800 mg, 480 - 1000 mg, or 800 - 1000 mg. In certain preferred embodiments, siltuximab is administered subcutaneously at a monthly equivalent dose of 80 - 800 mg. In various embodiments, siltuximab is administered subcutaneously at a monthly equivalent dose of about 50 mg, 80 mg. 100 mg, 160 mg, 240 mg. 320 mg, 480 mg. 800 mg, or 1000 mg. In certain embodiments, siltuximab is administered subcutaneously at a monthly equivalent dose of about 50 mg. In certain embodiments, siltuximab is administered subcutaneously at a monthly equivalent dose of about 80 mg. In certain embodiments, siltuximab is administered subcutaneously at a monthly equivalent dose of about 100 mg. In certain embodiments, siltuximab is administered subcutaneously at a monthly equivalent dose of about 160 mg. In certain embodiments, siltuximab is administered subcutaneously at a monthly equivalent dose of about 240 mg. In certain embodiments, siltuximab is administered subcutaneously at a monthly equivalent dose of about 320 mg. In certain embodiments, siltuximab is administered subcutaneously at a monthly equivalent dose of about 480 mg. In certain embodiments, siltuximab is administered subcutaneously at a monthly equivalent dose of about 800 mg. In certain embodiments, siltuximab is administered subcutaneously at a monthly equivalent dose of about 1000 mg.

In some embodiments, the IL-6 antagonist is gerilizumab. In various embodiments, gerilizumab is administered intravenously at a monthly equivalent dose of 0.05 - 2 mg, such as 0.05 - 0.075 mg, 0.05 - 0.1 mg, 0.05 - 0.12 mg, 0.05 - 0.3 mg, 0.05 - 0.6 mg, 0.05 - 0.9 mg, 0.05 - 1.8 mg, 0.075 - 0.1 mg, 0.075 - 0.12 mg, 0.075 - 0.3 mg, 0.075 - 0.6 mg, 0.075 - 0.9 mg, 0.075 - 1.8 mg, 0.075 - 2 mg, 0.1 - 0.12 mg, 0.1 - 0.3 mg, 0.1 - 0.6 mg, 0.1 - 0.9 mg, 0.1 - 1.8 mg, 0.1 - 2 mg, 0.12-0.3 mg, 0.12 - 0.6 mg, 0.12 - 0.9 mg, 0.12 - 1.8 mg, 0.12 - 2 mg, 0.3 - 0.6 mg, 0.3 - 0.9 mg, 0.3 - 1.8 mg, 0.3 - 2 mg, 0.6 - 0.9 mg, 0.6 - 1.8 mg, 0.6 - 2 mg, 0.9 - 1.8 mg, 0.9 - 2 mg, or 1.8 - 2 mg. In certain preferred embodiments, gerilizumab is administered intravenously at a monthly equivalent dose of 0.075 - 1.8 mg. In various embodiments, gerilizumab is administered intravenously at a monthly equivalent dose of about 0.05 mg, 0.075 mg, 0.1 mg, 0.12 mg, 0.3 mg, 0.6 mg, 0.9 mg, 1.8 mg, or 2 mg. In certain embodiments, gerilizumab is administered intravenously at a monthly equivalent dose of about 0.05 mg. In certain embodiments, gerilizumab is administered intravenously at a monthly equivalent dose of about 0.075 mg. In certain embodiments, gerilizumab is administered intravenously at a monthly equivalent dose of about 0.1 mg. In certain embodiments, gerilizumab is administered intravenously at a monthly equivalent dose of about 0.12 mg. In certain embodiments, gerilizumab is administered intravenously at a monthly equivalent dose of about 0.3 mg. In certain embodiments, gerilizumab is administered intravenously at a monthly equivalent dose of about 0.6 mg. In certain embodiments, gerilizumab is administered intravenously at a monthly equivalent dose of about 0.9 mg. In certain embodiments, gerilizumab is administered intravenously at a monthly equivalent dose of about 1.8 mg. In certain embodiments, gerilizumab is administered intravenously at a monthly equivalent dose of about 2 mg.

In various embodiments, gerilizumab is administered subcutaneously at a monthly equivalent dose of 0.1 - 5 mg, such as 0.1 - 0.125 mg, 0.1 - 0.15 mg, 0.1 - 0.2 mg, 0.1 - 0.5 mg, 0.1 - 1 mg, 0.1 - 1.5 mg, 0.1 - 2 mg, 0.1 - 3 mg, 0.1 - 4 mg, 0.125 - 0.15 mg, 0.125 - 0.2 mg, 0.125 - 0.5 mg, 0.125 - 1 mg, 0.125 - 1.5 mg, 0.125 - 2 mg, 0.125 - 3 mg, 0.125 - 4 mg, 0.125 - 5 mg, 0.15 - 0.2 mg, 0.15 - 0.5 mg, 0.15 - 1 mg, 0.15 - 1.5 mg, 0.15 - 2 mg, 0.15-3 mg, 0.15 - 4 mg, 0.15 - 5 mg, 0.2 - 0.5 mg, 0.2 - 1 mg, 0.2 - 1.5 mg, 0.2 - 2 mg, 0.2-3 mg, 0.2 - 4 mg, 0.2 - 5 mg, 0.5 - 1 mg, 0.5 - 1.5 mg, 0.5 - 2 mg, 0.5 - 3 mg, 0.5-4 mg, 0.5 - 5 mg, 1 - 1.5 mg, 1 - 2 mg, 1 - 3 mg, 1 - 4 mg, 1 - 5 mg, 1.5 - 2 mg, 1.5 - 3 mg, 1.5-4 mg, 1.5-5 mg, 2-3 mg, 2-4 mg, 2-5 mg, 3 - 4 mg, 3-5 mg, or 4 - 5 mg. In certain preferred embodiments, gerilizumab is administered subcutaneously at a monthly equivalent dose of 0.125 - 3 mg. In various embodiments, gerilizumab is administered subcutaneously at a monthly equivalent dose of about 0.1 mg, 0.125 mg, 0.15 mg, 0.2 mg, 0.5 mg, 1 mg, 1.5 mg, 2 mg, 3 mg, 4 mg, or 5 mg. In certain embodiments, gerilizumab is administered subcutaneously at a monthly equivalent dose of about 0.125 mg. In certain embodiments, gerilizumab is administered subcutaneously at a monthly equivalent dose of about 0.2 mg. In certain embodiments, gerilizumab is administered subcutaneously at a monthly equivalent dose of about 0.5 mg. In certain embodiments, gerilizumab is administered subcutaneously at a monthly equivalent dose of about 1 mg. In certain embodiments, gerilizumab is administered subcutaneously at a monthly equivalent dose of about 1.5 mg. In certain embodiments, gerilizumab is administered subcutaneously at a monthly equivalent dose of about 3 mg.

In some embodiments, the IL-6 antagonist is sirukumab. In various embodiments, sirukumab is administered intravenously at a monthly equivalent dose of 1 - 80 mg, such as 1 - 1.5 mg, 1-3 mg, 1-6 mg, 1-12 mg, 1-36 mg, 1-60 mg, 1.5-3 mg, 1.5-6 mg, 1.5 - 12 mg, 1.5 - 36 mg, 1.5 - 60 mg, 1.5 - 80 mg, 3 - 6 mg, 3 - 12 mg, 3-36 mg, 3-60 mg, 3 - 80 mg, 6 - 12 mg, 6 - 36 mg, 6 - 60 mg, 6 - 80 mg, 12 - 36 mg, 12 - 60 mg, 12 - 80 mg, 36 - 60 mg, 36 - 80 mg, or 60 - 80 mg. In certain preferred embodiments, sirukumab is administered intravenously at a monthly equivalent dose of 1.5 - 60 mg. In various embodiments, sirukumab is administered intravenously at a monthly equivalent dose of about 1 mg, 1.5 mg, 3 mg, 6 mg, 12 mg, 36 mg, 60 mg, or 80 mg. In certain embodiments, sirukumab is administered intravenously at a monthly equivalent dose of about 1 mg. In certain embodiments, sirukumab is administered intravenously at a monthly equivalent dose of about 1.5 mg. In certain embodiments, sirukumab is administered intravenously at a monthly equivalent dose of about 3 mg. In certain embodiments, sirukumab is administered intravenously at a monthly equivalent dose of about 6 mg. In certain embodiments, sirukumab is administered intravenously at a monthly equivalent dose of about 12 mg. In certain embodiments, sirukumab is administered intravenously at a monthly equivalent dose of about 36 mg. In certain embodiments, sirukumab is administered intravenously at a monthly equivalent dose of about 60 mg. In certain embodiments, sirukumab is administered intravenously at a monthly equivalent dose of about 80 mg.

In various embodiments, sirukumab is administered subcutaneously at a monthly equivalent dose of 1 - 100 mg, such as 1 - 2.5 mg, 1 - 5 mg, 1 - 10 mg, 1-20 mg, 1 - 30 mg, 1-40 mg, 1 - 50 mg, 1-60 mg, 2.5 - 5 mg, 2.5 - 10 mg, 2.5 - 20 mg, 2.5 - 30 mg, 2.5 - 40 mg, 2.5 - 50 mg, 2.5 - 60 mg, 2.5 - 100 mg, 5 - 10 mg, 5 - 20 mg, 5-30 mg, 5-40 mg, 5 - 50 mg, 5 - 60 mg, 5 - 100 mg, 10 - 20 mg, 10 - 30 mg, 10 - 40 mg, 10 - 50 mg, 10 - 60 mg, 10 - 100 mg, 20 - 30 mg, 20 - 40 mg, 20 - 50 mg, 20 - 60 mg, 20 - 100 mg, 30 - 40 mg, 30 - 50 mg, 30 - 60 mg, 30 - 100 mg, 40 - 50 mg, 40 - 60 mg, 40 - 100 mg, 50 - 60 mg, 50- 100 mg, or 60 - 100 mg. In certain preferred embodiments, sirukumab is administered subcutaneously at a monthly equivalent dose of 2.5 - 100 mg. In various embodiments, sirukumab is administered subcutaneously at a monthly equivalent dose of about 1 mg, 2.5 mg, 5 mg, 10 mg, 20 mg, 30 mg, 40 mg, 50 mg, 60 mg, or 100 mg. In certain embodiments, sirukumab is administered subcutaneously at a monthly equivalent dose of about 2.5 mg. In certain embodiments, sirukumab is administered subcutaneously at a monthly equivalent dose of about 5 mg. In certain embodiments, sirukumab is administered subcutaneously at a monthly equivalent dose of about 10 mg. In certain embodiments, sirukumab is administered subcutaneously at a monthly equivalent dose of about 20 mg. In certain embodiments, sirukumab is administered subcutaneously at a monthly equivalent dose of about 60 mg. In certain embodiments, sirukumab is administered subcutaneously at a monthly equivalent dose of about 100 mg.

In some embodiments, the IL-6 antagonist is clazakizumab. In various embodiments, clazakizumab is administered intravenously at a monthly equivalent dose of 1 - 80 mg, such as 1 - 3 mg, 1-6 mg, 1 - 12 mg, 1-24 mg, 1-36 mg, 1-60 mg, 3 - 6 mg, 3 - 12 mg, 3-24 mg, 3-36 mg, 3-60 mg, 3-80 mg, 6 - 12 mg, 6-24 mg, 6 - 36 mg, 6-60 mg, 6 - 80 mg, 12 - 24 mg, 12 - 36 mg, 12 - 60 mg, 12 - 80 mg, 24 - 36 mg, 24 - 60 mg, 24 - 80 mg, 36 - 60 mg, 36 - 80 mg, or 60 - 80 mg. In certain preferred embodiments, clazakizumab is administered intravenously at a monthly equivalent dose of 3 - 60 mg. In various embodiments, clazakizumab is administered intravenously at a monthly equivalent dose of about 1 mg, 3 mg, 6 mg, 12 mg, 24 mg, 36 mg, 60 mg or 80 mg. In certain embodiments, clazakizumab is administered intravenously at a monthly equivalent dose of about 1 mg. In certain embodiments, clazakizumab is administered intravenously at a monthly equivalent dose of about 3 mg. In certain embodiments, clazakizumab is administered intravenously at a monthly equivalent dose of about 6 mg. In certain embodiments, clazakizumab is administered intravenously at a monthly equivalent dose of about 12 mg. In certain embodiments, clazakizumab is administered intravenously at a monthly equivalent dose of about 24 mg. In certain embodiments, clazakizumab is administered intravenously at a monthly equivalent dose of about 36 mg. In certain embodiments, clazakizumab is administered intravenously at a monthly equivalent dose of about 60 mg. In certain embodiments, clazakizumab is administered intravenously at a monthly equivalent dose of about 80 mg.

In various embodiments, clazakizumab is administered subcutaneously at a monthly equivalent dose of 1 - 100 mg, such as 1 - 2 mg, 1-5 mg, 1 - 10 mg, 1-20 mg, 1 - 30 mg, 1-40 mg, 1 - 50 mg, 1-60 mg, 2 - 5 mg, 2 - 10 mg, 2-20 mg, 2-30 mg, 2-40 mg, 2 - 50 mg, 2-60 mg, 2 - 100 mg, 5 - 10 mg, 5-20 mg, 5-30 mg, 5-40 mg, 5-50 mg, 5 - 60 mg, 5 - 100 mg, 10 - 20 mg, 10 - 30 mg, 10 - 40 mg, 10 - 50 mg, 10 - 60 mg, 10 - 100 mg, 20 - 30 mg, 20 - 40 mg, 20 - 50 mg, 20 - 60 mg, 20 - 100 mg, 30 - 40 mg, 30 - 50 mg, 30 - 60 mg, 30 - 100 mg, 40 - 50 mg, 40 - 60 mg, 40 - 100 mg, 50 - 60 mg, 50 - 100 mg, or 60 - 100 mg. In certain preferred embodiments, clazakizumab is administered subcutaneously at a monthly equivalent dose of 5 - 100 mg. In various embodiments, clazakizumab is administered subcutaneously at a monthly equivalent dose of about 1 mg, 2 mg, 5 mg, 10 mg, 20 mg, 30 mg, 40 mg, 50 mg, 60 mg, or 100 mg. In certain embodiments, clazakizumab is administered subcutaneously at a monthly equivalent dose of about 5 mg. In certain embodiments, clazakizumab is administered subcutaneously at a monthly equivalent dose of about 10 mg. In certain embodiments, clazakizumab is administered subcutaneously at a monthly equivalent dose of about 20 mg. In certain embodiments, clazakizumab is administered subcutaneously at a monthly equivalent dose of about 40 mg. In certain embodiments, clazakizumab is administered subcutaneously at a monthly equivalent dose of about 60 mg. In certain embodiments, clazakizumab is administered subcutaneously at a monthly equivalent dose of about 100 mg.

In some embodiments, the IL-6 antagonist is olokizumab. In various embodiments, olokizumab is administered intravenously at a monthly equivalent dose of 1 - 80 mg, such as 1 - 1.8 mg, 1 - 3.6 mg, 1 - 9 mg, 1 - 18 mg, 1-45 mg, 1-60 mg, 1.8 - 3.6 mg, 1.8 - 9 mg, 1.8 - 18 mg, 1.8-45 mg, 1.8-60 mg, 1.8-80 mg, 3.6 - 9 mg, 3.6 - 18 mg, 3.6 - 45 mg, 3.6-60 mg, 3.6 - 80 mg, 9 - 18 mg, 9 - 45 mg, 9 - 60 mg, 9-80 mg, 18-45 mg, 18 - 60 mg, 18 - 80 mg, 45 - 60 mg, 45 - 80 mg, or 60 - 80 mg. In certain preferred embodiments, olokizumab is administered intravenously at a monthly equivalent dose of 1.8 - 60 mg. In various embodiments, olokizumab is administered intravenously at a monthly equivalent dose of about 1 mg, 1.8 mg, 3.6 mg, 9 mg, 18 mg. 45 mg, 60 mg, or 80 mg. In certain embodiments, olokizumab is administered intravenously at a monthly equivalent dose of about 1 mg. In certain embodiments, olokizumab is administered intravenously at a monthly equivalent dose of about 1.8 mg. In certain embodiments, olokizumab is administered intravenously at a monthly equivalent dose of about 3.6 mg. In certain embodiments, olokizumab is administered intravenously at a monthly equivalent dose of about 9 mg. In certain embodiments, olokizumab is administered intravenously at a monthly equivalent dose of about 18 mg. In certain embodiments, olokizumab is administered intravenously at a monthly equivalent dose of about 45 mg. In certain embodiments, olokizumab is administered intravenously at a monthly equivalent dose of about 60 mg. In certain embodiments, olokizumab is administered intravenously at a monthly equivalent dose of about 80 mg.

In various embodiments, olokizumab is administered subcutaneously at a monthly equivalent dose of 1 - 100 mg, such as 1 - 3 mg, 1-6 mg, 1 - 10 mg, 1 - 15 mg, 1-20 mg, 1-30 mg, 1 - 50 mg, 1-72 mg, 3 - 6 mg, 3 - 10 mg, 3 - 15 mg, 3-20 mg, 3-30 mg, 3 - 50 mg, 3-72 mg, 3 - 100 mg, 6 - 10 mg, 6 - 15 mg, 6-20 mg, 6 - 30 mg, 6 - 50 mg, 6 - 72 mg, 6 - 100 mg, 10 - 15 mg, 10 - 20 mg, 10 - 30 mg, 10 - 50 mg, 10 - 72 mg, 10 - 100 mg, 15 - 20 mg, 15 - 30 mg, 15 - 50 mg, 15 - 72 mg, 15 - 100 mg, 20 - 30 mg, 20 - 50 mg, 20 - 72 mg, 20 - 100 mg, 30 - 50 mg, 30 - 72 mg, 30 - 100 mg, 50 - 72 mg, 50 - 100 mg, or 72 - 100 mg. In certain preferred embodiments, olokizumab is administered subcutaneously at a monthly equivalent dose of 3 - 100 mg. In various embodiments, olokizumab is administered subcutaneously at a monthly equivalent dose of about 1 mg, 3 mg, 6 mg, 10 mg, 15 mg, 20 mg, 30 mg, 50 mg, 72 mg, or 100 mg. In certain embodiments, olokizumab is administered subcutaneously at a monthly equivalent dose of about 3 mg. In certain embodiments, olokizumab is administered subcutaneously at a monthly equivalent dose of about 6 mg. In certain embodiments, olokizumab is administered subcutaneously at a monthly equivalent dose of about 15 mg. In certain embodiments, olokizumab is administered subcutaneously at a monthly equivalent dose of about 30 mg. In certain embodiments, olokizumab is administered subcutaneously at a monthly equivalent dose of about 72 mg. In certain embodiments, olokizumab is administered subcutaneously at a monthly equivalent dose of about 100 mg.

In some embodiments, the IL-6 antagonist is tocilizumab. In various embodiments, tocilizumab is administered intravenously at a monthly equivalent dose of 10 - 500 mg, such as 10 - 20 mg, 10 - 50 mg, 10 - 100 mg, 10 - 150 mg, 10 - 200 mg, 10 - 250 mg, 10 - 300 mg, 10 - 350 mg, 10 - 400 mg, 20 - 50 mg, 20 - 100 mg, 20- 150 mg, 20 - 200 mg, 20 - 250 mg, 20 - 300 mg, 20 - 350 mg, 20 - 400 mg, 20 - 500 mg, 50 - 100 mg, 50 - 150 mg, 50 - 200 mg, 50 - 250 mg, 50 - 300 mg, 50 - 350 mg, 50 - 400 mg, 50 - 500 mg, 100 - 150 mg, 100 - 200 mg, 100 - 250 mg, 100 - 300 mg, 100 - 350 mg, 100 - 400 mg, 100 - 500 mg, 150 - 200 mg, 150 - 250 mg, 150 - 300 mg, 150 - 350 mg, 150 - 400 mg, 150 - 500 mg, 200 - 250 mg, 200 - 300 mg, 200 - 350 mg, 200 - 400 mg, 200 - 500 mg, 250 - 300 mg, 250 - 350 mg, 250 - 400 mg, 250 - 500 mg, 300 - 350 mg, 300 - 400 mg, 300 - 500 mg, 350 - 400 mg, 350 - 500 mg, or 400 - 500 mg. In certain preferred embodiments, tocilizumab is administered intravenously at a monthly equivalent dose of 50 - 500 mg. In various embodiments, tocilizumab is administered intravenously at a monthly equivalent dose of about 10 mg, 20 mg, 30 mg, 40 mg, 50 mg, 100 mg, 150 mg, 200 mg, 250 mg, 300 mg, 350 mg, 400 mg, or 500 mg. In certain embodiments, tocilizumab is administered intravenously at a monthly equivalent dose of about 50 mg. In certain embodiments, tocilizumab is administered intravenously at a monthly equivalent dose of about 100 mg. In certain embodiments, tocilizumab is administered intravenously at a monthly equivalent dose of about 150 mg. In certain embodiments, tocilizumab is administered intravenously at a monthly equivalent dose of about 250 mg. In certain embodiments, tocilizumab is administered intravenously at a monthly equivalent dose of about 350 mg. In certain embodiments, tocilizumab is administered intravenously at a monthly equivalent dose of about 500 mg.

In various embodiments, tocilizumab is administered subcutaneously at a monthly equivalent dose of 50 - 1000 mg, such 50 - 80 mg, 50- 160 mg, 50 - 240 mg, 50 - 400 mg, 50 - 560 mg, 50 - 800 mg, 80 - 160 mg, 80 - 240 mg, 80 - 400 mg, 80 - 560 mg, 80 - 800 mg, 80 - 1000 mg, 160 - 240 mg, 160 - 400 mg, 160 - 560, 160 - 800 mg, 160 - 1000 mg, 240 - 400 mg, 240 - 560 mg, 240 - 800 mg, 240 - 1000 mg, 400 - 560 mg, 400 - 800 mg, 400 - 1000 mg, 560 - 800 mg, 560 - 1000 mg, or 800 - 100 mg. In certain preferred embodiments, tocilizumab is administered subcutaneously at a monthly equivalent dose of 80 - 800 mg. In various embodiments, tocilizumab is administered subcutaneously at a monthly equivalent dose of about 50 mg, 80 mg, 160 mg, 240 mg, 400 mg, 560 mg, 800 mg, or 1000 mg. In certain embodiments, tocilizumab is administered subcutaneously at a monthly equivalent dose of about 50 mg. In certain embodiments, tocilizumab is administered subcutaneously at a monthly equivalent dose of about 80 mg. In certain embodiments, tocilizumab is administered subcutaneously at a monthly equivalent dose of about 160 mg. In certain embodiments, tocilizumab is administered subcutaneously at a monthly equivalent dose of about 240 mg. In certain embodiments, tocilizumab is administered subcutaneously at a monthly equivalent dose of about 400 mg. In certain embodiments, tocilizumab is administered subcutaneously at a monthly equivalent dose of about 560 mg. In certain embodiments, tocilizumab is administered subcutaneously at a monthly equivalent dose of about 800 mg. In certain embodiments, tocilizumab is administered subcutaneously at a monthly equivalent dose of about 1000 mg.

In some embodiments, the IL-6 antagonist is sarilumab. In various embodiments, sarilumab is administered intravenously at a monthly equivalent dose of 10 - 150 mg, such as 10 - 12 mg, 10 - 24 mg, 10 - 48 mg, 10 - 60 mg, 10 - 72 mg, 10 - 120 mg, 12 - 24 mg, 12 - 48 mg, 12 - 60 mg, 12 - 72 mg, 12 - 120 mg, 12 - 150 mg, 24 - 48 mg, 24 - 60 mg, 24 - 72 mg, 24 - 120 mg, 24 - 150 mg, 48 - 60 mg, 48 - 72 mg, 48 - 120 mg, 48- 150 mg, 60 - 72 mg, 60 - 120 mg, 60 - 150 mg, 72 - 120 mg, 72 - 150 mg, or 120 - 150 mg. In certain preferred embodiments, sarilumab is administered intravenously at a monthly equivalent dose of 12 - 120 mg. In various embodiments, sarilumab is administered intravenously at a monthly equivalent dose of 10 mg, 12 mg, 24 mg, 48 mg, 60 mg, 72 mg, 120 mg, or 150 mg. In certain embodiments, sarilumab is administered intravenously at a monthly equivalent dose of 10 mg. In certain embodiments, sarilumab is administered intravenously at a monthly equivalent dose of 12 mg. In certain embodiments, sarilumab is administered intravenously at a monthly equivalent dose of 24 mg. In certain embodiments, sarilumab is administered intravenously at a monthly equivalent dose of 48 mg. In certain embodiments, sarilumab is administered intravenously at a monthly equivalent dose of 60 mg. In certain embodiments, sarilumab is administered intravenously at a monthly equivalent dose of 72 mg. In certain embodiments, sarilumab is administered intravenously at a monthly equivalent dose of 120 mg. In certain embodiments, sarilumab is administered intravenously at a monthly equivalent dose of 150 mg.

In various embodiments, sarilumab is administered subcutaneously at a monthly equivalent dose of 10 - 200 mg, such as 10 - 20 mg, 10 - 40 mg, 10 - 60 mg, 10 - 80 mg, 10 - 100 mg, 10 - 120 mg, 20 - 40 mg, 20 - 60 mg, 20 - 80 mg, 20 - 100 mg, 20 - 120 mg, 20 - 200 mg, 40 - 60 mg, 40 - 80 mg, 40 - 100 mg, 40 - 120 mg, 40 - 200 mg, 60 - 80 mg, 60 - 100 mg, 60 - 120 mg, mg, 60 - 200 mg, 80- 100 mg, 80 - 120 mg, 80 - 200 mg, 100 - 120 mg, 100 - 200 mg, or 120 - 200 mg. In certain preferred embodiments, sarilumab is administered subcutaneously at a monthly equivalent dose of 20 - 200 mg. In various embodiments, sarilumab is administered subcutaneously at a monthly equivalent dose of about 10 mg, 20 mg, 30 mg, 40 mg, 50 mg, 60 mg, 80 mg, 100 mg, 120 mg, 150 mg, or 200 mg. In certain embodiments, sarilumab is administered subcutaneously at a monthly equivalent dose of about 20 mg. In certain embodiments, sarilumab is administered subcutaneously at a monthly equivalent dose of about 40 mg. In certain embodiments, sarilumab is administered subcutaneously at a monthly equivalent dose of about 80 mg. In certain embodiments, sarilumab is administered subcutaneously at a monthly equivalent dose of about 100 mg. In certain embodiments, sarilumab is administered subcutaneously at a monthly equivalent dose of about 120 mg. In certain embodiments, sarilumab is administered subcutaneously at a monthly equivalent dose of about 200 mg.

In some embodiments, the IL-6 antagonist is vobarilizumab. In various embodiments, vobarilizumab is administered intravenously at a monthly equivalent dose of 2 - 150 mg, such as 2 - 4 mg, 2 - 6 mg, 2-30 mg, 2 - 60 mg, 2 - 84 mg, 2 - 120 mg, 4 - 6 mg, 4 - 30 mg, 4 - 60 mg, 4 - 84 mg, 4 - 120 mg, 4 - 150 mg, 6 - 30 mg, 6 - 60 mg, 6 - 84 mg, 6 - 120 mg, 6 - 150 mg, 30 - 60 mg, 30 - 84 mg, 30 - 120 mg, 30 - 150 mg, 60 - 84 mg, 60 - 120 mg, 60 - 150 mg, 84 - 120 mg, 84 - 150 mg, or 120 - 150 mg. In certain preferred embodiments, vobarilizumab is administered intravenously at a monthly equivalent dose of 4 - 120 mg. In various embodiments, vobarilizumab is administered intravenously at a monthly equivalent dose of about 2 mg, 4 mg, 6 mg, 30 mg, 60 mg, 84 mg, 120 mg, or 150 mg. In certain embodiments, vobarilizumab is administered intravenously at a monthly equivalent dose of about 2 mg. In certain embodiments, vobarilizumab is administered intravenously at a monthly equivalent dose of about 4 mg. In certain embodiments, vobarilizumab is administered intravenously at a monthly equivalent dose of about 6 mg. In certain embodiments, vobarilizumab is administered intravenously at a monthly equivalent dose of about 30 mg. In certain embodiments, vobarilizumab is administered intravenously at a monthly equivalent dose of about 60 mg. In certain embodiments, vobarilizumab is administered intravenously at a monthly equivalent dose of about 84 mg. In certain embodiments, vobarilizumab is administered intravenously at a monthly equivalent dose of about 120 mg. In certain embodiments, vobarilizumab is administered intravenously at a monthly equivalent dose of about 150 mg.

In various embodiments, vobarilizumab is administered subcutaneously at a monthly equivalent dose of 5 - 200 mg, such as 5 - 7 mg, 5 - 10 mg, 5-20 mg, 5 - 50 mg, 5 - 70 mg, 5 - 100 mg, 5 - 140 mg, 7 - 10 mg, 7-20 mg, 7 - 50 mg, 7-70 mg, 7 - 100 mg, 7 - 140 mg, 7 - 200 mg, 10 - 20 mg, 10 - 50 mg, 10 - 70 mg, 10 - 100 mg, 10 - 140 mg, 10 - 200 mg, 20 - 50 mg, 20 - 70 mg, 20 - 100 mg, 20 - 140 mg, 20 - 200 mg, 50 - 70 mg, 50 - 100 mg, 50 - 140 mg, 50 - 200 mg, 70 - 100 mg, 70 - 140 mg, 70 - 200 mg, 100 - 140 mg, 100 - 200 mg, or 140 - 200 mg. In certain preferred embodiments, vobarilizumab is administered subcutaneously at a monthly equivalent dose of 7 - 200 mg. In various embodiments, vobarilizumab is administered subcutaneously at a monthly equivalent dose of about 5 mg, 7 mg, 10 mg, 20 mg, 40 mg, 50 mg, 70 mg, 100 mg, 140 mg, or 200 mg. In certain embodiments, vobarilizumab is administered subcutaneously at a monthly equivalent dose of about 7 mg. In certain embodiments, vobarilizumab is administered subcutaneously at a monthly equivalent dose of about 10 mg. In certain embodiments, vobarilizumab is administered subcutaneously at a monthly equivalent dose of about 50 mg. In certain embodiments, vobarilizumab is administered subcutaneously at a monthly equivalent dose of about 100 mg. In certain embodiments, vobarilizumab is administered subcutaneously at a monthly equivalent dose of about 140 mg. In certain embodiments, vobarilizumab is administered subcutaneously at a monthly equivalent dose of about 200 mg.

### 3. Additional Embodiments

**1.** An embodiment provided by the present disclosure is a method for treating IL-6-mediated inflammation in a patient, comprising:
   administering an IL-6 antagonist to a patient with IL-6-mediated inflammation at a dose that is sufficient to reduce inflammation without causing immune suppression.
**2.** The method of embodiment 1, wherein the patient has an elevated pre-treatment C-reactive protein (CRP) level.
**3.** The method of embodiment 2, wherein the pre-treatment CRP level of the patient is at least 2 mg/L.
**4.** The method of embodiment 3, wherein the pre-treatment CRP level of the patient is at least 4 mg/L.
**5.** The method of embodiment 4, wherein the pre-treatment CRP level of the patient is at least 6 mg/L.
**6.** The method of embodiment 5, wherein the pre-treatment CRP level of the patient is at least 10 mg/L.
**7.** The method of any of the above embodiments, wherein the patient has an elevated pre-treatment serum IL-6 level.
**8.** The method of embodiment 7, wherein the pre-treatment serum IL-6 level of the patient is at least 2 pg/mL.
**9.** The method of embodiment 8, wherein the pre-treatment serum IL-6 level of the patient is at least 4 pg/mL.
**10.** The method of embodiment 9, wherein the pre-treatment serum IL-6 level of the patient is at least 5 pg/mL.
**11.** The method of embodiment 10, wherein the pre-treatment serum IL-6 level of the patient is at least 10 pg/mL.
**12.** The method of any of the above embodiments, wherein the inflammation is measured by the level of C-reactive protein (CRP).
**13.** The method of embodiment 12, wherein the post-treatment CRP level is no more than 2 mg/L.
**14.** The method of embodiment 13, wherein the post-treatment CRP level is no more than 1 mg/L.
**15.** The method of embodiment 12, wherein the CRP level is decreased by at least 50% as compared to pre-treatment levels.
**16.** The method of embodiment 15, wherein the CRP level is decreased by at least 70% as compared to pre-treatment levels.
**17.** The method of embodiment 16, wherein the CRP level is decreased by at least 80% as compared to pre-treatment levels.
**18.** The method of embodiment 17, wherein the CRP level is decreased by at least 90% as compared to pre-treatment levels.
**19.** The method of any of the above embodiments, wherein the immune suppression is measured by absolute neutrophil count (ANC).
**20.** The method of embodiment 19, wherein the post-treatment ANC is at least 500 cells/µL.
**21.** The method of embodiment 20, wherein the post-treatment ANC is at least 1000 cells/µL.
**22.** The method of embodiment 21, wherein the post-treatment ANC is at least 1500 cells/µL.
**23.** The method of embodiment 21, wherein the post-treatment ANC is at least 2000 cells/µL.
**24.** The method of embodiment 19, wherein the ANC is decreased by no more than 2000 cells/µL as compared to pre-treatment levels.
**25.** The method of embodiment 24, wherein the ANC is decreased by no more than 1500 cells/µL as compared to pre-treatment levels.
**26.** The method of embodiment 25, wherein the ANC is decreased by no more than 1000 cells/µL as compared to pre-treatment levels.
**27.** The method of embodiment 26, wherein the ANC is decreased by no more than 500 cells/µL as compared to pre-treatment levels.
**28.** The method of embodiment 19, wherein the ANC is decreased by no more than 50% as compared to pre-treatment levels.
**29.** The method of embodiment 28, wherein the ANC is decreased by no more than 40% as compared to pre-treatment levels.
**30.** The method of embodiment 29, wherein the ANC is decreased by no more than 30% as compared to pre-treatment levels.
**31.** The method of embodiment 30, wherein the ANC is decreased by no more than 20% as compared to pre-treatment levels.
**32.** The method of embodiment 31, wherein the ANC is decreased by no more than 10% as compared to pre-treatment levels.
**33.** The method of embodiment 19, wherein the ANC is not decreased as compared to pre-treatment levels.
**34.** The method of any of the above embodiments, wherein the IL-6 antagonist is administered at a monthly equivalent dose that is no more than 30% of the monthly equivalent dose for treating rheumatoid arthritis with the same IL-6 antagonist.
**35.** The method of embodiment 34, wherein the IL-6 antagonist is administered at a monthly equivalent dose that is no more than 20% of the monthly equivalent dose for treating rheumatoid arthritis with the same IL-6 antagonist.
**36.** The method of embodiment 35, wherein the IL-6 antagonist is administered at a monthly equivalent dose that is no more than 10% of the monthly equivalent dose for treating rheumatoid arthritis with the same IL-6 antagonist.
**37.** The method of any one of embodiments 1 to 33, wherein the IL-6 antagonist is administered at a monthly equivalent dose that is about 25% of a monthly equivalent dose for treating rheumatoid arthritis with the same IL-6 antagonist.
**38.** The method of any one of embodiments 1 to 33, wherein the IL-6 antagonist is administered at a monthly equivalent dose that is about 20% of a monthly equivalent dose for treating rheumatoid arthritis with the same IL-6 antagonist.
**39.** The method of any one of embodiments 1 to 33, wherein the IL-6 antagonist is administered at a monthly equivalent dose that is about 15% of a monthly equivalent dose for treating rheumatoid arthritis with the same IL-6 antagonist.
**40.** The method of any one of embodiments 1 to 33, wherein the IL-6 antagonist is administered at a monthly equivalent dose that is about 10% of a monthly equivalent dose for treating rheumatoid arthritis with the same IL-6 antagonist.
**41.** The method of any one of embodiments 1 to 33, wherein the IL-6 antagonist is administered at a monthly equivalent dose that is about 5% of a monthly equivalent dose for treating rheumatoid arthritis with the same IL-6 antagonist.
**42.** The method of any of the above embodiments, wherein the IL-6 antagonist is an anti-IL-6 antibody.
**43.** The method of embodiment 42, wherein the anti-IL-6 antibody is ziltivekimab.
**44.** The method of embodiment 43, wherein ziltivekimab is administered intravenously at a monthly equivalent dose of 2 - 40 mg.
**45.** The method of embodiment 44, wherein ziltivekimab is administered intravenously at a monthly equivalent dose of 7.5 - 30 mg.
**46.** The method of embodiment 45, wherein ziltivekimab is administered intravenously at a monthly equivalent dose of 7.5 - 15 mg.
**47.** The method of embodiment 45, wherein ziltivekimab is administered intravenously at a monthly equivalent dose of 15 - 30 mg.
**48.** The method of embodiment 43, wherein ziltivekimab is administered intravenously at a monthly equivalent dose of about 2 mg.
**49.** The method of embodiment 43, wherein ziltivekimab is administered intravenously at a monthly equivalent dose of about 4 mg.
**50.** The method of embodiment 43, wherein ziltivekimab is administered intravenously at a monthly equivalent dose of about 6 mg.
**51.** The method of embodiment 43, wherein ziltivekimab is administered intravenously at a monthly equivalent dose of about 7.5 mg.
**52.** The method of embodiment 43, wherein ziltivekimab is administered intravenously at a monthly equivalent dose of about 10 mg.
**53.** The method of embodiment 43, wherein ziltivekimab is administered intravenously at a monthly equivalent dose of about 15 mg.
**54.** The method of embodiment 43, wherein ziltivekimab is administered intravenously at a monthly equivalent dose of about 20 mg.
**55.** The method of embodiment 43, wherein ziltivekimab is administered intravenously at a monthly equivalent dose of about 30 mg.
**56.** The method of embodiment 43, wherein ziltivekimab is administered intravenously at a monthly equivalent dose of about 40 mg.
**57.** The method of embodiment 43, wherein ziltivekimab is administered subcutaneously at a monthly equivalent dose of 3-70 mg.
**58.** The method of embodiment 57, wherein ziltivekimab is administered subcutaneously at a monthly equivalent dose of 7.5-30 mg.
**59.** The method of embodiment 58, wherein ziltivekimab is administered subcutaneously at a monthly equivalent dose of 7.5-15 mg.
**60.** The method of embodiment 58, wherein ziltivekimab is administered subcutaneously at a monthly equivalent dose of 15-30 mg.
**61.** The method of embodiment 43, wherein ziltivekimab is administered subcutaneously at a monthly equivalent dose of about 3 mg.
**62.** The method of embodiment 43, wherein ziltivekimab is administered subcutaneously at a monthly equivalent dose of about 7 mg.
**63.** The method of embodiment 43, wherein ziltivekimab is administered subcutaneously at a monthly equivalent dose of about 7.5 mg.
**64.** The method of embodiment 43, wherein ziltivekimab is administered subcutaneously at a monthly equivalent dose of about 10 mg.
**65.** The method of embodiment 43, wherein ziltivekimab is administered subcutaneously at a monthly equivalent dose of about 15 mg.
**66.** The method of embodiment 43, wherein ziltivekimab is administered subcutaneously at a monthly equivalent dose of about 17 mg.
**67.** The method of embodiment 43, wherein ziltivekimab is administered subcutaneously at a monthly equivalent dose of about 30 mg.
**68.** The method of embodiment 43, wherein ziltivekimab is administered subcutaneously at a monthly equivalent dose of about 35 mg.
**69.** The method of embodiment 43, wherein ziltivekimab is administered subcutaneously at a monthly equivalent dose of about 70 mg.
**70.** The method of embodiment 42, wherein the anti-IL-6 antibody is siltuximab.
**71.** The method of embodiment 70, wherein siltuximab is administered intravenously at a monthly equivalent dose of 50-500 mg.
**72.** The method of embodiment 70, wherein siltuximab is administered intravenously at a monthly equivalent dose of about 50 mg.
**73.** The method of embodiment 70, wherein siltuximab is administered intravenously at a monthly equivalent dose of about 100 mg.
**74.** The method of embodiment 70, wherein siltuximab is administered intravenously at a monthly equivalent dose of about 150 mg.
**75.** The method of embodiment 70, wherein siltuximab is administered intravenously at a monthly equivalent dose of about 200 mg.
**76.** The method of embodiment 70, wherein siltuximab is administered intravenously at a monthly equivalent dose of about 300 mg.
**77.** The method of embodiment 70, wherein siltuximab is administered intravenously at a monthly equivalent dose of about 500 mg.
**78.** The method of embodiment 70, wherein siltuximab is administered subcutaneously at a monthly equivalent dose of 80-800 mg.
**79.** The method of embodiment 70, wherein siltuximab is administered subcutaneously at a monthly equivalent dose of about 80 mg.
**80.** The method of embodiment 70, wherein siltuximab is administered subcutaneously at a monthly equivalent dose of about 160 mg.
**81.** The method of embodiment 70, wherein siltuximab is administered subcutaneously at a monthly equivalent dose of about 240 mg.
**82.** The method of embodiment 70, wherein siltuximab is administered subcutaneously at a monthly equivalent dose of about 320 mg.
**83.** The method of embodiment 70, wherein siltuximab is administered subcutaneously at a monthly equivalent dose of about 480 mg.
**84.** The method of embodiment 70, wherein siltuximab is administered subcutaneously at a monthly equivalent dose of about 800 mg.
**85.** The method of embodiment 42, wherein the anti-IL-6 antibody is gerilizumab.
**86.** The method of embodiment 85, wherein gerilizumab is administered intravenously at a monthly equivalent dose of 0.075-1.8 mg.
**87.** The method of embodiment 85, wherein gerilizumab is administered intravenously at a monthly equivalent dose of about 0.075 mg.
**88.** The method of embodiment 85, wherein gerilizumab is administered intravenously at a monthly equivalent dose of about 0.12 mg.
**89.** The method of embodiment 85, wherein gerilizumab is administered intravenously at a monthly equivalent dose of about 0.3 mg.
**90.** The method of embodiment 85, wherein gerilizumab is administered intravenously at a monthly equivalent dose of about 0.6 mg.
**91.** The method of embodiment 85, wherein gerilizumab is administered intravenously at a monthly equivalent dose of about 0.9 mg.
**92.** The method of embodiment 85, wherein gerilizumab is administered intravenously at a monthly equivalent dose of about 1.8 mg.
**93.** The method of embodiment 85, wherein gerilizumab is administered subcutaneously at a monthly equivalent dose of 0.125-3 mg.
**94.** The method of embodiment 85, wherein gerilizumab is administered subcutaneously at a monthly equivalent dose of about 0.125 mg.
**95.** The method of embodiment 85, wherein gerilizumab is administered subcutaneously at a monthly equivalent dose of about 0.2 mg.
**96.** The method of embodiment 85, wherein gerilizumab is administered subcutaneously at a monthly equivalent dose of about 0.5 mg.
**97.** The method of embodiment 85, wherein gerilizumab is administered subcutaneously at a monthly equivalent dose of about 1 mg.
**98.** The method of embodiment 85, wherein gerilizumab is administered subcutaneously at a monthly equivalent dose of about 1.5 mg.
**99.** The method of embodiment 85, wherein gerilizumab is administered subcutaneously at a monthly equivalent dose of about 3 mg.
**100.** The method of embodiment 42, wherein the anti-IL-6 antibody is sirukumab.
**101.** The method of embodiment 100, wherein sirukumab is administered intravenously at a monthly equivalent dose of 1.5-60 mg.
**102.** The method of embodiment 100, wherein sirukumab is administered intravenously at a monthly equivalent dose of about 1.5 mg.
**103.** The method of embodiment 100, wherein sirukumab is administered intravenously at a monthly equivalent dose of about 3 mg.
**104.** The method of embodiment 100, wherein sirukumab is administered intravenously at a monthly equivalent dose of about 6 mg.
**105.** The method of embodiment 100, wherein sirukumab is administered intravenously at a monthly equivalent dose of about 12 mg.
**106.** The method of embodiment 100, wherein sirukumab is administered intravenously at a monthly equivalent dose of about 36 mg.
**107.** The method of embodiment 100, wherein sirukumab is administered intravenously at a monthly equivalent dose of about 60 mg.
**108.** The method of embodiment 100, wherein sirukumab is administered subcutaneously at a monthly equivalent dose of 2.5-100 mg.
**109.** The method of embodiment 100, wherein sirukumab is administered subcutaneously at a monthly equivalent dose of about 2.5 mg.
**110.** The method of embodiment 100, wherein sirukumab is administered subcutaneously at a monthly equivalent dose of about 5 mg.
**111.** The method of embodiment 100, wherein sirukumab is administered subcutaneously at a monthly equivalent dose of about 10 mg.
**112.** The method of embodiment 100, wherein sirukumab is administered subcutaneously at a monthly equivalent dose of about 20 mg.
**113.** The method of embodiment 100, wherein sirukumab is administered subcutaneously at a monthly equivalent dose of about 60 mg.
**114.** The method of embodiment 100, wherein sirukumab is administered subcutaneously at a monthly equivalent dose of about 100 mg.
**115.** The method of embodiment 42, wherein the anti-IL-6 antibody is clazakizumab.
**116.** The method of embodiment 115, wherein clazakizumab is administered intravenously at a monthly equivalent dose of 3-60 mg.
**117.** The method of embodiment 115, wherein clazakizumab is administered intravenously at a monthly equivalent dose of about 3 mg.
**118.** The method of embodiment 115, wherein clazakizumab is administered intravenously at a monthly equivalent dose of about 6 mg.
**119.** The method of embodiment 115, wherein clazakizumab is administered intravenously at a monthly equivalent dose of about 12 mg.
**120.** The method of embodiment 115, wherein clazakizumab is administered intravenously at a monthly equivalent dose of about 24 mg.
**121.** The method of embodiment 115, wherein clazakizumab is administered intravenously at a monthly equivalent dose of about 36 mg.
**122.** The method of embodiment 115, wherein clazakizumab is administered intravenously at a monthly equivalent dose of about 60 mg.
**123.** The method of embodiment 115, wherein clazakizumab is administered subcutaneously at a monthly equivalent dose of 5-100 mg.
**124.** The method of embodiment 115, wherein clazakizumab is administered subcutaneously at a monthly equivalent dose of about 5 mg.
**125.** The method of embodiment 115, wherein clazakizumab is administered subcutaneously at a monthly equivalent dose of about 10 mg.
**126.** The method of embodiment 115, wherein clazakizumab is administered subcutaneously at a monthly equivalent dose of about 20 mg.
**127.** The method of embodiment 115, wherein clazakizumab is administered subcutaneously at a monthly equivalent dose of about 40 mg.
**128.** The method of embodiment 115, wherein clazakizumab is administered subcutaneously at a monthly equivalent dose of about 60 mg.
**129.** The method of embodiment 115, wherein clazakizumab is administered subcutaneously at a monthly equivalent dose of about 100 mg.
**130.** The method of embodiment 42, wherein the anti-IL-6 antibody is olokizumab.
**131.** The method of embodiment 130, wherein olokizumab is administered intravenously at a monthly equivalent dose of 1.8-60 mg.
**132.** The method of embodiment 130, wherein olokizumab is administered intravenously at a monthly equivalent dose of about 1.8 mg.
**133.** The method of embodiment 130, wherein olokizumab is administered intravenously at a monthly equivalent dose of about 3.6 mg.
**134.** The method of embodiment 130, wherein olokizumab is administered intravenously at a monthly equivalent dose of about 9 mg.
**135.** The method of embodiment 130, wherein olokizumab is administered intravenously at a monthly equivalent dose of about 18 mg.
**136.** The method of embodiment 130, wherein olokizumab is administered intravenously at a monthly equivalent dose of about 45 mg.
**137.** The method of embodiment 130, wherein olokizumab is administered intravenously at a monthly equivalent dose of about 60 mg.
**138.** The method of embodiment 130, wherein olokizumab is administered subcutaneously at a monthly equivalent dose of 3-100 mg.
**139.** The method of embodiment 130, wherein olokizumab is administered subcutaneously at a monthly equivalent dose of about 3 mg.
**140.** The method of embodiment 130, wherein olokizumab is administered subcutaneously at a monthly equivalent dose of about 6 mg.
**141.** The method of embodiment 130, wherein olokizumab is administered subcutaneously at a monthly equivalent dose of about 15 mg.
**142.** The method of embodiment 130, wherein olokizumab is administered subcutaneously at a monthly equivalent dose of about 30 mg.
**143.** The method of embodiment 130, wherein olokizumab is administered subcutaneously at a monthly equivalent dose of about 72 mg.
**144.** The method of embodiment 130, wherein olokizumab is administered subcutaneously at a monthly equivalent dose of about 100 mg.
**145.** The method of embodiment 42, wherein the anti-IL-6 antibody is VX30 (VOP-R003; Vaccinex).
**146.** The method of embodiment 145, wherein VX30 (VOP-R003; Vaccinex) is administered intravenously.
**147.** The method of embodiment 145, wherein VX30 (VOP-R003; Vaccinex) is administered subcutaneously.
**148.** The method of embodiment 42, wherein the anti-IL-6 antibody is EB-007 (EBI-029; Eleven Bio).
**149.** The method of embodiment 148, wherein EB-007 (EBI-029; Eleven Bio) is administered intravenously.
**150.** The method of embodiment 148, wherein EB-007 (EBI-029; Eleven Bio) is administered subcutaneously.
**151.** The method of embodiment 42, wherein the anti-IL-6 antibody is FM101 (Femta Pharmaceuticals, Lonza).
**152.** The method of embodiment 151, wherein FM101 (Femta Pharmaceuticals, Lonza) is administered intravenously.
**153.** The method of embodiment 151, wherein FM101 (Femta Pharmaceuticals, Lonza) is administered subcutaneously.
**154.** The method of any one of embodiments 1 to 41, wherein the IL-6 antagonist is an anti-IL-6R antibody.
**155.** The method of embodiment 154, wherein the anti-IL-6R antibody is tocilizumab.
**156.** The method of embodiment 155, wherein tocilizumab is administered intravenously at a monthly equivalent dose of 50-500 mg.
**157.** The method of embodiment 155, wherein tocilizumab is administered intravenously at a monthly equivalent dose of about 50 mg.
**158.** The method of embodiment 155, wherein tocilizumab is administered intravenously at a monthly equivalent dose of about 100 mg.
**159.** The method of embodiment 155, wherein tocilizumab is administered intravenously at a monthly equivalent dose of about 150 mg.
**160.** The method of embodiment 155, wherein tocilizumab is administered intravenously at a monthly equivalent dose of about 250 mg.
**161.** The method of embodiment 155, wherein tocilizumab is administered intravenously at a monthly equivalent dose of about 350 mg.
**162.** The method of embodiment 155, wherein tocilizumab is administered intravenously at a monthly equivalent dose of about 500 mg.
**163.** The method of embodiment 155, wherein tocilizumab is administered subcutaneously at a monthly equivalent dose of 80-800 mg.
**164.** The method of embodiment 155, wherein tocilizumab is administered subcutaneously at a monthly equivalent dose of about 80 mg.
**165.** The method of embodiment 155, wherein tocilizumab is administered subcutaneously at a monthly equivalent dose of about 160 mg.
**166.** The method of embodiment 155, wherein tocilizumab is administered subcutaneously at a monthly equivalent dose of about 240 mg.
**167.** The method of embodiment 155, wherein tocilizumab is administered subcutaneously at a monthly equivalent dose of about 400 mg.
**168.** The method of embodiment 155, wherein tocilizumab is administered subcutaneously at a monthly equivalent dose of about 560 mg.
**169.** The method of embodiment 155, wherein tocilizumab is administered subcutaneously at a monthly equivalent dose of about 800 mg.
**170.** The method of embodiment 154, wherein the anti-IL-6R antibody is sarilumab.
**171.** The method of embodiment 170, wherein sarilumab is administered intravenously at a monthly equivalent dose of 12-120 mg.
**172.** The method of embodiment 170, wherein sarilumab is administered intravenously at a monthly equivalent dose of about 12 mg.
**173.** The method of embodiment 170, wherein sarilumab is administered intravenously at a monthly equivalent dose of about 24 mg.
**174.** The method of embodiment 170, wherein sarilumab is administered intravenously at a monthly equivalent dose of about 48 mg.
**175.** The method of embodiment 170, wherein sarilumab is administered intravenously at a monthly equivalent dose of about 60 mg.
**176.** The method of embodiment 170, wherein sarilumab is administered intravenously at a monthly equivalent dose of about 72 mg.
**177.** The method of embodiment 170, wherein sarilumab is administered intravenously at a monthly equivalent dose of about 120 mg.
**178.** The method of embodiment 170, wherein sarilumab is administered subcutaneously at a monthly equivalent dose of 20-200 mg.
**179.** The method of embodiment 170, wherein sarilumab is administered subcutaneously at a monthly equivalent dose of about 20 mg.
**180.** The method of embodiment 170, wherein sarilumab is administered subcutaneously at a monthly equivalent dose of about 40 mg.
**181.** The method of embodiment 170, wherein sarilumab is administered subcutaneously at a monthly equivalent dose of about 80 mg.
**182.** The method of embodiment 170, wherein sarilumab is administered subcutaneously at a monthly equivalent dose of about 100 mg.
**183.** The method of embodiment 170, wherein sarilumab is administered subcutaneously at a monthly equivalent dose of about 120 mg.
**184.** The method of embodiment 170, wherein sarilumab is administered subcutaneously at a monthly equivalent dose of about 200 mg.
**185.** The method of embodiment 154, wherein the anti-IL-6R antibody is vobarilizumab.
**186.** The method of embodiment 185, wherein vobarilizumab is administered intravenously at a monthly equivalent dose of 4-120 mg.
**187.** The method of embodiment 185, wherein vobarilizumab is administered intravenously at a monthly equivalent dose of about 4 mg.
**188.** The method of embodiment 185, wherein vobarilizumab is administered intravenously at a monthly equivalent dose of about 6 mg.
**189.** The method of embodiment 185, wherein vobarilizumab is administered intravenously at a monthly equivalent dose of about 30 mg.
**190.** The method of embodiment 185, wherein vobarilizumab is administered intravenously at a monthly equivalent dose of about 60 mg.
**191.** The method of embodiment 185, wherein vobarilizumab is administered intravenously at a monthly equivalent dose of about 84 mg.
**192.** The method of embodiment 185, wherein vobarilizumab is administered intravenously at a monthly equivalent dose of about 120 mg.
**193.** The method of embodiment 185, wherein vobarilizumab is administered subcutaneously at a monthly equivalent dose of 7-200 mg.
**194.** The method of embodiment 185, wherein vobarilizumab is administered subcutaneously at a monthly equivalent dose of about 7 mg.
**195.** The method of embodiment 185, wherein vobarilizumab is administered subcutaneously at a monthly equivalent dose of about 10 mg.
**196.** The method of embodiment 185, wherein vobarilizumab is administered subcutaneously at a monthly equivalent dose of about 50 mg.
**197.** The method of embodiment 185, wherein vobarilizumab is administered subcutaneously at a monthly equivalent dose of about 100 mg.
**198.** The method of embodiment 185, wherein vobarilizumab is administered subcutaneously at a monthly equivalent dose of about 140 mg.
**199.** The method of embodiment 185, wherein vobarilizumab is administered subcutaneously at a monthly equivalent dose of about 200 mg.
**200.** The method of any one of embodiments 1 to 41, wherein the IL-6 antagonist is a JAK inhibitor.
**201.** The method of any one of embodiments 1 to 41, wherein the IL-6 antagonist is a STAT3 inhibitor.
**202.** The method of any of the above embodiments, wherein the patient has a hepcidin-mediated disorder.
**203.** The method of any one of embodiments 1 to 201, wherein the patient has kidney disease.
**204.** The method of embodiment 203, wherein the patient has chronic kidney disease.
**205.** The method of embodiment 204, wherein the patient has KDOQI stage 1-5 chronic kidney disease.
**206.** The method of embodiment 205, wherein the patient has KDOQI stage 3-5 chronic kidney disease.
**207.** The method of embodiment 206, wherein the patient is not on dialysis.
**208.** The method of embodiment 205, wherein the patient has KDOQI stage 5 chronic kidney disease.
**209.** The method of embodiment 208, wherein the patient is on dialysis.
**210.** The method of embodiment 204, wherein the patient has cardiorenal syndrome (CRS).
**211.** The method of embodiment 210, wherein the patient has CRS Type 4.
**212.** The method of embodiment 204, wherein the patient has atherosclerotic cardiovascular disease (ASCVD).
**213.** The method of any one of embodiments 1 to 201, wherein the patient has cardiovascular disease.
**214.** The method of embodiment 213, wherein the patient has diuretic resistant heart failure.
**215.** The method of embodiment 213, wherein the patient has congestive heart failure (CHF).
**216.** The method of embodiment 215, wherein the patient has congestive heart failure (CHF) with reduced ejection fraction.
**217.** The method of embodiment 215, wherein the patient has congestive heart failure (CHF) with mid-range ejection fraction.
**218.** The method of embodiment 215, wherein the patient has congestive heart failure (CHF) with preserved ejection fraction.
**219.** The method of embodiment 213, wherein the patient has acute coronary syndrome.
**220.** The method of embodiment 213, wherein the patient has atherosclerosis.
**221.** The method of embodiment 213, wherein the patient has atherosclerotic cardiovascular disease (ASCVD).
**222.** The method of any one of embodiments 1 to 201, wherein the patient has anemia.
**223.** The method of embodiment 222, wherein the patient has anemia of chronic disease.
**224.** The method of embodiment 222, wherein the patient has iron-refractory iron-deficiency anemia (IRIDA).
**225.** The method of any one of embodiments 1 to 201, wherein the patient has diabetes.
**226.** The method of embodiment 225, wherein the patient has type II diabetes.
**227.** The method of embodiment 225, wherein the patient has insulin-resistant diabetes.
**228.** The method of any one of embodiments 1 to 201, wherein the patient has liver disease.
**229.** The method of embodiment 228, wherein the patient has non-alcoholic steatohepatitis (NASH).
**230.** The method of any one of embodiments 1 to 201, wherein the patient has osteoporosis.
**231.** The method of any one of embodiments 1 to 201, wherein the patient has depression.
**232.** The method of any one of embodiments 1 to 201, wherein the patient has asthma.
**233.** The method of any one of embodiments 1 to 201, wherein the patient has neuroinflammatory disorder.
**234.** The method of embodiment 233, wherein the patient has Alzheimer's disease.
**235.** The method of embodiment 233, wherein the patient has Parkinson's disease.
**236.** The method of embodiment 233, wherein the patient has multiple sclerosis.
**237.** The method of embodiment 233, wherein the patient has amyotrophic lateral sclerosis (ALS).
**238.** The method of any one of embodiments 1 to 201, wherein the patient has age-related macular degeneration (AMD).
**239.** The method of any one of embodiments 1 to 201, wherein the patient has cancer.
**240.** The method of embodiment 239, wherein the cancer is selected from the group consisting of: solid tumors, small cell lung cancer, non-small cell lung cancer, hematological cancer, multiple myeloma, leukemia, chronic lymphocytic leukemia (CLL), chronic myeloid leukemia (CML), lymphomas, Hodgkin's lymphoma and hepatic adenoma.
**241.** The method of any one of embodiments 1 to 201, wherein the patient has skin disease.
**242.** The method of any one of embodiments 1 to 201, wherein the method prevents aging in the patient.
**243.** An embodiment of the present disclosure is a method for treating inflammation in a patient with cardiovascular disease, comprising:
   administering an IL-6 antagonist to a patient with cardiovascular disease and CRP level greater than 2 mg/L at a dose that is sufficient to reduce CRP levels to 2 mg/L or less without causing neutropenia.
**244.** The method of embodiment 243, wherein the IL-6 antagonist is administered at a monthly equivalent dose that is no more than 30% of the monthly equivalent dose for treating rheumatoid arthritis with the same IL-6 antagonist.
**245.** The method of embodiment 244, wherein the IL-6 antagonist is administered at a monthly equivalent dose that is no more than 20% of the monthly equivalent dose for treating rheumatoid arthritis with the same IL-6 antagonist.
**246.** The method of embodiment 245, wherein the IL-6 antagonist is administered at a monthly equivalent dose that is no more than 10% of the monthly equivalent dose for treating rheumatoid arthritis with the same IL-6 antagonist.
**247.** An embodiment of the present disclosure is a method for treating inflammation in a patient with chronic kidney disease (CKD), comprising:
   administering an IL-6 antagonist to a patient with CKD and a CRP level greater than 2 mg/L at a dose that is sufficient to reduce CRP levels to 2 mg/L or less without causing neutropenia.
**248.** The method of claim 247, wherein the IL-6 antagonist is administered at a monthly equivalent dose that is no more than 30% of the monthly equivalent dose for treating rheumatoid arthritis with the same IL-6 antagonist.
**249.** The method of embodiment 248, wherein the IL-6 antagonist is administered at a monthly equivalent dose that is no more than 20% of the monthly equivalent dose for treating rheumatoid arthritis with the same IL-6 antagonist.
**250.** The method of embodiment 249, wherein the IL-6 antagonist is administered at a monthly equivalent dose that is no more than 10% of the monthly equivalent dose for treating rheumatoid arthritis with the same IL-6 antagonist.

In some embodiments, the anti-IL-6 antibody is siltuximab. In some embodiments, siltuximab is administered intravenously at a monthly equivalent dose of 50-500 mg. In some embodiments, siltuximab is administered intravenously at a monthly equivalent dose of about 50 mg. In some embodiments, siltuximab is administered intravenously at a monthly equivalent dose of about 100 mg. In some embodiments, siltuximab is administered intravenously at a monthly equivalent dose of about 150 mg. In some embodiments, siltuximab is administered intravenously at a monthly equivalent dose of about 200 mg. In some embodiments, siltuximab is administered intravenously at a monthly equivalent dose of about 300 mg. In some embodiments, siltuximab is administered intravenously at a monthly equivalent dose of about 500 mg. In some embodiments, siltuximab is administered subcutaneously at a monthly equivalent dose of 80-800 mg. In some embodiments, siltuximab is administered subcutaneously at a monthly equivalent dose of about 80 mg. In some embodiments, siltuximab is administered subcutaneously at a monthly equivalent dose of about 160 mg. In some embodiments, siltuximab is administered subcutaneously at a monthly equivalent dose of about 240 mg. In some embodiments, siltuximab is administered subcutaneously at a monthly equivalent dose of about 320 mg. In some embodiments, siltuximab is administered subcutaneously at a monthly equivalent dose of about 480 mg. In some embodiments, siltuximab is administered subcutaneously at a monthly equivalent dose of about 800 mg.

In some embodiments, the anti-IL-6 antibody is gerilizumab. In some embodiments, gerilizumab is administered intravenously at a monthly equivalent dose of 0.075-1.8 mg. In some embodiments, gerilizumab is administered intravenously at a monthly equivalent dose of about 0.075 mg. In some embodiments, gerilizumab is administered intravenously at a monthly equivalent dose of about 0.12 mg. In some embodiments, gerilizumab is administered intravenously at a monthly equivalent dose of about 0.3 mg. In some embodiments, gerilizumab is administered intravenously at a monthly equivalent dose of about 0.6 mg. In some embodiments, gerilizumab is administered intravenously at a monthly equivalent dose of about 0.9 mg. In some embodiments, gerilizumab is administered intravenously at a monthly equivalent dose of about 1.8 mg. In some embodiments, gerilizumab is administered subcutaneously at a monthly equivalent dose of 0.125-3 mg. In some embodiments, gerilizumab is administered subcutaneously at a monthly equivalent dose of about 0.125 mg. In some embodiments, gerilizumab is administered subcutaneously at a monthly equivalent dose of about 0.2 mg. In some embodiments, gerilizumab is administered subcutaneously at a monthly equivalent dose of about 0.5 mg. In some embodiments, gerilizumab is administered subcutaneously at a monthly equivalent dose of about 1 mg. In some embodiments, gerilizumab is administered subcutaneously at a monthly equivalent dose of about 1.5 mg. In some embodiments, gerilizumab is administered subcutaneously at a monthly equivalent dose of about 3 mg.

In some embodiments, the anti-IL-6 antibody is sirukumab. In some embodiments, sirukumab is administered intravenously at a monthly equivalent dose of 1.5-60 mg. In some embodiments, sirukumab is administered intravenously at a monthly equivalent dose of about 1.5 mg. In some embodiments, sirukumab is administered intravenously at a monthly equivalent dose of about 3 mg. In some embodiments, sirukumab is administered intravenously at a monthly equivalent dose of about 6 mg. In some embodiments, sirukumab is administered intravenously at a monthly equivalent dose of about 12 mg. In some embodiments, sirukumab is administered intravenously at a monthly equivalent dose of about 36 mg. In some embodiments, sirukumab is administered intravenously at a monthly equivalent dose of about 60 mg. In some embodiments, sirukumab is administered subcutaneously at a monthly equivalent dose of 2.5-100 mg. In some embodiments, sirukumab is administered subcutaneously at a monthly equivalent dose of about 2.5 mg. In some embodiments, sirukumab is administered subcutaneously at a monthly equivalent dose of about 5 mg. In some embodiments, sirukumab is administered subcutaneously at a monthly equivalent dose of about 10 mg. In some embodiments, sirukumab is administered subcutaneously at a monthly equivalent dose of about 20 mg. In some embodiments, sirukumab is administered subcutaneously at a monthly equivalent dose of about 60 mg. In some embodiments, sirukumab is administered subcutaneously at a monthly equivalent dose of about 100 mg.

In some embodiments, the anti-IL-6 antibody is clazakizumab. In some embodiments, clazakizumab is administered intravenously at a monthly equivalent dose of 3-60 mg. In some embodiments, clazakizumab is administered intravenously at a monthly equivalent dose of about 3 mg. In some embodiments, clazakizumab is administered intravenously at a monthly equivalent dose of about 6 mg. In some embodiments, clazakizumab is administered intravenously at a monthly equivalent dose of about 12 mg. In some embodiments, clazakizumab is administered intravenously at a monthly equivalent dose of about 24 mg. In some embodiments, clazakizumab is administered intravenously at a monthly equivalent dose of about 36 mg. In some embodiments, clazakizumab is administered intravenously at a monthly equivalent dose of about 60 mg. In some embodiments, clazakizumab is administered subcutaneously at a monthly equivalent dose of 5-100 mg. In some embodiments, clazakizumab is administered subcutaneously at a monthly equivalent dose of about 5 mg. In some embodiments, clazakizumab is administered subcutaneously at a monthly equivalent dose of about 10 mg. In some embodiments, clazakizumab is administered subcutaneously at a monthly equivalent dose of about 20 mg. In some embodiments, clazakizumab is administered subcutaneously at a monthly equivalent dose of about 40 mg. In some embodiments, clazakizumab is administered subcutaneously at a monthly equivalent dose of about 60 mg. In some embodiments, clazakizumab is administered subcutaneously at a monthly equivalent dose of about 100 mg.

In some embodiments, the anti-IL-6 antibody is olokizumab. In some embodiments, olokizumab is administered intravenously at a monthly equivalent dose of 1.8-60 mg. In some embodiments, olokizumab is administered intravenously at a monthly equivalent dose of about 1.8 mg. In some embodiments, olokizumab is administered intravenously at a monthly equivalent dose of about 3.6 mg. In some embodiments, olokizumab is administered intravenously at a monthly equivalent dose of about 9 mg. In some embodiments, olokizumab is administered intravenously at a monthly equivalent dose of about 18 mg. In some embodiments, olokizumab is administered intravenously at a monthly equivalent dose of about 45 mg. In some embodiments, olokizumab is administered intravenously at a monthly equivalent dose of about 60 mg. In some embodiments, olokizumab is administered subcutaneously at a monthly equivalent dose of 3-100 mg. In some embodiments, olokizumab is administered subcutaneously at a monthly equivalent dose of about 3 mg. In some embodiments, olokizumab is administered subcutaneously at a monthly equivalent dose of about 6 mg. In some embodiments, olokizumab is administered subcutaneously at a monthly equivalent dose of about 15 mg. In some embodiments, olokizumab is administered subcutaneously at a monthly equivalent dose of about 30 mg. In some embodiments, olokizumab is administered subcutaneously at a monthly equivalent dose of about 72 mg. In some embodiments, olokizumab is administered subcutaneously at a monthly equivalent dose of about 100 mg.

In some embodiments, the anti-IL-6 antibody is VX30 (VOP-R003; Vaccinex). In some embodiments, VX30 (VOP-R003; Vaccinex) is administered intravenously. In some embodiments, VX30 (VOP-R003; Vaccinex) is administered subcutaneously.

In some embodiments, the anti-IL-6 antibody is EB-007 (EBI-029; Eleven Bio). In some embodiments, EB-007 (EBI-029; Eleven Bio) is administered intravenously. In some embodiments, EB-007 (EBI-029; Eleven Bio) is administered subcutaneously.

In some embodiments, the anti-IL-6 antibody is FM101 (Femta Pharmaceuticals, Lonza). In some embodiments, FM101 (Femta Pharmaceuticals, Lonza) is administered intravenously. In some embodiments, FM101 (Femta Pharmaceuticals, Lonza) is administered subcutaneously.

In some embodiments, the IL-6 antagonist is an anti-IL-6R antibody.

In some embodiments, the anti-IL-6R antibody is tocilizumab. In some embodiments, tocilizumab is administered intravenously at a monthly equivalent dose of 50-500 mg. In some embodiments, tocilizumab is administered intravenously at a monthly equivalent dose of about 50 mg. In some embodiments, tocilizumab is administered intravenously at a monthly equivalent dose of about 100 mg. In some embodiments, tocilizumab is administered intravenously at a monthly equivalent dose of about 150 mg. In some embodiments, tocilizumab is administered intravenously at a monthly equivalent dose of about 250 mg. In some embodiments, tocilizumab is administered intravenously at a monthly equivalent dose of about 350 mg. In some embodiments, tocilizumab is administered intravenously at a monthly equivalent dose of about 500 mg. In some embodiments, tocilizumab is administered subcutaneously at a monthly equivalent dose of 80-800 mg. In some embodiments, tocilizumab is administered subcutaneously at a monthly equivalent dose of about 80 mg. In some embodiments, tocilizumab is administered subcutaneously at a monthly equivalent dose of about 160 mg. In some embodiments, tocilizumab is administered subcutaneously at a monthly equivalent dose of about 240 mg. In some embodiments, tocilizumab is administered subcutaneously at a monthly equivalent dose of about 400 mg. In some embodiments, tocilizumab is administered subcutaneously at a monthly equivalent dose of about 560 mg. In some embodiments, tocilizumab is administered subcutaneously at a monthly equivalent dose of about 800 mg.

In some embodiments, the anti-IL-6R antibody is sarilumab. In some embodiments, sarilumab is administered intravenously at a monthly equivalent dose of 12-120 mg. In some embodiments, sarilumab is administered intravenously at a monthly equivalent dose of about 12 mg. In some embodiments, sarilumab is administered intravenously at a monthly equivalent dose of about 24 mg. In some embodiments, sarilumab is administered intravenously at a monthly equivalent dose of about 48 mg. In some embodiments, sarilumab is administered intravenously at a monthly equivalent dose of about 60 mg. In some embodiments, sarilumab is administered intravenously at a monthly equivalent dose of about 72 mg. In some embodiments, sarilumab is administered intravenously at a monthly equivalent dose of about 120 mg. In some embodiments, sarilumab is administered subcutaneously at a monthly equivalent dose of 20-200 mg. In some embodiments, sarilumab is administered subcutaneously at a monthly equivalent dose of about 20 mg. In some embodiments, sarilumab is administered subcutaneously at a monthly equivalent dose of about 40 mg. In some embodiments, sarilumab is administered subcutaneously at a monthly equivalent dose of about 80 mg. In some embodiments, sarilumab is administered subcutaneously at a monthly equivalent dose of about 100 mg. In some embodiments, sarilumab is administered subcutaneously at a monthly equivalent dose of about 120 mg. In some embodiments, sarilumab is administered subcutaneously at a monthly equivalent dose of about 200 mg.

In some embodiments, the anti-IL-6R antibody is vobarilizumab. In some embodiments, vobarilizumab is administered intravenously at a monthly equivalent dose of 4-120 mg. In some embodiments, vobarilizumab is administered intravenously at a monthly equivalent dose of about 4 mg. In some embodiments, vobarilizumab is administered intravenously at a monthly equivalent dose of about 6 mg. In some embodiments, vobarilizumab is administered intravenously at a monthly equivalent dose of about 30 mg. In some embodiments, vobarilizumab is administered intravenously at a monthly equivalent dose of about 60 mg. In some embodiments, vobarilizumab is administered intravenously at a monthly equivalent dose of about 84 mg. In some embodiments, vobarilizumab is administered intravenously at a monthly equivalent dose of about 120 mg. In some embodiments, vobarilizumab is administered subcutaneously at a monthly equivalent dose of 7-200 mg. In some embodiments, vobarilizumab is administered subcutaneously at a monthly equivalent dose of about 7 mg. In some embodiments, vobarilizumab is administered subcutaneously at a monthly equivalent dose of about 10 mg. In some embodiments, vobarilizumab is administered subcutaneously at a monthly equivalent dose of about 50 mg. In some embodiments, vobarilizumab is administered subcutaneously at a monthly equivalent dose of about 100 mg. In some embodiments, vobarilizumab is administered subcutaneously at a monthly equivalent dose of about 140 mg. In some embodiments, vobarilizumab is administered subcutaneously at a monthly equivalent dose of about 200 mg.

In some embodiments, the IL-6 antagonist is a JAK inhibitor. In some embodiments, the IL-6 antagonist is a STAT3 inhibitor.

### 4. Examples

The following examples are provided by way of exemplification and illustration, not limitation.

### 4.1. Example 1: Phase 1/2 Clinical Study

A Phase 1/2 clinical study was conducted to assess the safety, pharmacokinetics, and pharmacodynamics of multiple IV doses of COR-001.

### 4.1.1. Drug Product (COR-001)

COR-001 (also known as "ziltivekimab" and "MEDI5117") is a human IgG1, kappa antibody directed against interleukin-6 (IL-6). COR-001 contains a "YTE" mutation in its Fc region. The sequence and other features of COR-001 are described in Chapter 2.7.1.1 above.

### 4.1.2. Study Design

The study was a randomized, double-blind, placebo-controlled trial designed to evaluate the safety, pharmacokinetics, and pharmacodynamic effects of multiple doses of COR-001 (ziltivekimab, MEDI5117) or placebo administered to sequential cohorts of hemodialysis patients.

Key inclusion criteria include stage 5 chronic kidney disease (CKD-5) on hemodialysis, positive for TMPRSS6 736A genotype (major allele), IL-6 level greater than 4 pg/mL, and erythropoietic resistive index greater than 8.

Ten hemodialysis patients were randomized to COR-001 or placebo within each dosing cohort. When a higher dose than studied in a prior cohort was initiated, the first 2 (sentinel) patients in that cohort (randomized 1:1 to COR-001 or placebo) were randomized first and the remaining patients were randomized at least 48 hours later, in a 7:1 ratio of COR-001 to placebo. The final ratio of patients treated with COR-001 vs. placebo were 8:2 in each cohort of 10 patients. The maximum tolerated dose (MTD) assessment was based on safety data from Weeks 1 to 3. If more than 2 of 8 active patients in a cohort experienced a dose-limiting toxicity (DLT), the MTD was considered to have been exceeded.

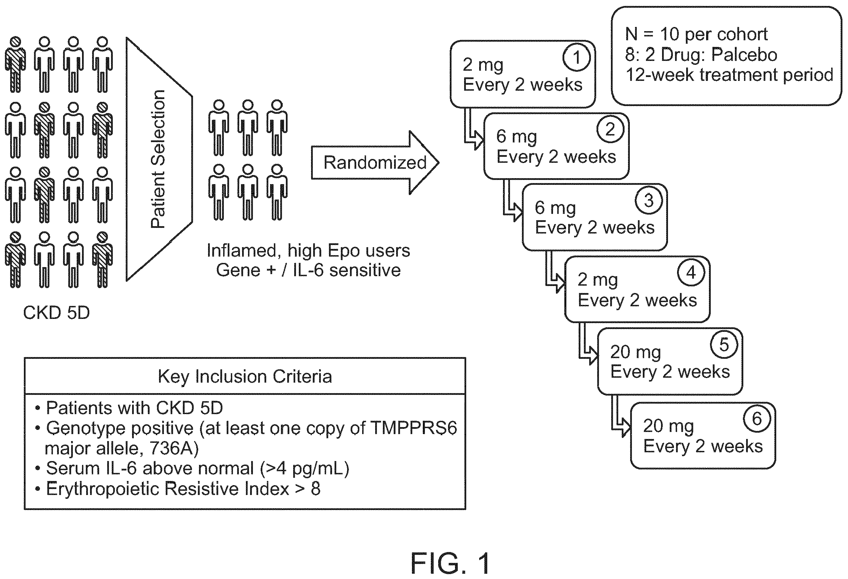

The Dose Escalation Schematic is shown in **FIG. 1****.** COR-001 was administered as an intravenous infusion, started any time before the last 1 hour of the dialysis treatment. The COR-001 dose regimens are shown in **Table 1** below.

| ***Table 1*** | | | |
|---|---|---|---|
| *Dose Cohort* | *Dose Regimen (IV)* | *Number of Doses* | *Total Cumulative Dose* |
| *1* | 2 mg every 14 days | 6 | 12 mg |
| *2* | 6 mg every 14 days | 6 | 36 mg |
| *3* | 6 mg every 14 days | 6 | 36 mg |
| *4* | 2 mg every 14 days | 6 | 12 mg |
| *5* | 20 mg every 14 days | 6 | 120 mg |
| *6* | 20 mg every 14 days | 6 | 120 mg |

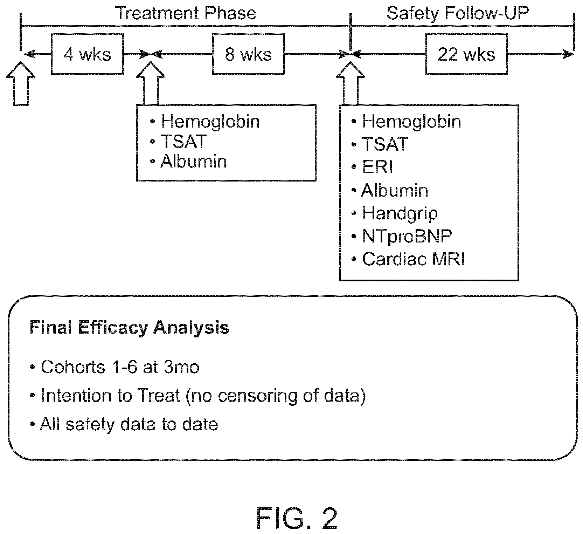

The total study duration for an individual patient was approximately 9 months, excluding the screening period of up to 4 weeks. As shown in **FIG. 2**, the study included a treatment period of 12 weeks (Week 1 through Week 12), a safety follow-up period of 12 weeks (Week 13 through Week 24), and an extended follow-up period of 10 weeks (Week 25 through Week 35).

Interim study-collected data were summarized by treatment group for the appropriate analysis population, using descriptive statistics. Descriptive statistics for continuous variables included number of patients (n), mean, standard deviation (SD), median, quartiles (Q1 and Q3), minimum (min) and maximum (max) values. Analysis of categorical variables included frequency and percentages.

The changes in high-sensitivity C-reactive protein (hsCRP), absolute neutrophil count (ANC), lipoprotein(a) level, LDL level, hemoglobin, transferrin saturation (TSAT), albumin, erythropoietic resistive index (ERI), handgrip, NT-proBNP, and cardiac MRI were recorded during the study.

### 4.1.3. Analysis of Clinical Data

Analyses were performed to determine the effect of COR-001 on C-reactive protein (CRP), the effect of COR-001 on hemoglobin level, the effect of COR-001 on various cardiac parameters, and the effect of COR-001 on levels of neutrophils and platelets.

C-reactive protein (CRP) is a marker of inflammation. CRP levels increase in response to inflammation, and can be measured with an hsCRP (high-sensitivity C-reactive protein) test. The hsCRP level was measured over the course of the treatment period and the safety follow-up period in patients of the placebo-treated, 2 mg dose regimen, 6 mg dose regimen, and 20 mg dose regimen groups, respectively.

The percentages of patients with post-treatment average hsCRP < 2 mg/L at Week 12 were 44%, 62%, and 85% in the 2 mg dose regimen, 6 mg dose regimen, and 20 mg dose regimen groups, respectively, as compared to 14% in the placebo group. The hsCRP responder analysis shows that COR-001 (anti-IL-6) has a superior effect on hsCRP than has been reported for canakinumab (anti-IL-1β) in the CANTOS trial. The hsCRP responder rates of COR-001 in stage 5 chronic kidney disease patients on dialysis at IV doses of 20 mg and 6 mg (**FIG. 3A**) were higher than the hsCRP responder rates of canakinumab at equivalent doses in the CANTOS trial (**FIG. 3B**). The *in vivo* IC50 concentration of COR-001 for CRP (50% reduction of baseline CRP) is 206 ng/mL.

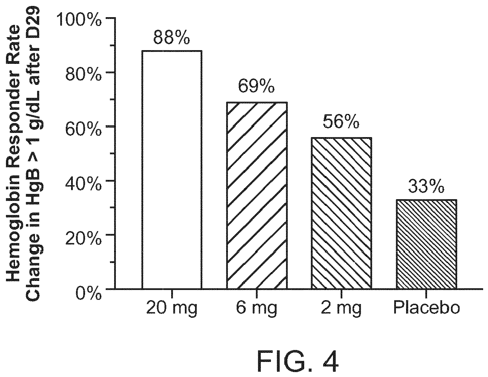

COR-001 improved a primary indicator of anemia, hemoglobin levels. The hemoglobin responder analysis indicated a dose-dependent hemoglobin responder rate of COR-001 treatment (**FIG. 4**).

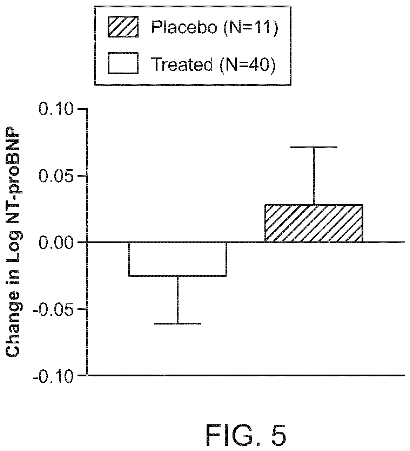

The effect of COR-001 on various biomarkers of heart failure was determined. As shown in **FIG. 5**, COR-001 decreased the level of the N-terminal prohormone of brain natriuretic peptide (NT-proBNP). The result indicates that treatment of COR-001 can reduce heart failure.

Anti-inflammatory therapies in general, and IL-6 inhibitory therapies in particular, create a risk of inducing immune suppression, thereby promoting the emergence of infections, sometimes serious in nature. Immune suppression can be measured by neutrophil counts. The effect of COR-001 on neutrophil counts was determined.

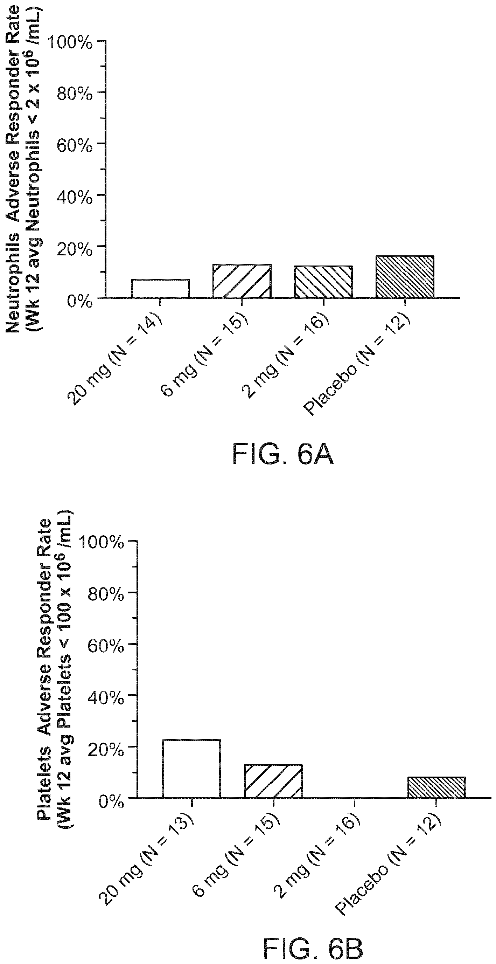

Surprisingly, despite significant reduction in inflammation, as measured by hsCRP levels (**FIG. 3A**), the absolute neutrophil count of patients treated with COR-001 did not decline to below normal levels. No opportunistic infections were observed during the treatment. As shown in **FIG. 6A**, the percentages of patients with absolute neutrophil count below 2.0×10⁹/L were not increased with COR-001 at all tested doses as compared to the placebo group. All patients treated with COR-001 at all tested doses had an absolute neutrophil count above 1.5×10⁹/L. The *in vivo* IC50 concentration of COR-001 for neutrophil count (50% reduction of baseline neutrophil count) is 5540 ng/mL.

The percentages of patients with platelet count below 100×10⁹/L were less than 30% with COR-001 for all tested doses (**FIG. 6B**). The *in vivo* IC50 concentration of COR-001 for platelet count (50% reduction of baseline platelet count) is 13800 ng/mL.

In summary, the clinical data indicate that COR-001 treatment at doses of 2 mg, 6mg, and 20 mg can reduce inflammation without inducing immune suppression in patients with stage 5 chronic kidney disease (CKD-5) on dialysis, whereas the absolute neutrophil count was not decreased significantly in patients treated with COR-001.

Administration of COR-001 reduced CRP in a dose-dependent matter. In addition, COR-001 increased hemoglobin level in these patients. COR-001 decreased the biomarkers of heart failure NT-proBNP.

### 4.2. Example 2: A Phase 2, Randomized, Double-Blind, Placebo-Controlled Trial to Evaluate REduction in Inflammation in PatientS with advanced Chronic Renal Disease Utilizing Antibody MEdiated IL-6 inhibition (RESCUE)

A Phase 2 clinical study was conducted to confirm the efficacy, safety, and pharmacokinetics of multiple subcutaneous (SC) doses of Ziltivekimab.

### 4.2.1. Ziltivekimab (COR-001)

Ziltivekimab (also known as COR-001, MEDI5117) is an extended half-life anti-IL-6 antibody (human IgG1κ anti-human IL-6 monoclonal antibody) with three amino acid substitutions ("YTE") in the CH2 region of the Fc domain. The sequence and other features of Ziltivekimab are described in Chapter 2.7.1.1 above.

### 4.2.2. Objectives of the Study

An objective was to evaluate the effects of Ziltivekimab compared to placebo on markers of inflammation (i.e. hs-CRP).

Additional objectives were to evaluate the effects of Ziltivekimab compared to placebo on markers of inflammation and cardiovascular risk: serum amyloid A (SAA) and fibrinogen.

Further objectives included evaluating the effects of Ziltivekimab compared to placebo on markers of cardiovascular risk (i.e. N-terminal prohormone-B-type natriuretic peptide (NT-pro-BNP)), atherosclerosis risk (i.e. LDL-C, triglycerides, ApoB, ApoA1, ApoB/ApoA1, lipid profile by nuclear magnetic resonance (NMR) spectroscopy, and Lp(a)), anemia (i.e. hemoglobin), inflammation-malnutrition (i.e. albumin), and kidney function, (i.e. cystatin C, estimated GFR) and kidney damage (i.e. urine albumin-to-creatinine ratio (UACR)).

The pharmacokinetic objectives were to evaluate the pharmacokinetics (PK) and PK-pharmacodynamic (PK-PD) modeling of Ziltivekimab following multiple doses at three different levels.

The safety objectives were to evaluate the safety of three dose levels of Ziltivekimab compared to placebo.

### 4.2.3. Pharmacodynamic Endpoints

A pharmacodynamic endpoint was the difference in percent change in hs-CRP levels from Baseline (average of the hs-CRP value prior to randomization and Day 1) to Week 13 between each active group and placebo.

Additional pharmacodynamic endpoints included: the difference in change in SAA from Baseline to Week 13 between each active group and placebo; and the difference in percent change in fibrinogen from Baseline to Week 13 between each active group and placebo.

Further pharmacodynamic endpoints included:
- Difference in percent change in hs-CRP levels from Baseline to End of Treatment (Weeks 23 through 24) between each active group and placebo.
- Difference in percent change in serum NT-pro-BNP from Baseline (average of the NT-pro-BNP value prior to randomization and Day 1) to Week 13 and to End of Treatment (Weeks 23 through 24) between each active group and placebo.
- Difference in change in SAA from Baseline to End of Treatment (Weeks 23 through 24) between each active group and placebo.
- Difference in percent change in fibrinogen from Baseline to End of Treatment (Week 24) between each active group and placebo.
- Difference in change in hemoglobin from Baseline (average of the two most recent hemoglobin values prior to randomization and Day 1) to Week 13 and to End of Treatment (Weeks 23 through 24) between each active group and placebo.
- Difference in change in serum albumin from Baseline (average of the two most recent serum albumin values prior to randomization and Day 1) to Week 13 and to End of Treatment (Weeks 23 through 24) between each active group and placebo.
- Proportion of patients achieving hs-CRP response at Week 13 and to End of Treatment (Weeks 23 through 24), defined as hs-CRP <2.0 mg/L in each active group and placebo.
- Difference in change in ST2 from Baseline to Week 13 and to End of Treatment (Week 24) between each active group and placebo.
- Difference in percent change in Lp(a) from Baseline to Week 13 and to End of Treatment (Weeks 23 through 24) between each active group and placebo.
- Difference in percent change in LDL-C, triglycerides, ApoB, ApoA1, ApoB/ApoA1, and lipid profile by NMR spectroscopy from Baseline to Week 13 and to End of Treatment (Weeks 23 through 24) between each active group and placebo.
- Difference in change of creatinine based eGFR and cystatin C-based eGFR from Baseline to Week 13 and to the End of Treatment (Weeks 23 through 24) between each active group and placebo.
- Difference in change of UACR from Baseline to Week 13 and to End of Treatment (Weeks 23 through 24) between each active group and placebo.
- Difference in change of the total fatigue score (PROMIS Fatigue 13a short form and selected items from the PROMIS Fatigue item bank) from Baseline to Week 13 and to End of Treatment (Weeks 23 through 24) between each active group and placebo.
- Difference in change of the PROMIS interest in sexual activity item from Baseline to Week 13 and to End of Treatment (Week 24) between each active group and placebo.
- Difference in change of the Corvidia ePRO items from Baseline to Week 12 and to End of Treatment (Weeks 23 through 24) between each active group and placebo.
- Difference in change of the PGIS index from Baseline to Week 12 and to End of Treatment (Week 24) between each active group and placebo.
- Descriptive analyses of the PGIC index at Weeks 5, 13 and End of Treatment (Week 24) in each active group and placebo.
- Difference in change of the Optum SF-36 v2^{®} Health Survey physical component summary (PCS), mental component summary (MCS) and domain scores from baseline to Week 13 and to End of Treatment (Weeks 23 through 24) between each active group and placebo.
- Difference in change of the EQ-5D-5L index from Baseline to Week 12 and to End of Treatment (Week 24) between each active group and placebo.
- Evaluation of the psychometric properties of the PROMIS Fatigue 13a short form and selected items from the PROMIS Fatigue item bank and the Corvidia ePRO items in CKD patients - to be described in a PRO psychometric analysis plan.
- Descriptive analyses by dose and treatment may be conducted on samples stored for analysis of exploratory biomarkers, genomic and transcriptomic analysis.
- Difference in change in TSAT from Baseline to peak level, Week 13 and End of Treatment (Weeks 23 through 24), between each active group and placebo.
- Difference in change in CHr from Baseline to peak level, Week 13 and to End of Treatment (Week 24) between each active group and placebo.
- Difference in change in TIBC from Baseline to Week 13 and to End of Treatment (Weeks 23 through 24) between each active group and placebo.
- Difference in change in serum ferritin from Baseline to Week 13 and to End of Treatment (Weeks 23 through 24) between each active group and placebo.
- Difference in change in serum iron from Baseline to Week 13 and to End of Treatment (Weeks 23 through 24) between each active group and placebo.
- Difference in change in serum hepcidin from Baseline to Week 13 and to End of Treatment (Week 24) between each active group and placebo.
- Difference in percent change in serum NT-pro-BNP from Baseline to Week 13 and to End of Treatment (Weeks 23 through 24) between each active group and placebo in patients with baseline NT-pro-BNP > 250 pg/mL.
- Difference in change in hemoglobin from Baseline (average of the two most recent hemoglobin values prior to randomization and Day 1) to Week 13 and to End of Treatment (Weeks 23 through 24) between each active group and placebo in patients with baseline hemoglobin < 11 g/dL.
- Difference in change in serum albumin from Baseline (average of the two most recent serum albumin values prior to randomization and Day 1) to Week 13 and to End of Treatment (Weeks 23 through 24) between each active group and placebo in patients with baseline albumin < 4.0 g/dL.

### 4.2.4. Study Design

This was a randomized, double-blind, placebo-controlled trial designed to evaluate the efficacy, safety, and pharmacokinetics of Ziltivekimab at three dose levels (7.5 mg, 15 mg and 30 mg s.c. once every 28 days) compared to placebo in patients with stage 3-5 CKD, not on dialysis, who have evidence of inflammation with high cardiovascular risk.

Key inclusion criteria included stage 3-5 non-dialysis dependent chronic kidney disease (NDD-CKD) and serum hs-CRP level ≥ 2.0 mg/L measured during the screening period.

264 patients were randomized 1:1:1:1 (66 per group) to Ziltivekimab Dose #1 (7.5 mg), Ziltivekimab Dose #2 (15 mg), Ziltivekimab Dose #3 (30 mg), or Placebo as they became eligible to proceed with dosing on Day 1. Patient randomization was stratified by baseline hemoglobin (≥11 or <11 g/dL) and CKD stage (3, 4 or 5).

The study consisted of three periods: screening period (up to 2 weeks; Days -14 to -1), treatment assessment period (24 weeks; Week 1, Day 1 through Week 24), and safety follow-up period (8 weeks; Week 25 through 32), with the total study duration for an individual patient lasting approximately 8-9 months. Patients were dosed once every 28 days out to Week 21 (i.e., a total of 6 treatments on Weeks 1, 5, 9, 13, 17, and 21).

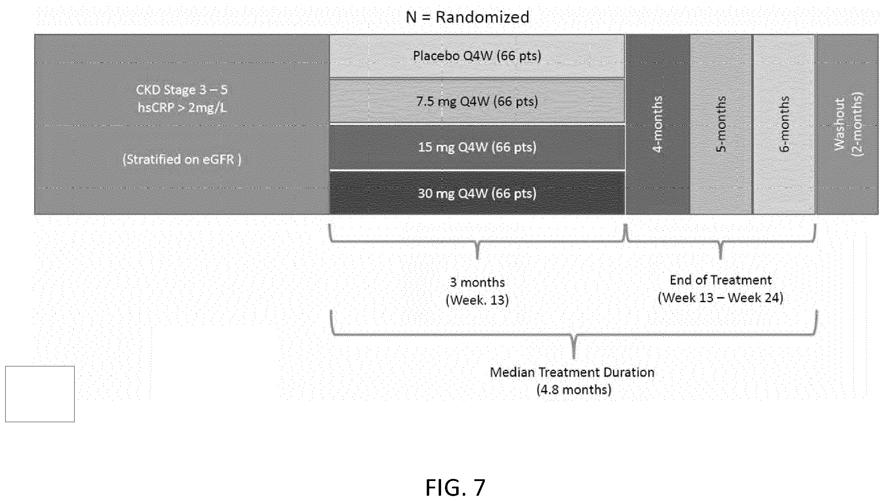

The schematic of the study design is shown in **FIG. 7****.**

### 4.2.5. Analysis of Clinical Data

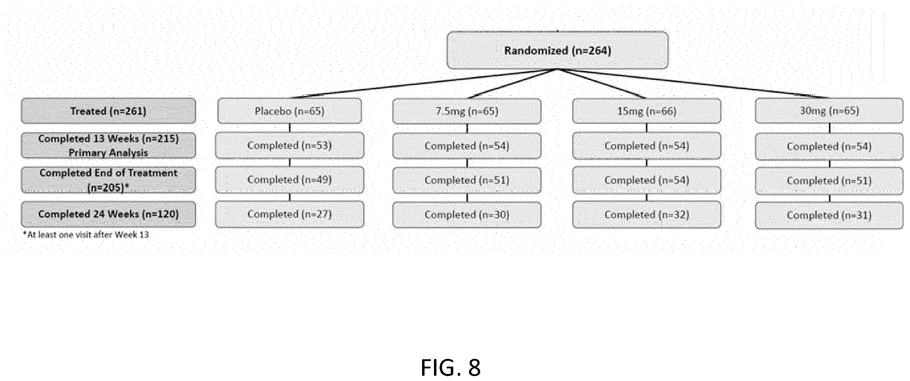

Among the 264 randomized patients, 261 patents were treated with Ziltivekimab or placebo. 215 patients completed 13 weeks of study. 205 patients completed end of treatment (at least one visit after week 13). 120 patients completed 24 weeks of study. The schematic of the patient disposition is shown in **FIG. 8****.**

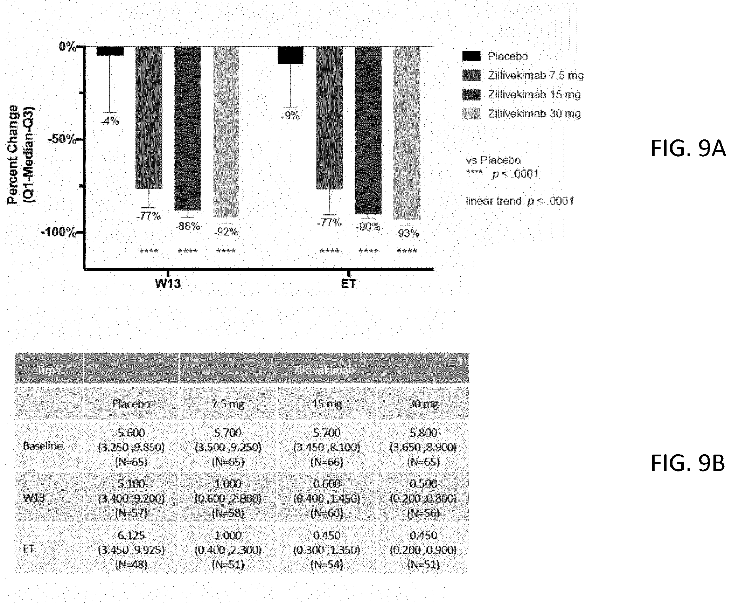

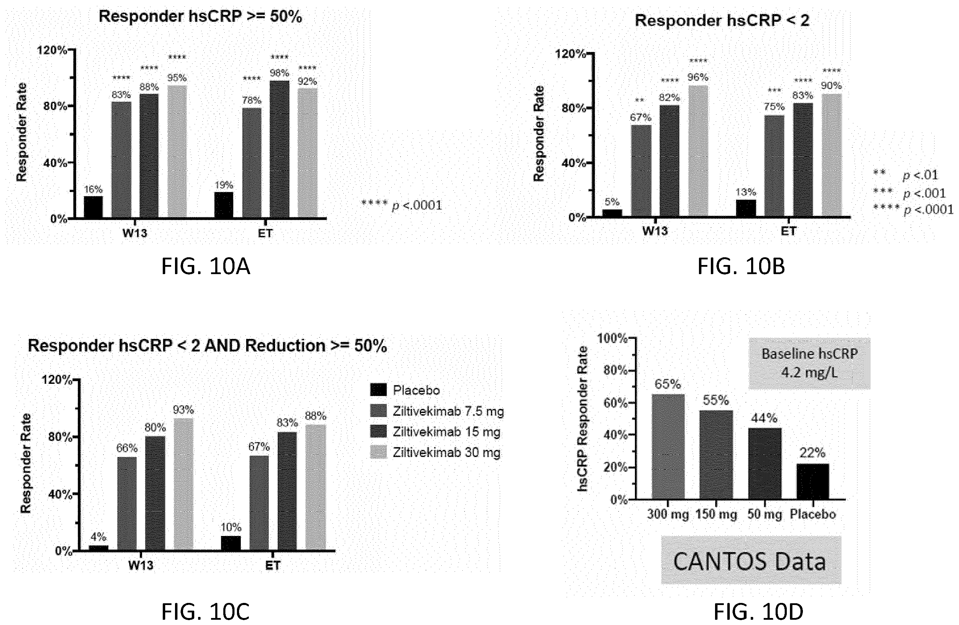

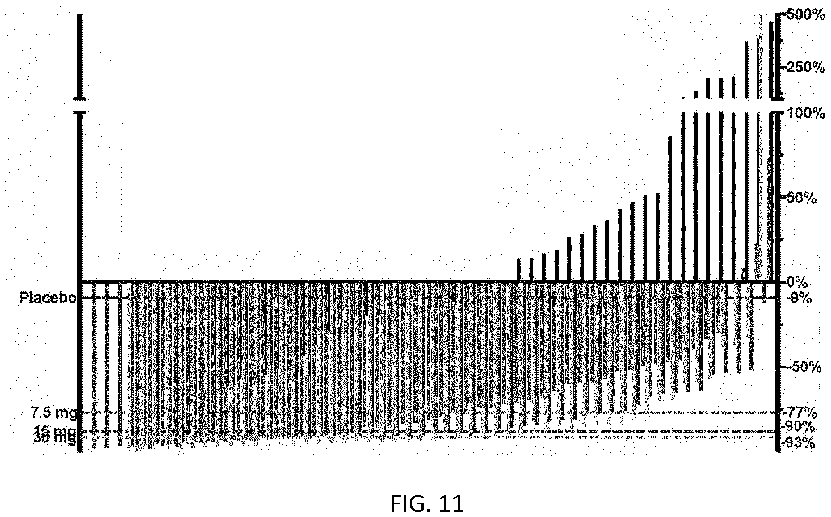

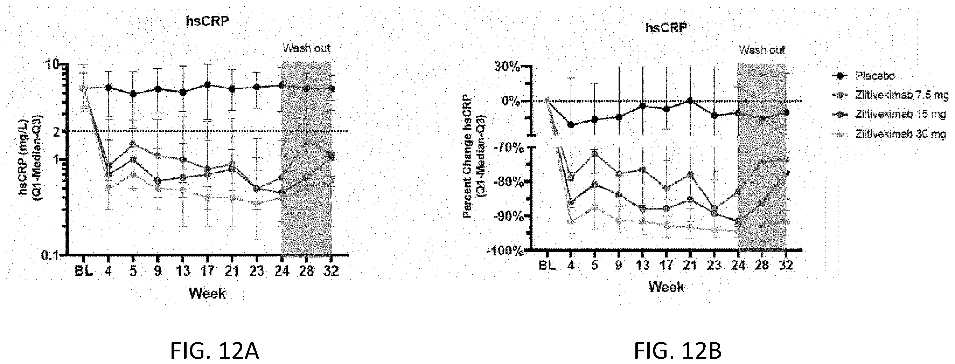

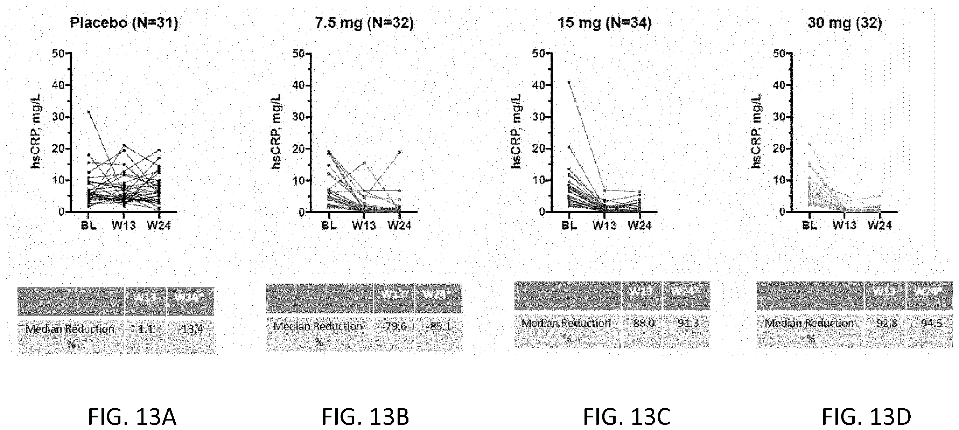

To evaluate the effects of Ziltivekimab compared to placebo on markers of inflammation, serum high-sensitivity C-reactive protein (hsCRP) level was measured. Patients who were treated with Ziltivekimab showed a medium percent reduction in hsCRP of 77%, 88%, and 92% at 7.5 mg, 15 mg, and 30 mg respectively at week 13 compared to baseline and a medium percent reduction in hsCRP of 77%, 90%, and 93% at 7.5 mg, 15 mg, and 30 mg respectively at end of treatment compared to baseline (**FIGs 9A and 9B**). In comparison, only 4% and 9% hsCRP reduction was observed with placebo treated group at week 13 and end of treatment respectively. As shown in **FIGs 10A-10D****,** the responder rate achieved with Ziltivekimab defined by hsCRP reduction ≥ 50%, hsCRP level < 2 mg/L, or hsCRP reduction ≥ 50% and hsCRP level < 2 mg/L is superior than the responder rate achieved with canakinumab (anti-IL1β) in the CANTOS trial. *See* Ridker et al., 2018, J Am Coll Cardiol; 71:2405-2414. Notably, Ziltivekimab treatment at 15 mg and 30 mg showed about 90% median percent reduction in hsCRP and very few non-responders at end of treatment (**FIG. 11**). The strong suppression of hsCRP was maintained during the entire treatment period of 24 weeks in patient groups treated with Ziltivekimab (**FIGs 12A and 12B****;** **FIGs 13A-13D**).

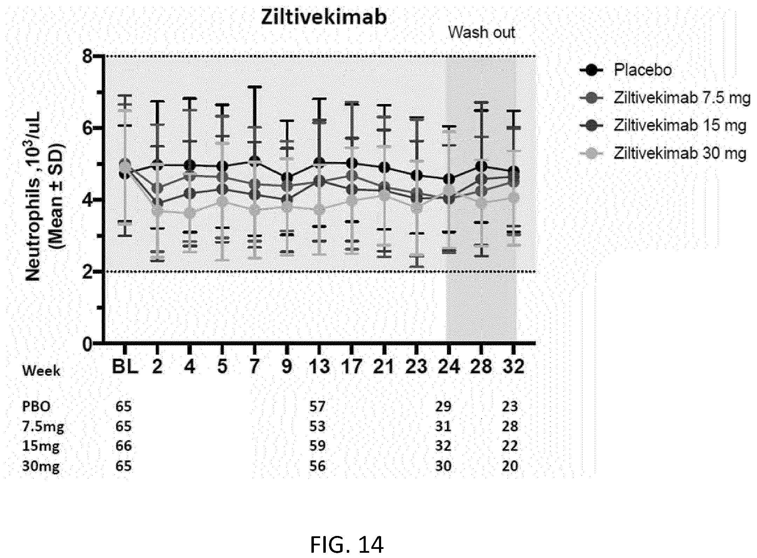

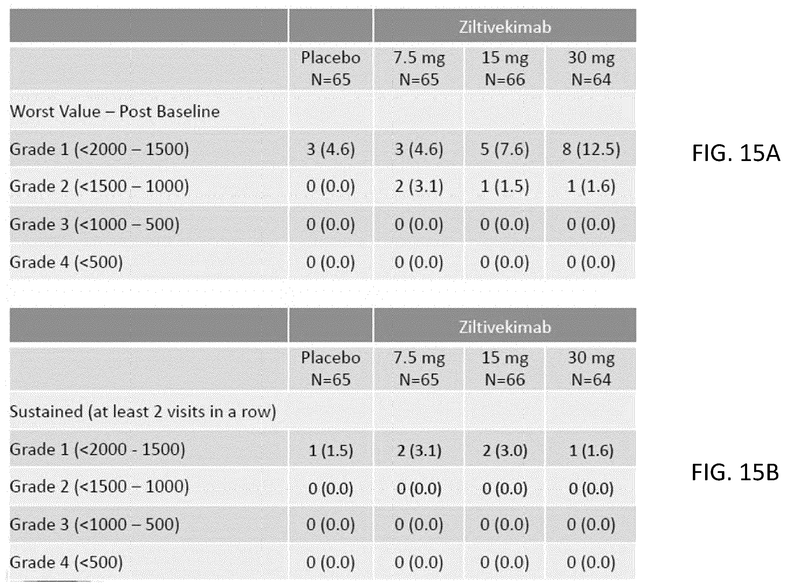

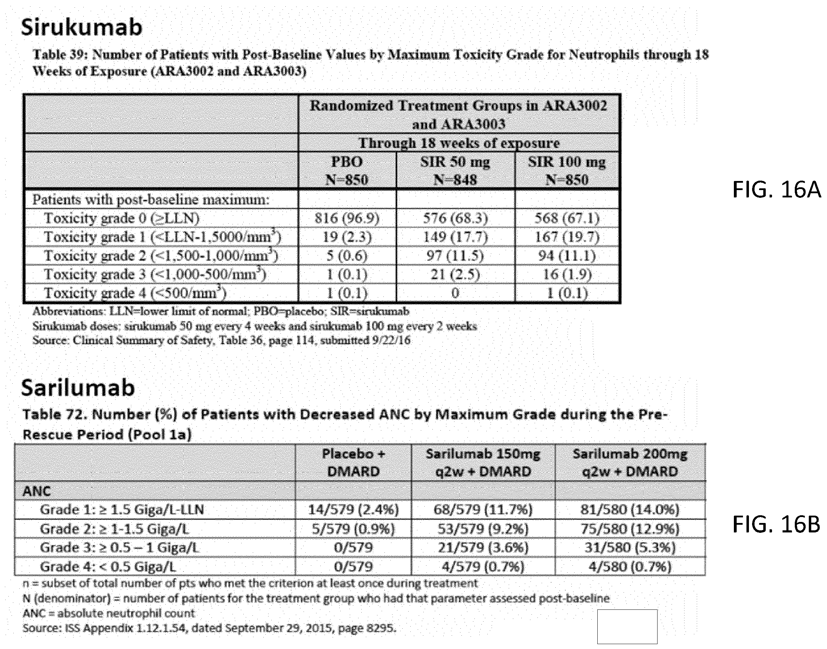

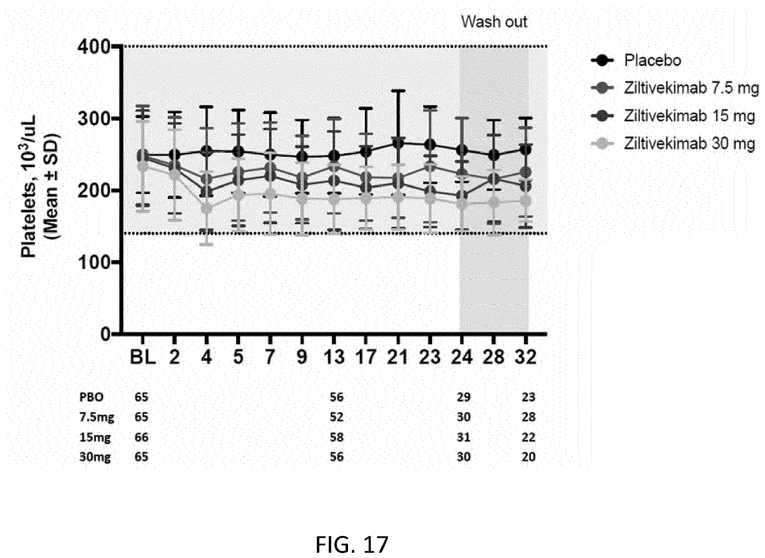

Despite the significant reduction in inflammation, as measured by hsCRP, Ziltivekimab did not reduce the neutrophil count (**FIG. 14**) or the platelet number (**FIG. 17**) below the normal ranges (normal range of ANC = 8.0 × 10⁹/L to 2.0 × 10⁹/L; normal range of platelet count = 400 × 10⁹/L to 120 × 10⁹/L). As shown in **FIG. 15A****,** very few patients (2, 1, and 1 with Ziltivekimab at 7.5 mg, 15 mg, and 30 mg respectively) exhibited neutropenia of Grade 2 or above. No patient exhibited Grade 2 or above sustained neutropenia with Ziltivekimab (**FIG. 15B**). In contrast, as reported in the literature, 14% and 13.1% patients had neutropenia of Grade 2 or above with sirukumab treatment at 50 mg and 100 mg respectively (**FIG. 16A**), and 13.5% and 18.9% patients had neutropenia of Grade 2 or above with sarilumab treatment at 150 mg and 200 mg respectively (**FIG. 16B**).

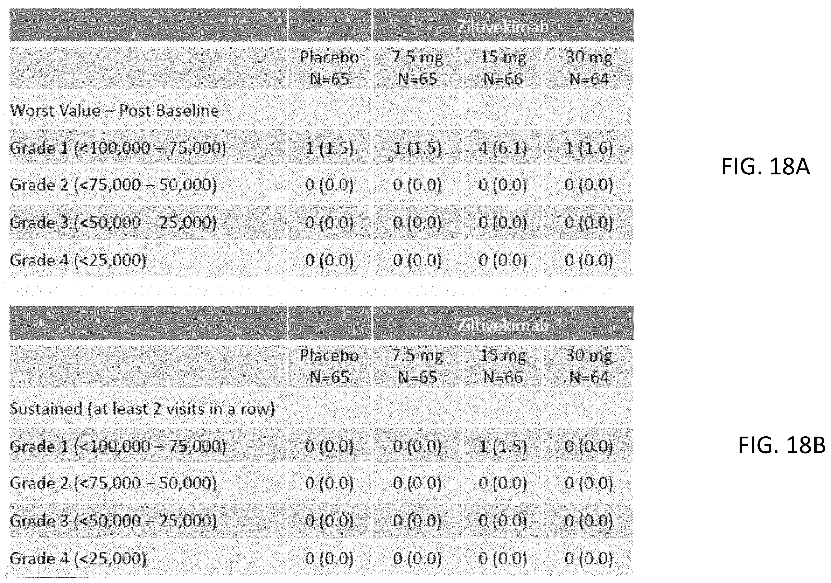

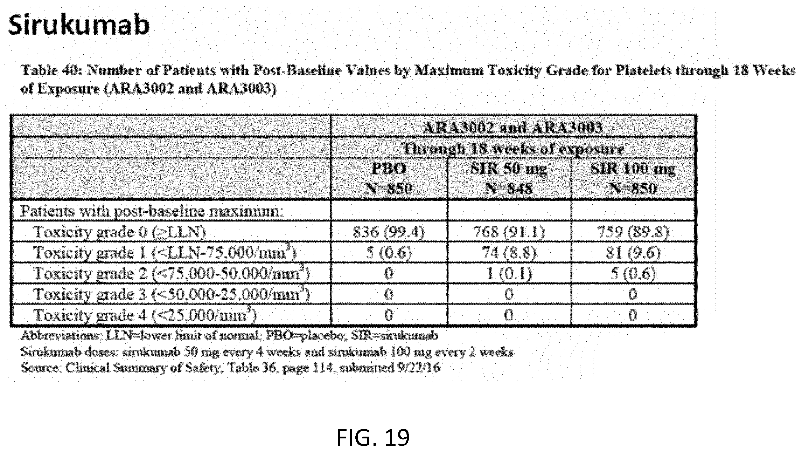

As shown in **FIGs. 18A and 18B**, no patient exhibited thrombocytopenia or sustained thrombocytopenia of Grade 2 or above with Ziltivekimab treatment. In contrast, as reported in the literature, Grade 2 thrombocytopenia was detected in patients treated with sirukumab at both 50 mg and 100 mg albeit at small percentages (**FIG. 19**).

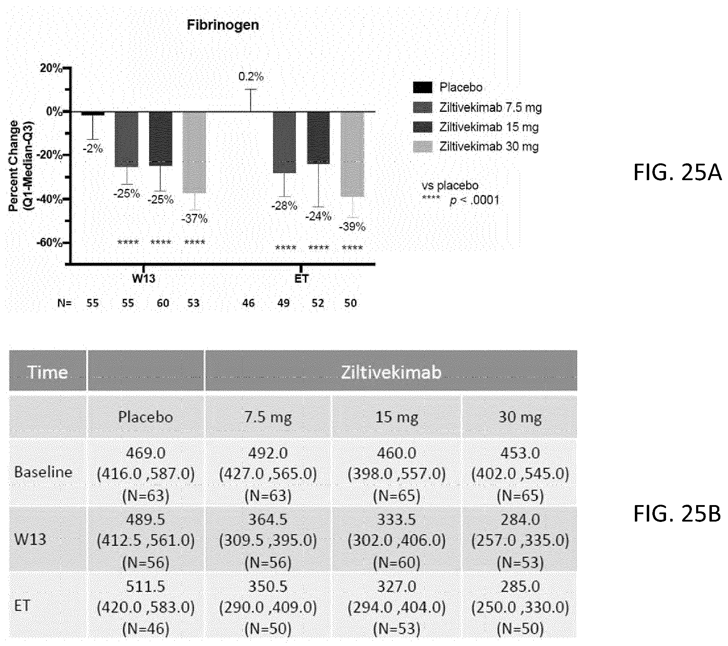

Fibrinogen is an acute phase reactant and a mediator of coagulation, and therefore a marker of cardiovascular risk. *See* Ridker et al., 2012, Circulation, 126:2739-2748. Percent changes in fibrinogen from baseline to week 13 and to end of treatment were determined. Ziltivekimab at 7.5 mg, 15 mg, and 30 mg significantly reduced fibrinogen (**FIGs. 25A and 25B**).

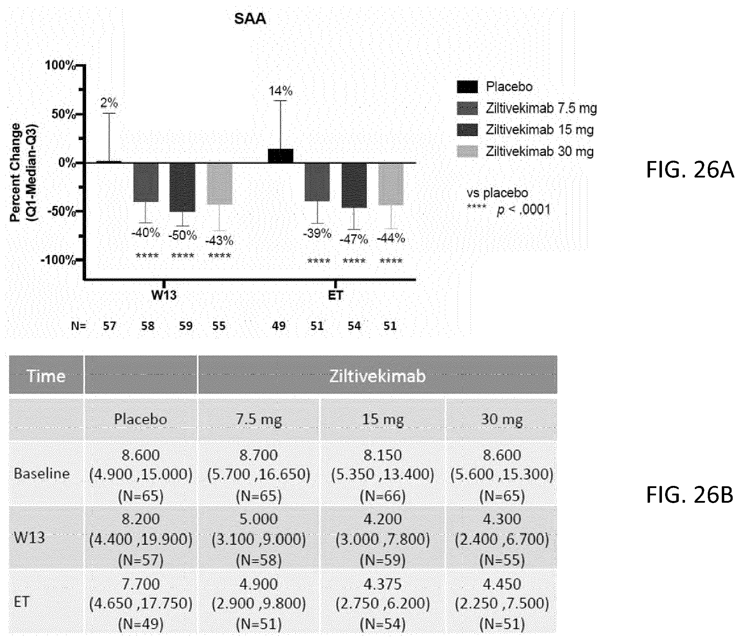

Serum Amyloid A (SAA) is another acute phase reactant that is persistently elevated in chronic inflammatory conditions. Elevated levels of SAA predict cardiovascular risk in humans. *See* Shridas et al., 2019, Curr Opin Lipidol., 30:320-325. Percent changes in SAA from baseline to week 13 and to end of treatment were determined. Ziltivekimab at 7.5 mg, 15 mg, and 30 mg had sustained robust effect in reducing SAA levels (**FIGs. 26A and 26B**).

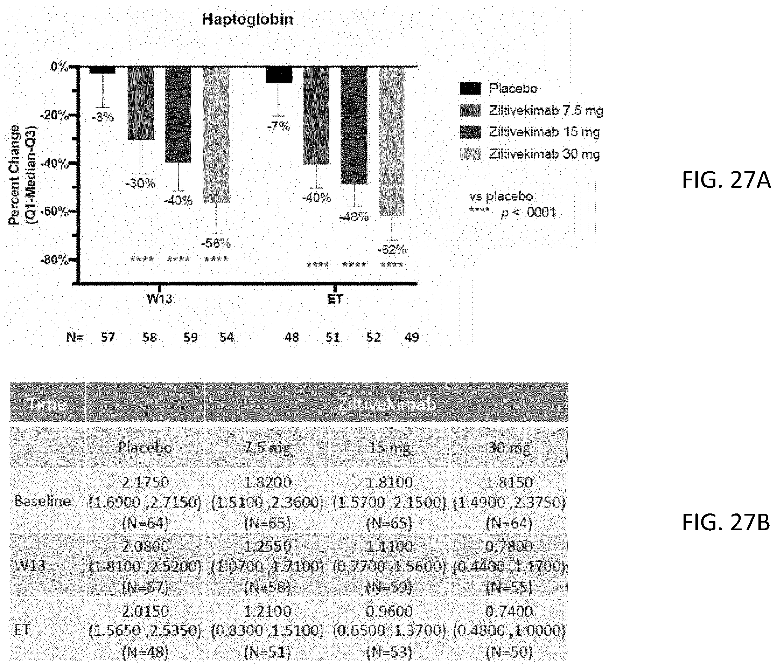

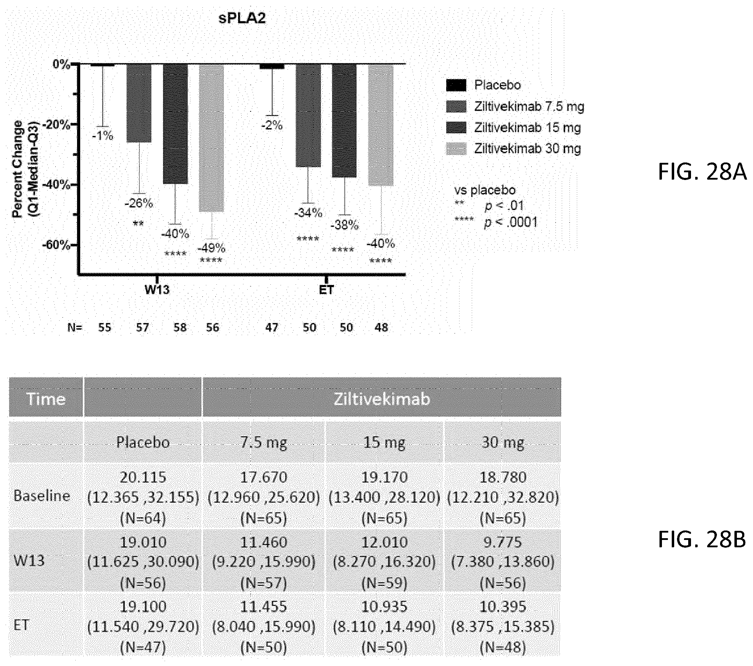

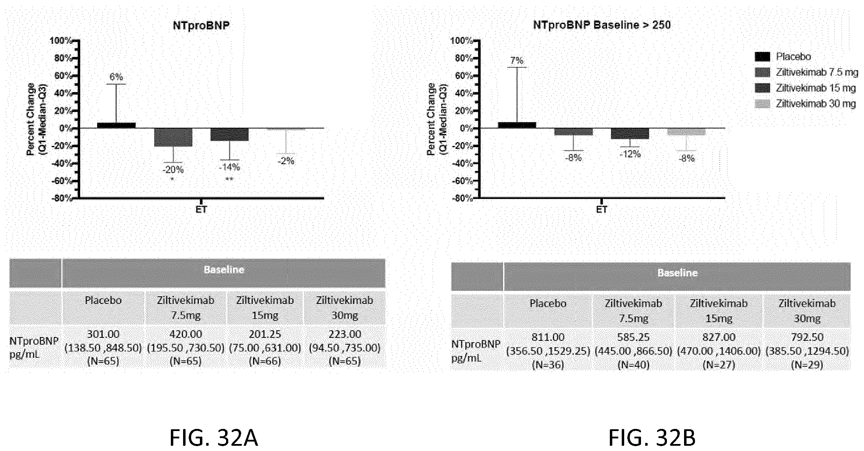

Haptoglobin, an established risk factor for cardiovascular disease, showed dose-dependent reduction with Ziltivekimab treatment at 7.5 mg, 15 mg, and 30 mg (**FIGs. 27A and 27B**). Ziltivekimab also decreased secretory phospholipase A2 (sPLA2), a vascular inflammatory marker, in a dose-dependent manner (**FIGs. 28A and 28B**). Additionally, consistent with the results from the Phase 1/2 Clinical Study described in Example 1, Ziltivekimab decreased the level of NTproBNP by 20% and 14% at 7.5 mg and 15 mg respectively, suggesting a potential benefit for congestive heart failure (CHF) (**FIGs. 32A and 32B**).

The effect of Ziltivekimab on markers of atherosclerosis risk was determined by evaluating the changes in lipoprotein (a) (Lp(a)), low density lipoprotein (LDL), high density lipoprotein (HDL), triglyceride (TG), total cholesterol/high density lipoprotein (TC/HDL), apolipoprotein B (ApoB), apolipoprotein A1 (ApoA1), and the ratio of ApoB/ApoA1.

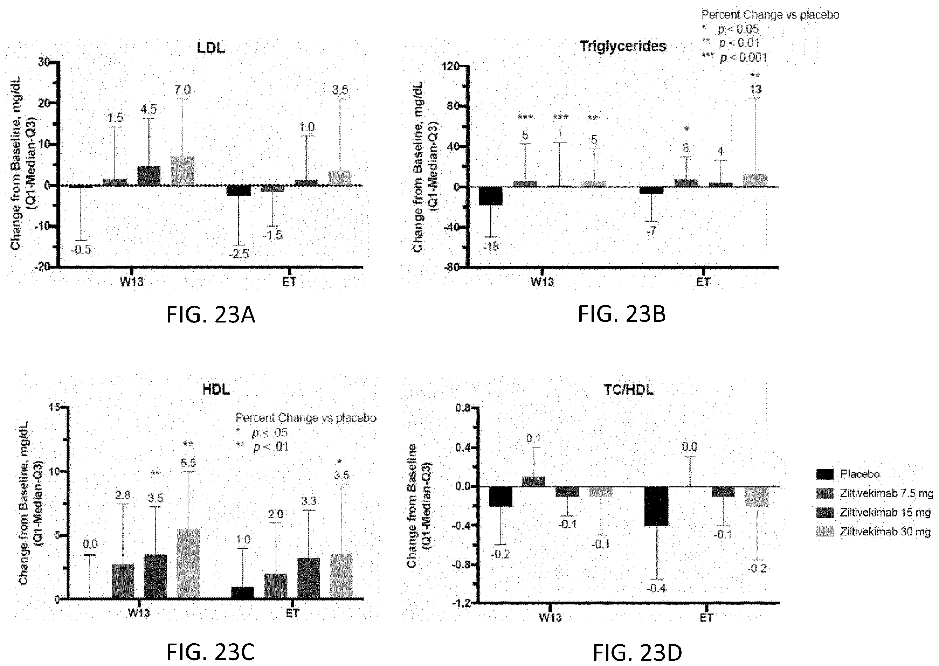

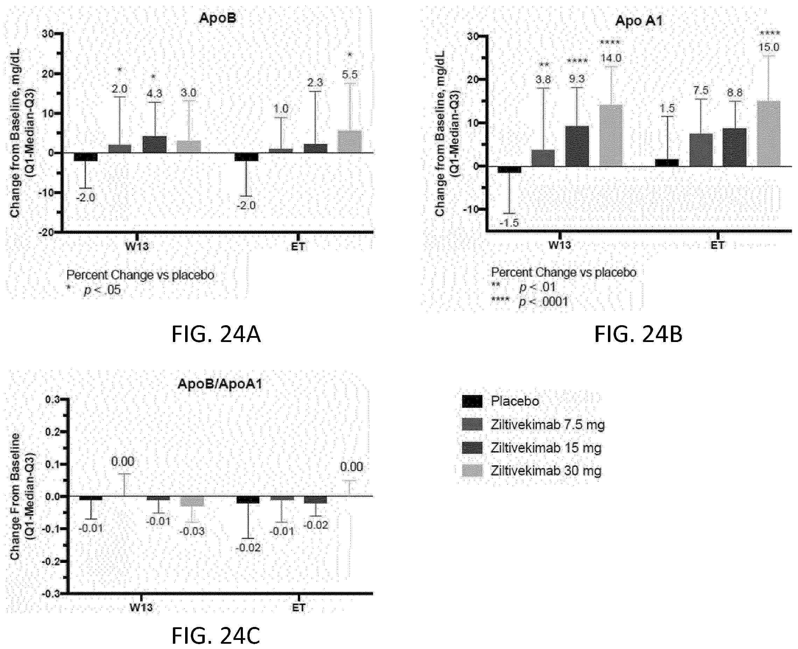

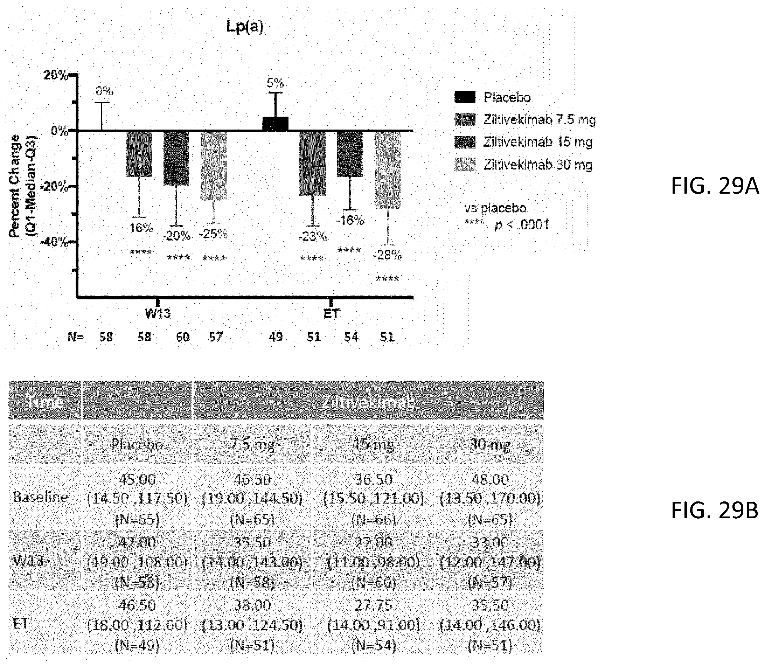

Ziltivekimab at 7.5 mg, 15 mg, and 30 mg significantly reduced the atherosclerosis risk factor, Lp(a), at week 13 and end of treatment compared to placebo (**FIGs. 29A and 29B**). Ziltivekimab did not significantly increase the LDL level (**FIG. 23A**). Modest increases in TG levels were observed in patients treated with Ziltivekimab (**FIG**. **23B**). Ziltivekimab improved the HDL level compared to placebo (**FIG. 23C**). In addition, Ziltivekimab at 7.5 mg, 15 mg, and 30 mg did not significantly change the ApoB/ApoA1 ratio (**FIG 24C**).

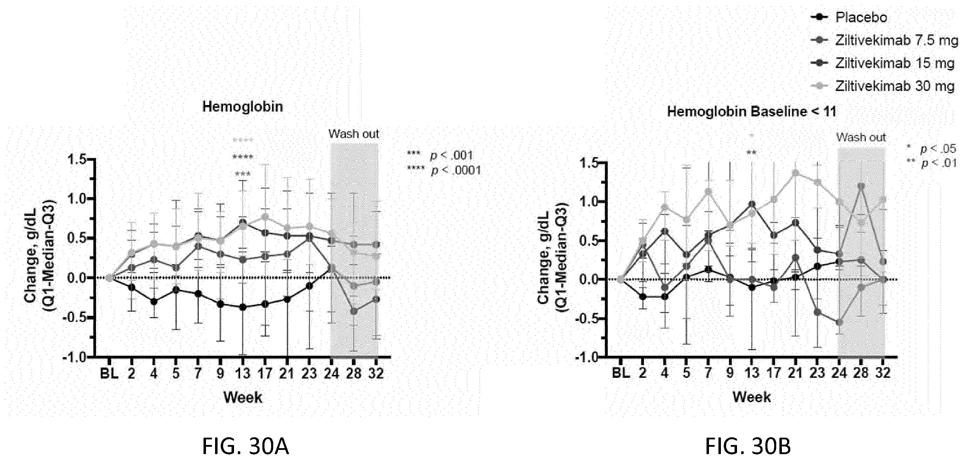

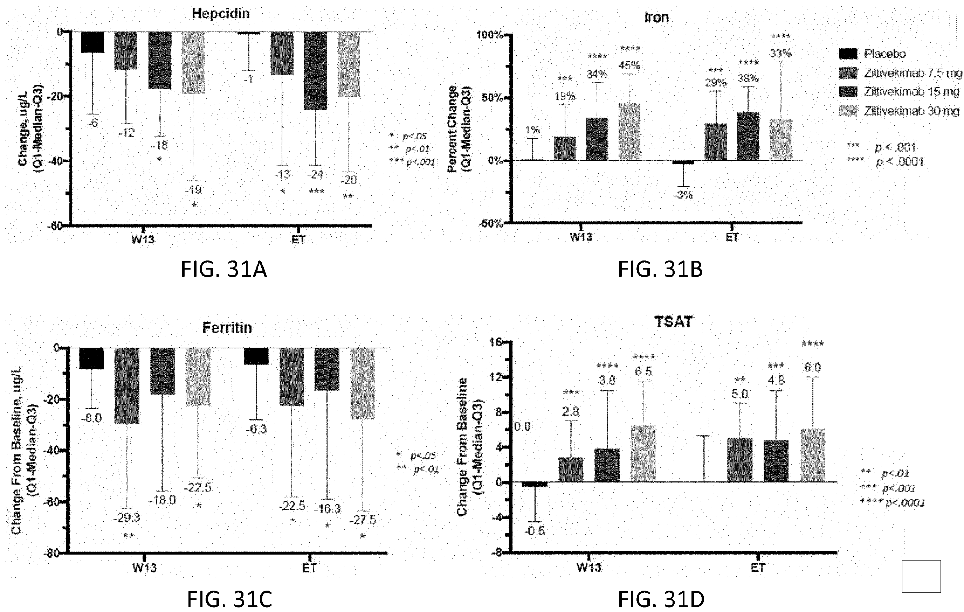

To determine the effect of Ziltivekimab on inflammatory anemia, the changes in hemoglobin, hepcidin, iron, ferritin, and transferrin saturation (TSAT) were determined. Ziltivekimab at 7.5 mg, 15 mg, and 30 mg increased hemoglobin level (**FIG. 30A**). In the anemia subgroup with hemoglobin baseline level < 11 g/dL, Ziltivekimab at 15 mg and 30 mg significantly improved hemoglobin level (**FIG. 30B**). Ziltivekimab also improved additional iron indices hepcidin, iron, ferritin, and TSAT (**FIGs. 31A-D**).

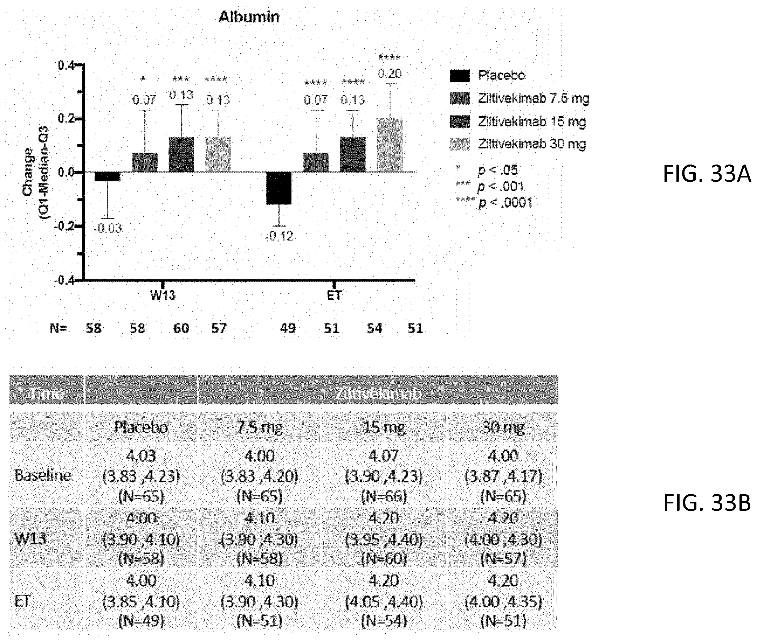

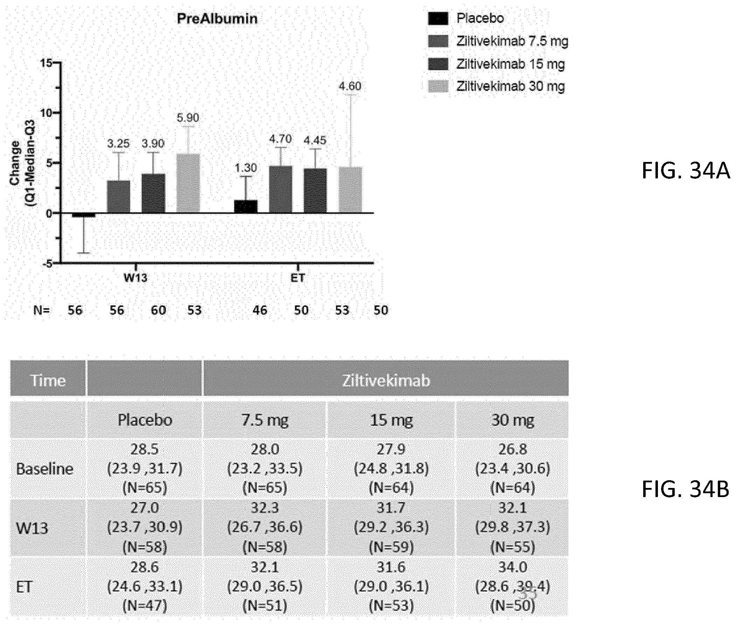

Persistent inflammation in CKD is also one of the key players in the development of malnutrition/protein-energy wasting (PEW), which led to the description of the malnutrition-inflammation-cachexia syndrome (MICS) in CKD. *See* Kalantar-Zadeh et al., 2003, Am J Kidney Dis, 42:761-773 and Kalantar-Zadeh et al., 2005, J Am Soc Nephrol, 16:3070-3080. To determine the effect of Ziltivekimab on inflammation-malnutrition, the changes in albumin and prealbumin were determined. Ziltivekimab increased the levels of both albumin and prealbumin (**FIGs. 33A and 33B****;** **FIGs. 34A and 34B**), suggesting the potential benefit of Ziltivekimab in improving inflammation-malnutrition in CKD patients.

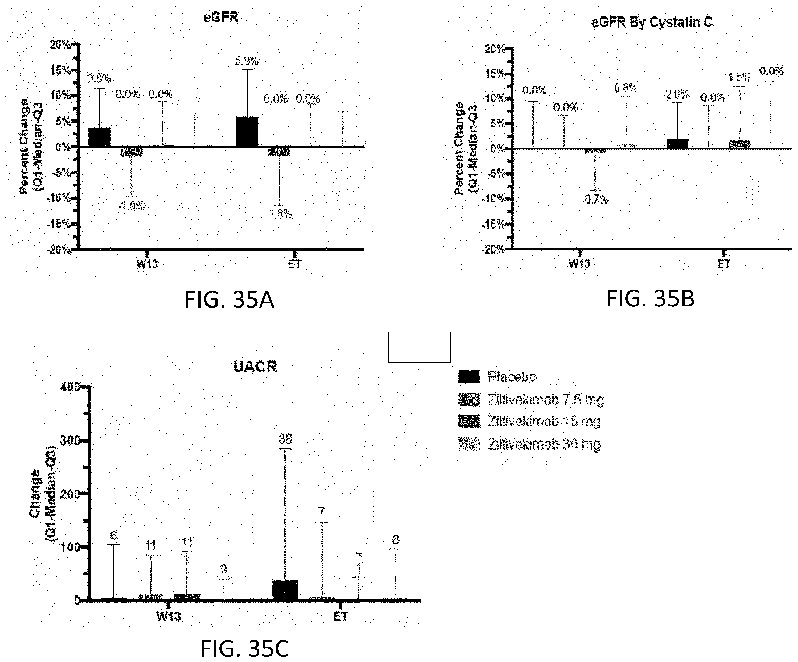

No significant effect of Ziltivekimab on creatinine-based eGFR or cystatin C-based eGFR was observed at week 13 or end of treatment (**FIGs. 35A and 35B**). Ziltivekimab also did not significantly impact the urine albumin-to-creatinine ratio (UACR) (**FIG. 35C**).

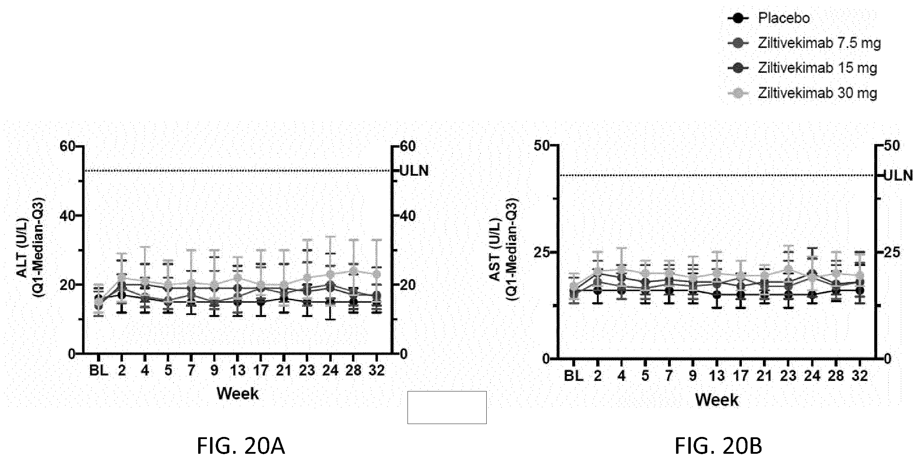

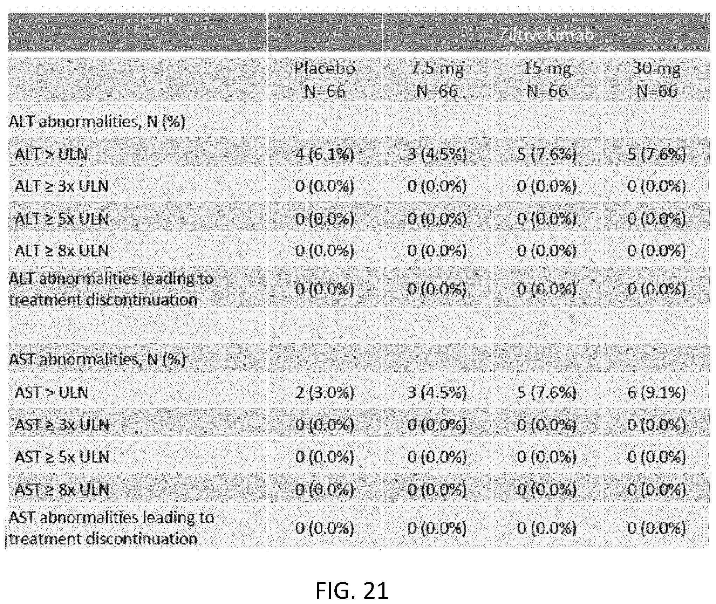

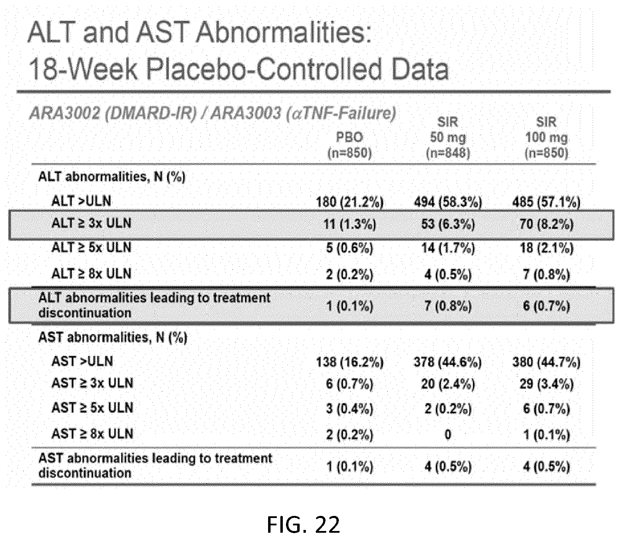

As indicators of liver toxicity, alanine transaminase (ALT) and aspartate transaminase (AST) levels were measured. **FIGs. 20A and 20B** show that Ziltivekimab did not significantly increase the levels of ALT and AST at 7.5 mg, 15 mg, or 30 mg. No ALT abnormality (ALT ≥ 3×ULN) or AST abnormality (AST ≥ 3×ULN) was found in patients treated with Ziltivekimab (**FIG. 21**). In contrast, as reported in the literature, in patients treated with 50 mg and 100 mg sirukumab, 6.3% and 8.2% patients respectively had ALT levels ≥ 3×ULN, and 2.4% and 3.4% patients had AST levels ≥ 3×ULN (**FIG. 22**).

As shown in **Table 2** and **Table 3** below, the numbers of adverse events and the adverse events of special interest did not significant increase in patients treated with Ziltivekimab compared to placebo.

| **Table 2: adverse events and serious adverse events** | | | | |
|---|---|---|---|---|
| **AE/SAE type** | **Placebo** | **7.5mg** | **15mg** | **30mg** |
| SAE Death | 1 | 0 | 0 | 0 |
| AE Special Interest* | 4 | 6 | 2 | 3 |
| SAE Infection | 2 | 3 | 3 | 1 |
| SAE Cardiac | 3 | 1 | 2 | 2 |
| AE Infection | 19 | 16 | 22 | 9 |
| AE Possible or Probable Related | 7 | 4 | 7 | 7 |

| | | | | |
|---|---|---|---|---|
| *AE of special interest was defined per the eCRF as Serious infection, Malignancy, Anaphylaxis, Gastrointestinal perforations, Hypersensitivity reaction during IP administration, severe Neutropenia, severe Neutropenia with evidence of concurrent infection, severe injection-related reaction, moderate Thrombocytopenia with evidence of concurrent, TIMI major bleeding, or severe Thrombocytopenia. | | | | |

| **Table 3: adverse events of special interest** | | | | | |
|---|---|---|---|---|---|
| | **Placebo** | **7.5mg** | **15mg** | **30mg** | **Total** |
| **N (%)** | **4 (6.2)** | **6 (9.2)** | **2 (3.0)** | **3 (4.6)** | **15 (5.7)** |
| Serious infection | 3 (4.6) | 6 (9.2) | 1 (1.5) | 2 (3.1) | 12 (4.6) |
| Malignancy | 1 (1.5) | 0 (0.0) | 1 (1.5) | 1 (1.5) | 3 (1.1) |
| Anaphylaxis | 0 (0.0) | 0 (0.0) | 0 (0.0) | 0 (0.0) | 0 (0.0) |
| Gastrointestinal perforations | 0 (0.0) | 0 (0.0) | 0 (0.0) | 0 (0.0) | 0 (0.0) |
| Hypersensitivity reaction during IP administration | 0 (0.0) | 0 (0.0) | 0 (0.0) | 0 (0.0) | 0 (0.0) |
| Neutropenia, severe | 0 (0.0) | 0 (0.0) | 0 (0.0) | 0 (0.0) | 0 (0.0) |
| Neutropenia, severe with evidence of concurrent infection | 0 (0.0) | 0 (0.0) | 0 (0.0) | 0 (0.0) | 0 (0.0) |
| Severe injection-related reaction | 0 (0.0) | 0 (0.0) | 0 (0.0) | 0 (0.0) | 0 (0.0) |
| Thrombocytopenia, moderate with evidence of concurrent TIMI major bleeding | 0 (0.0) | 0 (0.0) | 0 (0.0) | 0 (0.0) | 0 (0.0) |
| Thrombocytopenia, severe | 0 (0.0) | 0 (0.0) | 0 (0.0) | 0 (0.0) | 0 (0.0) |

In summary, the clinical data of the phase 2 RESCUE study indicate that Ziltivekimab at SC doses of 7.5 mg, 15 mg, and 30 mg once every 28 days can reduce inflammation without inducing immune suppression in patients with stage 3-5 CKD. In addition, Ziltivekimab improved markers of cardiovascular risk, atherosclerosis risk, inflammatory anemia, and inflammation-malnutrition. Ziltivekimab treatment did not lead to significant liver toxicity or increase in the number of adverse events.

### 5. INCORPORATION BY REFERENCE

All publications, patents, patent applications and other documents cited in this application are hereby incorporated by reference in their entireties for all purposes to the same extent as if each individual publication, patent, patent application or other document were individually indicated to be incorporated by reference for all purposes.

### 6. EQUIVALENTS

While various specific embodiments have been illustrated and described, the above specification is not restrictive. It will be appreciated that various changes can be made without departing from the spirit and scope of the invention(s). Many variations will become apparent to those skilled in the art upon review of this specification.

## Claims

1. A method of treating cardiovascular morbidity and mortality comprising administering ziltivekimab to a patient in need thereof, wherein the patient has chronic kidney disease (CKD).

2. The method according to claim 1, wherein ziltivekimab is administered subcutaneously at a dose of 7.5-30 mg once every 28 days.

3. The method according to claim 1, wherein ziltivekimab is administered subcutaneously at a monthly dose of 7.5-30 mg.

4. The method according to claim 1, wherein ziltivekimab is administered subcutaneously at a monthly equivalent dose of 7.5-30 mg.

5. The method according to claims 2-4, wherein ziltivekimab is administered at a dose of 7.5, 15 or 30 mg.

6. The method according to any preceding claim, wherein the patient has Kidney Disease Outcomes Quality Initiative (KDOQI) stage 3-5 CKD.

7. The method according to any preceding claim, wherein the patient has atherosclerotic cardiovascular disease (ASCVD).

8. The method according to any preceding claim, wherein the patient has inflammation.

9. The method according to any preceding claim, wherein the patient has IL-6 mediated inflammation.

10. The method according to claim 8 or 9, wherein the treatment is sufficient to reduce the inflammation without causing immune suppression.

11. The method according to any one of claims 8-10, wherein the inflammation is measured by C-reactive protein (CRP) levels.

12. The method according to claim 11, wherein the patient has a CRP level greater than 2 mg/L, and wherein the treatment is sufficient to reduce the CRP level to 2 mg/L or less.

13. The method according to claim 10, wherein the immune suppression is measured by absolute neutrophil count (ANC).

14. The method according to claim 13, wherein the post-treatment ANC is at least 1500 cells/µL.

15. A method of treating cardiovascular morbidity and mortality comprising administering ziltivekimab to a patient in need thereof; wherein the patient has CKD and ASCVD; wherein ziltivekimab is administered subcutaneously at a dose of 15 mg once every 28 days.

16. The method according to any preceding claim, wherein the cardiovascular morbidity and mortality is selected from a group consisting of (i) nonfatal myocardial infarction, (ii) nonfatal stroke, and (iii) cardiovascular death.

17. The method according to any preceding claim, wherein the cardiovascular morbidity and mortality is heart failure.
